(19) Europäisches Patentamt
European Patent Office
Office européen des brevets

(11) EP 4 656 626 A1

(12) EUROPEAN PATENT APPLICATION
published in accordance with Art. 153(4) EPC

(43) Date of publication:
**03.12.2025 Bulletin 2025/49**

(21) Application number: **24747098.2**

(22) Date of filing: **10.01.2024**

(51) International Patent Classification (IPC):
***C07C 69/44*** (2006.01) ***B32B 5/18*** (2006.01)
***B32B 27/22*** (2006.01) ***B32B 27/40*** (2006.01)
***C07C 43/205*** (2006.01) ***C07C 43/215*** (2006.01)
***C07C 69/34*** (2006.01) ***C07C 69/82*** (2006.01)
***C07C 271/12*** (2006.01) ***C08K 5/00*** (2006.01)
***C08L 27/06*** (2006.01) ***C08L 67/00*** (2006.01)
***C08L 101/00*** (2006.01)

(52) Cooperative Patent Classification (CPC):
**B32B 5/18; B32B 27/22; B32B 27/40; C07C 43/205; C07C 43/215; C07C 69/34; C07C 69/44; C07C 69/82; C07C 271/12; C08K 5/00; C08L 27/06; C08L 67/00; C08L 101/00**

(86) International application number:
**PCT/JP2024/000325**

(87) International publication number:
**WO 2024/157767 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.01.2023 JP 2023011401**

(71) Applicant: **Zeon Corporation**
**Tokyo 100-8246 (JP)**

(72) Inventors:
- **NISHIMURA Shota**
**Tokyo 100-8246 (JP)**
- **SAKAMOTO Kei**
**Tokyo 100-8246 (JP)**

(74) Representative: **Maiwald GmbH**
**Elisenhof**
**Elisenstraße 3**
**80335 München (DE)**

# (54) COMPOUND AND METHOD FOR USING SAME, PLASTICIZER COMPOSITION, RESIN COMPOSITION, RESIN MOLDED BODY, AND LAMINATED BODY

(57) Provided is a compound that can be used in production of a resin composition that is capable of forming a resin molded product having excellent low-temperature tensile elongation and heat shrinkage resistance. The compounded is represented by formula (1), shown below (in formula (1), Ax represents an organic group having a carbon number of 3 to 20, $Y^1$, $Y^2$, $Y^3$, and $Y^4$ each independently represent a single bond, -C(=O)-, etc., $R^1$ represents a hydrogen atom, a methyl group, etc., $SP^1$, $SP^2$, $SP^3$, and $SP^4$ each independently represent a specific organic group, a, b, c, and d each independently represent an integer of 1 to 30, $R^a$, $R^b$, $R^c$, and $R^d$ each independently represent an optionally substituted chain aliphatic hydrocarbon group having a carbon number of 12 to 18, and x and y each independently represent 0 or 1).

EP 4 656 626 A1

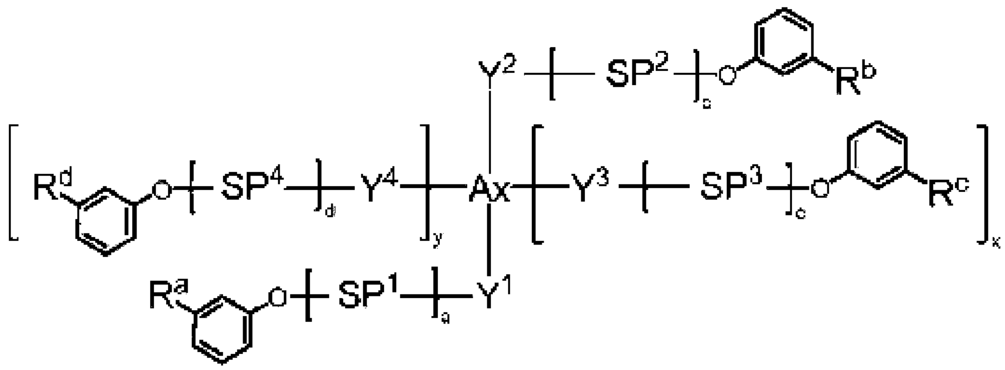

(1)

## Description

### TECHNICAL FIELD

The present disclosure relates to a compound and method of using the same, a plasticizer composition, a resin composition, a resin molded product, and a laminate.

### BACKGROUND

Resins such as vinyl chloride resins are used in a variety of applications due to generally having excellent characteristics in terms of cold resistance, heat resistance, oil resistance, and so forth.

Specifically, automobile interior materials such as a surface skin formed of a resin molded product in which a vinyl chloride resin or other resin is used and a laminate obtained by lining a surface skin formed of such a resin molded product with a foam such as foamed polyurethane are used in the formation of automobile interior components such as automobile instrument panels and door trims, for example.

A resin molded product that constitutes a surface skin of an automobile interior component such as an automobile instrument panel is produced, for example, by performing molding by a powder molding method such as powder slush molding with respect to a resin composition that contains a resin such as a vinyl chloride resin, a plasticizer, and additives (for example, refer to Patent Literature (PTL) 1).

As one specific example, a vinyl chloride resin molded product is produced in PTL 1 through powder slush molding of a vinyl chloride resin composition that contains vinyl chloride resin particles, a plasticizer such as a polyester plasticizer, and additives such as a hydrotalcite stabilizer, a zeolite stabilizer, and a β-diketone.

### CITATION LIST

#### Patent Literature

PTL 1: JP2012-197394A

### SUMMARY

#### (Technical Problem)

It is desirable for a resin molded product such as described above to have excellent tensile elongation at low temperature (hereinafter, also referred to as "low-temperature tensile elongation").

Moreover, it may be the case that a resin molded product such as described above is lined with a foam of polyurethane (hereinafter, also referred to as a "foamed polyurethane molded product") in order to produce a laminate as an automobile interior material. In a situation in which the laminate serving as an automobile interior material is placed at high temperature, a plasticizer that is contained in the resin molded product may migrate into the foamed polyurethane molded product, and heat shrinkage of the resin molded product may occur. Therefore, it is desirable that a resin molded product used with a foamed polyurethane molded product to form a laminate inhibits the heat shrinkage described above (i.e., has heat shrinkage resistance).

However, there is room for improvement of low-temperature tensile elongation and heat shrinkage resistance of a resin molded product that is formed when using a resin composition containing a plasticizer according to the conventional technique described above.

Accordingly, one object of the present disclosure is to provide a compound that can be used in production of a resin composition that is capable of forming a resin molded product having excellent low-temperature tensile elongation and heat shrinkage resistance and a method of using this compound.

Another object of the present disclosure is to provide a plasticizer composition that can be used in production of a resin composition that is capable of forming a resin molded product having excellent low-temperature tensile elongation and heat shrinkage resistance.

Another object of the present disclosure is to provide a resin composition that is capable of forming a resin molded product having excellent low-temperature tensile elongation and heat shrinkage resistance.

Another object of the present disclosure is to provide a resin molded product having excellent low-temperature tensile elongation and heat shrinkage resistance.

Another object of the present disclosure is to provide a laminate including this resin molded product.

(Solution to Problem)

The inventors conducted diligent investigation with the aim of solving the problems set forth above. The inventors discovered that when a compound having a specific structure is used in production of a resin composition, a resin molded product that is formed using the produced resin composition has both excellent low-temperature tensile elongation and excellent heat shrinkage resistance. In this manner, the inventors completed the present disclosure.

Specifically, with the aim of advantageously solving the problems set forth above, [1] a presently disclosed compound is represented by formula (1), shown below:

[Chem. 1]

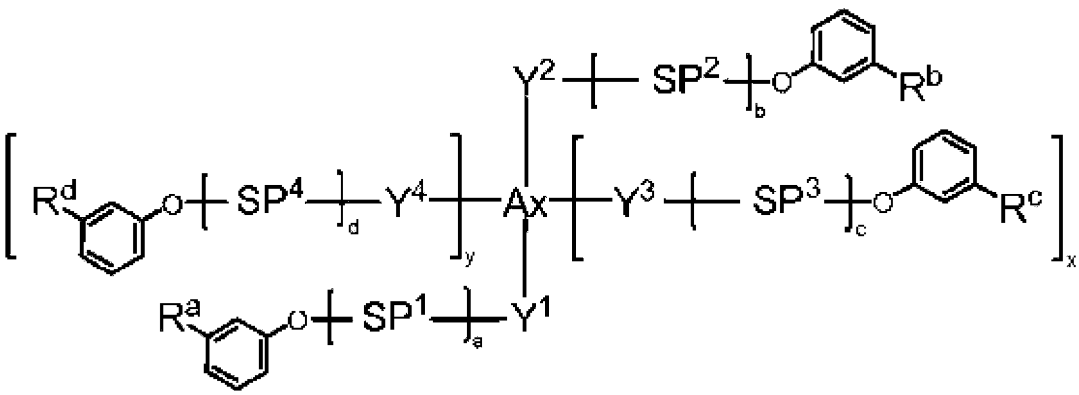

· · · (1)

where, in formula (1):

Ax represents an organic group having a carbon number of 3 to 20;
$Y^1$ and $Y^4$ each independently represent a single bond, -C(=O)-, -C(=O)-$NR^1$-, or -C(=O)-O-;
$R^1$ represents a hydrogen atom, a methyl group, or an ethyl group;
$Y^2$ and $Y^3$ each independently represent a single bond, -C(=O)-, -$NR^2$-C(=O)-, or -O-C(=O)-;
$R^2$ represents a hydrogen atom, a methyl group, or an ethyl group;
$SP^1$ and $SP^4$ each independently represent $-CH_2CH_2O-$, $-CH_2CH(CH_3)O-$, $-CH(CH_3)CH_2O-$, $-CH_2CH(CH_2CH_3)O-$, or $-CH(CH_2CH_3)CH_2O-$;
$SP^2$ and $SP^3$ each independently represent $-OCH_2CH_2-$, $-OCH(CH_3)CH_2-$, $-OCH_2CH(CH_3)-$, $-OCH(CH_2CH_3)CH_2-$, or $-OCH_2CH(CH_2CH_3)-$;
a, b, c, and d each independently represent an integer of 1 to 30;
$R^a$, $R^b$, $R^c$, and $R^d$ each independently represent an optionally substituted chain aliphatic hydrocarbon group having a carbon number of 12 to 18;
x and y each independently represent 0 or 1;
in a case in which x is 0, a structure represented by formula (2), shown below:

[Chem. 2]

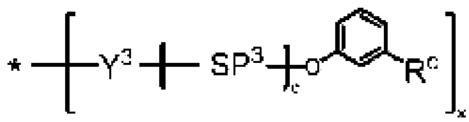

· · · (2)

represents a hydrogen atom;
in a case in which y is 0, a structure represented by formula (3), shown below:

[Chem. 3]

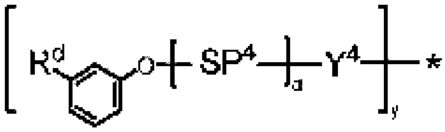

... (3)

represents a hydrogen atom; and

* in formula (2) and formula (3) represents a bonding position with Ax.

By using a compound having the specific structure set forth above in this manner, it is possible to produce a resin composition that is capable of forming a resin molded product having excellent low-temperature tensile elongation and heat shrinkage resistance.

[2] In the compound according to the foregoing [1], Ax is preferably any organic group among:

(i) an optionally substituted chain aliphatic hydrocarbon group having a carbon number of 3 to 20;
(ii) an optionally substituted cyclic aliphatic hydrocarbon group having a carbon number of 4 to 12;
(iii) an optionally substituted aromatic hydrocarbon group having a carbon number of 6 to 14; and
(iv) an organic group having a carbon number of 3 to 20 in which at least one single bond included in an optionally substituted chain aliphatic hydrocarbon group has been replaced by -O-, -C(=O)-, -O-C(=O)-, or -C(=O)-O-, with a proviso that a case in which -O- is consecutively present twice or more, a case in which -C(=O)- is consecutively present twice or more, and a case in which -O- or -C(=O)- is located at an end bonded to $Y^1$, $Y^2$, $Y^3$, or $Y^4$ are excluded.

[3] In the compound according to the foregoing [1] or [2], Ax is preferably a group represented by any one of formulae (2-1) to (2-35), shown below:

[Chem. 4]

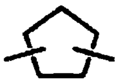
(2-1)

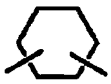
(2-2)

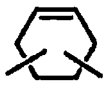
(2-3)

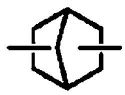
(2-4)

$-(CH_2)_3-$ (2-5)

$-(CH_2)_4-$ (2-6)

$-(CH_2)_5-$ (2-7)

$-(CH_2)_6-$ (2-8)

$-(CH_2)_7-$ (2-9)

$-(CH_2)_8-$ (2-10)

$-(CH_2)_9-$ (2-11)

$-(CH_2)_{10}-$ (2-12)

$-(CH_2)_{11}-$ (2-13)

$-(CH_2)_{12}-$ (2-14)

$-(CH_2)_{13}-$ (2-15)

$-(CH_2)_{14}-$ (2-16)

$-(CH_2)_{15}-$ (2-17)

$-(CH_2)_{16}-$ (2-18)

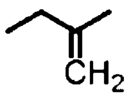
(2-19)

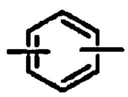
(2-20)

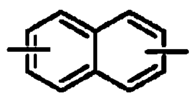
(2-21)

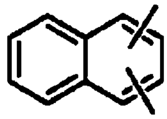
(2-22)

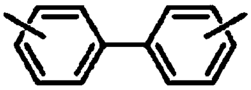
(2-23)

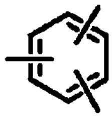
(2-24)

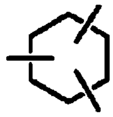
(2-25)

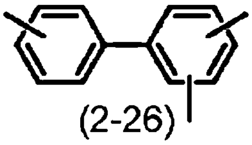
(2-26)

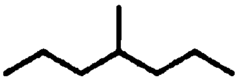
(2-27)

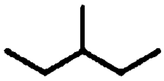
(2-28)

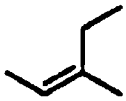
(2-29)

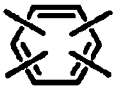
(2-30)

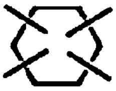
(2-31)

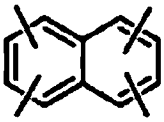
(2-32)

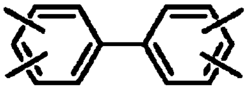
(2-33)

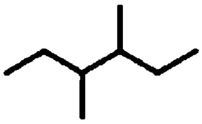
(2-34)

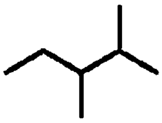
(2-35)

that is optionally substituted.

Moreover, with the aim of advantageously solving the problems set forth above, [4] a presently disclosed method comprises using the compound according to any one of the foregoing [1] to [3] as a plasticizer.

By using any one of the compounds set forth above as a plasticizer in this manner, it is possible to produce a resin composition that is capable of forming a resin molded product having excellent low-temperature tensile elongation and heat shrinkage resistance.

Furthermore, with the aim of advantageously solving the problems set forth above, [5] a presently disclosed plasticizer composition comprises the compound according to any one of the foregoing [1] to [3].

By using a plasticizer composition that contains any one of the compounds set forth above in this manner, it is possible to produce a resin composition that is capable of forming a resin molded product having excellent low-temperature tensile elongation and heat shrinkage resistance.

[6] The plasticizer composition according to the foregoing [5] can comprise two or more types of the compound that have different structures to each other.

[7] The plasticizer composition according to the foregoing [5] or [6] preferably further comprises a polyester plasticizer.

When the plasticizer composition further contains a polyester plasticizer, heat shrinkage resistance of a resin molded product that is formed using a resin composition containing the plasticizer composition can be further improved.

[8] In the plasticizer composition according to the foregoing [7], the polyester plasticizer preferably includes an adipic acid polyester.

When an adipic acid polyester is used as the polyester plasticizer, heat shrinkage resistance of a resin molded product that is formed using a resin composition containing the plasticizer composition can be even further improved.

[9] In the plasticizer composition according to the foregoing [7] or [8], content of the compound is preferably not less than 1 part by mass and not more than 20 parts by mass relative to 100 parts by mass of the polyester plasticizer.

When the content of the presently disclosed compound set forth above in the plasticizer composition is within the specific range set forth above, sufficiently high heat shrinkage resistance of a formed resin molded product can be ensured while also improving adhesiveness of the resin molded product to a foamed polyurethane molded product and further improving low-temperature tensile elongation of the resin molded product.

Also, with the aim of advantageously solving the problems set forth above, [10] a presently disclosed resin composition comprises: a resin; and the plasticizer composition according to any one of the foregoing [5] to [9].

Through a resin composition that contains a resin and the plasticizer composition set forth above in this manner, it

is possible to form a resin molded product having excellent low-temperature tensile elongation and heat shrinkage resistance.

[11] In the resin composition according to the foregoing [10], the resin preferably contains a halogen.

[12] In the resin composition according to the foregoing [10] or [11], the resin preferably has a glass-transition temperature of not lower than 50°C and not higher than 100°C.

Note that the "glass-transition temperature" of a resin referred to in the present disclosure can be measured in accordance with JIS K 7121 using a differential scanning calorimeter (DSC).

[13] In the resin composition according to any one of the foregoing [10] to [12], the resin preferably includes a vinyl chloride resin.

[14] In the resin composition according to any one of the foregoing [10] to [13], content of the compound is preferably not less than 1 part by mass and not more than 20 parts by mass relative to 100 parts by mass of the resin.

When the content of the presently disclosed compound set forth above in the resin composition is within the specific range set forth above, sufficiently high heat shrinkage resistance of a formed resin molded product can be ensured while also improving adhesiveness of the resin molded product to a foamed polyurethane molded product and further improving low-temperature tensile elongation of the resin molded product.

[15] The resin composition according to any one of the foregoing [10] to [14] is preferably used in powder molding.

By using the resin composition in powder molding, it is easy to obtain a resin molded product that can be used well as an automobile interior material such as a surface skin for an automobile instrument panel, for example.

[16] The resin composition according to any one of the foregoing [10] to [15] is preferably used in powder slush molding.

By using the resin composition in powder slush molding, it is even easier to obtain a resin molded product that can be used well as an automobile interior material such as a surface skin for an automobile instrument panel, for example.

Moreover, with the aim of advantageously solving the problems set forth above, [17] a presently disclosed resin molded product is obtained through molding of the resin composition according to any one of the foregoing [10] to [16].

A resin molded product that is obtained through molding of the resin composition set forth above in this manner can be used well as an automobile interior material as a result of having excellent low-temperature tensile elongation and heat shrinkage resistance.

[18] The resin molded product according to the foregoing [17] is preferably for an automobile instrument panel surface skin.

By using the presently disclosed resin molded product as a surface skin of an automobile instrument panel, it is possible to produce an automobile instrument panel having a surface skin with excellent low-temperature tensile elongation and heat shrinkage resistance.

Furthermore, with the aim of advantageously solving the problems set forth above, [19] a presently disclosed laminate comprises: a foamed polyurethane molded product; and the resin molded product according to the foregoing [17] or [18].

A laminate that includes a foamed polyurethane molded product and the resin molded product set forth above has a resin molded product part with excellent low-temperature tensile elongation and heat shrinkage resistance.

[20] The laminate according to the foregoing [19] is preferably for an automobile instrument panel.

By using the presently disclosed laminate as an automobile instrument panel, it is possible to improve low-temperature tensile elongation and heat shrinkage resistance of a surface skin of the produced automobile instrument panel.

(Advantageous Effect)

According to the present disclosure, it is possible to provide a compound that can be used in production of a resin composition that is capable of forming a resin molded product having excellent low-temperature tensile elongation and heat shrinkage resistance and a method of using this compound.

Moreover, according to the present disclosure, it is possible to provide a plasticizer composition that can be used in production of a resin composition that is capable of forming a resin molded product having excellent low-temperature tensile elongation and heat shrinkage resistance.

Furthermore, according to the present disclosure, it is possible to provide a resin composition that is capable of forming a resin molded product having excellent low-temperature tensile elongation and heat shrinkage resistance.

Also, according to the present disclosure, it is possible to provide a resin molded product having excellent low-temperature tensile elongation and heat shrinkage resistance.

Moreover, according to the present disclosure, it is possible to provide a laminate including this resin molded product.

BRIEF DESCRIPTION OF THE DRAWINGS

In the accompanying drawings:

FIG. 1 illustrates a spectrum obtained through $^{1}H$-NMR measurement of Cardolite LITE 2020 (produced by Cardolite Corporation) used in synthesis examples;
FIG. 2 illustrates a spectrum obtained through $^{13}C$-NMR measurement of Cardolite LITE 2020 (produced by Cardolite Corporation) used in synthesis examples;
FIG. 3 illustrates a spectrum obtained through C-H COSY measurement of Cardolite LITE 2020 (produced by Cardolite Corporation) used in synthesis examples;
FIG. 4 illustrates a spectrum obtained through infrared spectroscopy (attenuated total reflection) (hereinafter, also abbreviated as "IR (ATR)") measurement of Cardolite LITE 2020 (produced by Cardolite Corporation) used in synthesis examples;
FIG. 5 illustrates a spectrum obtained through $^{1}H$-NMR measurement of Cardolite NX-7507 (produced by Cardolite Corporation) used in synthesis examples;
FIG. 6 illustrates a spectrum obtained through $^{13}C$-NMR measurement of Cardolite NX-7507 (produced by Cardolite Corporation) used in synthesis examples;
FIG. 7 illustrates a spectrum obtained through C-H COSY measurement of Cardolite NX-7507 (produced by Cardolite Corporation) used in synthesis examples;
FIG. 8 illustrates a spectrum obtained through IR (ATR) measurement of Cardolite NX-7507 (produced by Cardolite Corporation) used in synthesis examples;
FIG. 9 illustrates a spectrum obtained through $^{1}H$-NMR measurement of a diester mixture 1 obtained in Synthesis Example 1;
FIG. 10 illustrates a spectrum obtained through $^{13}C$-NMR measurement of the diester mixture 1 obtained in Synthesis Example 1;
FIG. 11 illustrates a spectrum obtained through IR (ATR) measurement of the diester mixture 1 obtained in Synthesis Example 1;
FIG. 12 illustrates a spectrum obtained through $^{1}H$-NMR measurement of a dicarbamate mixture 1 obtained in Synthesis Example 2;
FIG. 13 illustrates a spectrum obtained through $^{13}C$-NMR measurement of the dicarbamate mixture 1 obtained in Synthesis Example 2;
FIG. 14 illustrates a spectrum obtained through IR (ATR) measurement of the dicarbamate mixture 1 obtained in Synthesis Example 2;
FIG. 15 illustrates a spectrum obtained through $^{1}H$-NMR measurement of a diether mixture 1 obtained in Synthesis Example 3;
FIG. 16 illustrates a spectrum obtained through IR (ATR) measurement of the diether mixture 1 obtained in Synthesis Example 3;
FIG. 17 illustrates a spectrum obtained through $^{1}H$-NMR measurement of a diester mixture 2 obtained in Synthesis Example 4;
FIG. 18 illustrates a spectrum obtained through $^{13}C$-NMR measurement of the diester mixture 2 obtained in Synthesis Example 4;
FIG. 19 illustrates a spectrum obtained through IR (ATR) measurement of the diester mixture 2 obtained in Synthesis Example 4;
FIG. 20 illustrates a spectrum obtained through $^{1}H$-NMR measurement of a triester mixture 1 obtained in Synthesis Example 5;
FIG. 21 illustrates a spectrum obtained through $^{13}C$-NMR measurement of the triester mixture 1 obtained in Synthesis Example 5;
FIG. 22 illustrates a spectrum obtained through IR (ATR) measurement of the triester mixture 1 obtained in Synthesis Example 5;
FIG. 23 illustrates a spectrum obtained through $^{1}H$-NMR measurement of a diester mixture 3 obtained in Synthesis Example 6;
FIG. 24 illustrates a spectrum obtained through $^{13}C$-NMR measurement of the diester mixture 3 obtained in Synthesis Example 6;
FIG. 25 illustrates a spectrum obtained through IR (ATR) measurement of the diester mixture 3 obtained in Synthesis Example 6;
FIG. 26 illustrates a spectrum obtained through $^{1}H$-NMR measurement of a diester mixture 4 obtained in Synthesis Example 7;
FIG. 27 illustrates a spectrum obtained through $^{13}C$-NMR measurement of the diester mixture 4 obtained in Synthesis

Example 7; and
FIG. 28 illustrates a spectrum obtained through IR (ATR) measurement of the diester mixture 4 obtained in Synthesis Example 7.

DETAILED DESCRIPTION

The following provides a detailed description of embodiments of the present disclosure.
The presently disclosed compound can be used in production of the presently disclosed plasticizer composition, for example.
Moreover, the presently disclosed plasticizer composition can be used in production of the presently disclosed resin composition, for example.
Furthermore, the presently disclosed resin composition can be used in formation of the presently disclosed resin molded product, for example. A resin molded product formed using the presently disclosed resin composition can suitably be used as an automobile interior material such as a surface skin included in an automobile interior component such as an automobile instrument panel or a door trim, for example.
Also, the presently disclosed resin molded product can be used in formation of the presently disclosed laminate, for example. A laminate formed using the presently disclosed resin molded product can suitably be used as an automobile interior material used in production of an automobile interior component such as an automobile instrument panel or a door trim, for example.

(Compound)

The presently disclosed compound is a compound represented by formula (1), shown below.

[Chem. 5]

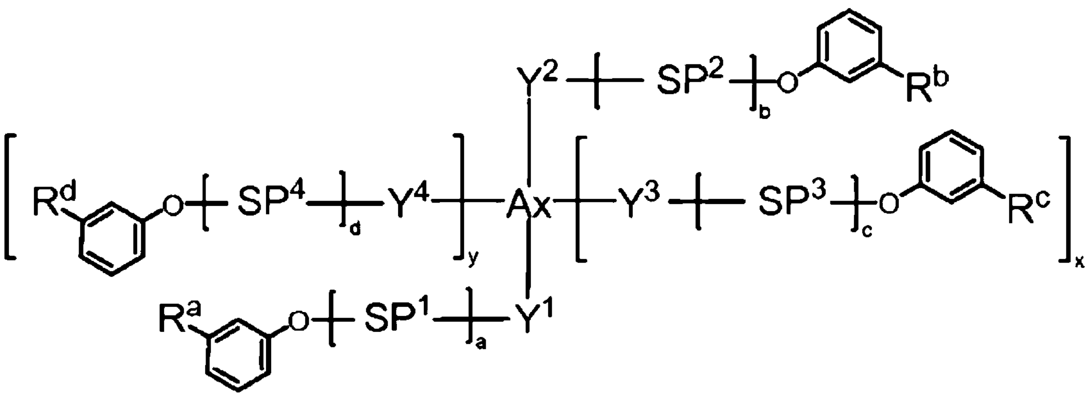

・・・(1)

The presently disclosed compound can sufficiently improve low-temperature tensile elongation and heat shrinkage resistance of a resin molded product that is obtained through molding of a resin composition produced using the compound.

<x and y>

In formula (1), x and y each independently represent 0 or 1.
In a case in which x is 0, a structure represented by formula (2), shown below:

[Chem. 6]

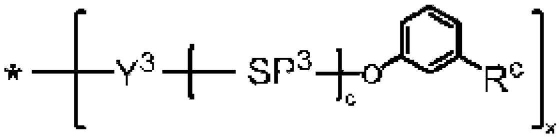
· · · (2)

represents a hydrogen atom.

Moreover, in a case in which y is 0, a structure represented by formula (3), shown below:

[Chem. 7]

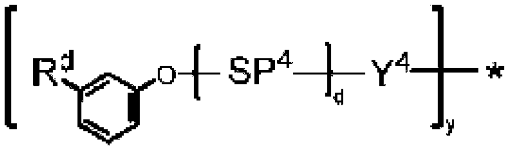
· · · (3)

represents a hydrogen atom.

Note that * in formulae (2) and (3) represents a bonding position with Ax.

Although it may be the case that x and y are each 0, that x and y are each 1, or that one of x and y is 0 and the other thereof is 1, it is preferable that x and y are each 0 from a viewpoint of further improving low-temperature tensile elongation and heat shrinkage resistance of a resin molded product.

<Ax>

In formula (1), Ax represents an organic group having a carbon number of 3 to 20.

It may be the case that a single carbon atom in the organic group constituting Ax is bonded to two or more of $Y^1$, $Y^2$, $Y^3$ (limited to a case in which x = 1), and $Y^4$ (limited to a case in which y = 1), or it may be the case that a single carbon atom in the organic group constituting Ax is not bonded to two or more of $Y^1$, $Y^2$, $Y^3$ (limited to a case in which x = 1), and $Y^4$ (limited to a case in which y = 1).

From a viewpoint of further improving low-temperature tensile elongation of a resin molded product, it is preferable that a single carbon atom in the organic group constituting Ax is not bonded to two or more of $Y^1$, $Y^2$, $Y^3$ (limited to a case in which x = 1), and $Y^4$ (limited to a case in which y = 1) (i.e., that a carbon atom bonded to $Y^1$, a carbon atom bonded to $Y^2$, a carbon atom bonded to $Y^3$ (limited to a case in which x = 1), and a carbon atom bonded to $Y^4$ (limited to a case in which y = 1) are different carbon atoms to one another).

Moreover, in a case in which a single carbon atom in the organic group constituting Ax is not bonded to two or more of $Y^1$, $Y^2$, $Y^3$ (limited to a case in which x = 1), and $Y^4$ (limited to a case in which y = 1), a carbon atom bonded to $Y^1$, a carbon atom bonded to $Y^2$, a carbon atom bonded to $Y^3$ (limited to a case in which x = 1), and a carbon atom bonded to $Y^4$ (limited to a case in which y = 1) may be adjacent to one another or may not be adjacent to one another in the organic group constituting Ax.

Furthermore, from a viewpoint of even further improving low-temperature tensile elongation of a resin molded product, it is preferable that a carbon atom bonded to $Y^1$, a carbon atom bonded to $Y^2$, a carbon atom bonded to $Y^3$ (limited to a case in which x = 1), and a carbon atom bonded to $Y^4$ (limited to a case in which y = 1) are not adjacent to one another in the organic group constituting Ax.

Ax is preferably any organic group among:

(i) an optionally substituted chain aliphatic hydrocarbon group having a carbon number of 3 to 20;
(ii) an optionally substituted cyclic aliphatic hydrocarbon group having a carbon number of 4 to 12;
(iii) an optionally substituted aromatic hydrocarbon group having a carbon number of 6 to 14; and
(iv) an organic group having a carbon number of 3 to 20 in which at least one single bond included in an optionally substituted chain aliphatic hydrocarbon group has been replaced by -O-, -C(=O)-, -O-C(=O)-, or -C(=O)-O- (with a proviso that a case in which -O- is consecutively present twice or more, a case in which -C(=O)- is consecutively present twice or more, and a case in which -O- or -C(=O)- is located at an end bonded to $Y^1$, $Y^2$, $Y^3$, or $Y^4$ are excluded),

is more preferably any organic group among (i) and (iii), and is even more preferably an organic group of (i).

Note that the phrase "optionally substituted" as used in the present disclosure means "unsubstituted or substituted". Moreover, in a case in which an organic group included in a general formula is substituted, the number

of substituents included in the organic group may be one or may be two or more. Furthermore, when an organic group included in a general formula has a substituent, the carbon number of the organic group including that substituent does not include the carbon number of the substituent unless otherwise specified. For example, in a case in which a chain aliphatic hydrocarbon group having a carbon number of 3 to 20 has a substituent, the carbon number of the chain aliphatic hydrocarbon group having a carbon number of 3 to 20 is considered to not include the carbon number of the substituent.

The carbon number of the chain aliphatic hydrocarbon group having a carbon number of 3 to 20 in (i) described above is preferably 3 to 15, more preferably 3 to 12, even more preferably 3 to 10, further preferably 3 to 8, even further preferably 3 to 6, and particularly preferably 3 to 4.

However, the total carbon number of the chain aliphatic hydrocarbon group having a carbon number of 3 to 20 in (i) described above, inclusive of the carbon number of any substituents, is 3 to 20.

The chain aliphatic hydrocarbon group having a carbon number of 3 to 20 in (i) described above may be linear or may be branched.

The chain aliphatic hydrocarbon group having a carbon number of 3 to 20 in (i) described above may be an unsaturated aliphatic hydrocarbon group that includes an unsaturated bond (carbon-carbon double bond or carbon-carbon triple bond) or may be a saturated aliphatic hydrocarbon group that does not include an unsaturated bond. Moreover, in a case in which the chain aliphatic hydrocarbon group having a carbon number of 3 to 20 in (i) described above includes an unsaturated bond, the number of unsaturated bonds may be not less than 1 and not more than 3, for example.

Examples of possible substituents of the chain aliphatic hydrocarbon group having a carbon number of 3 to 20 in (i) described above include halogen atoms and the like, for example, but are not specifically limited thereto.

The carbon number of the cyclic aliphatic hydrocarbon group having a carbon number of 4 to 12 in (ii) described above is preferably 4 to 15, more preferably 4 to 12, even more preferably 4 to 10, further preferably 4 to 8, and even further preferably 4 to 6.

However, the total carbon number of the cyclic aliphatic hydrocarbon group having a carbon number of 4 to 12 in (ii) described above, inclusive of the carbon number of any substituents, is 3 to 20.

The cyclic aliphatic hydrocarbon group having a carbon number of 4 to 12 in (ii) described above may be an unsaturated aliphatic hydrocarbon group that includes an unsaturated bond (carbon-carbon double bond or carbon-carbon triple bond) or may be a saturated aliphatic hydrocarbon group that does not include an unsaturated bond.

Examples of possible substituents of the cyclic aliphatic hydrocarbon group having a carbon number of 4 to 12 in (ii) described above include halogen atoms, alkyl groups having a carbon number of 1 to 8, and the like, for example.

The carbon number of the aromatic hydrocarbon ring group having a carbon number of 6 to 14 in (iii) described above is preferably 6 to 12.

However, the total carbon number of the aromatic hydrocarbon ring group having a carbon number of 6 to 14 in (iii) described above, inclusive of the carbon number of any substituents, is 3 to 20.

Examples of possible substituents of the aromatic hydrocarbon ring group having a carbon number of 6 to 14 in (iii) described above include halogen atoms, alkyl groups having a carbon number of 1 to 6, and the like.

The carbon number of the organic group having a carbon number of 3 to 20 in (iv) described above is preferably 3 to 15, more preferably 3 to 12, even more preferably 3 to 10, further preferably 3 to 8, even further preferably 3 to 6, and particularly preferably 3 to 4.

Note that in the organic group having a carbon number of 3 to 20 in (iv) described above, in a case in which at least one single bond included in the optionally substituted chain aliphatic hydrocarbon group has been replaced by -C(=O)-, -O-C(=O)-, or -C(=O)-O-, the carbon number of -C(=O)-, -O-C(=O)-, or -C(=O)-O- is considered to be included in the carbon number of the organic group having a carbon number of 3 to 20 in (iv) described above.

However, the total carbon number of the organic group having a carbon number of 3 to 20 in (iv) described above, inclusive of the carbon number of any substituents, is 3 to 20.

Examples of possible substituents of the organic group having a carbon number of 3 to 20 in (iv) described above include halogen atoms and the like, for example.

Specific examples of Ax that are suitable include groups represented by formulae (2-1) to (2-35), shown below, that are optionally substituted. The term "substituted" as used here means that any hydrogen atom of a group represented by any one of the following formulae (2-1) to (2-35) is substituted.

[Chem. 8]

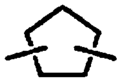
(2-1)

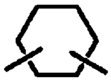
(2-2)

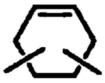
(2-3)

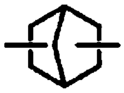
(2-4)

$-(CH_2)_3-$
(2-5)

$-(CH_2)_4-$
(2-6)

$-(CH_2)_5-$
(2-7)

$-(CH_2)_6-$
(2-8)

$-(CH_2)_7-$
(2-9)

$-(CH_2)_8-$
(2-10)

$-(CH_2)_9-$
(2-11)

$-(CH_2)_{10}-$
(2-12)

$-(CH_2)_{11}-$
(2-13)

$-(CH_2)_{12}-$
(2-14)

$-(CH_2)_{13}-$
(2-15)

$-(CH_2)_{14}-$
(2-16)

$-(CH_2)_{15}-$
(2-17)

$-(CH_2)_{16}-$
(2-18)

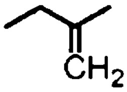
(2-19)

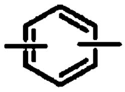
(2-20)

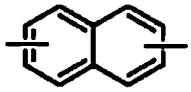
(2-21)

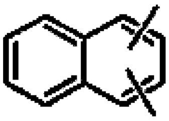
(2-22)

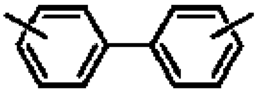
(2-23)

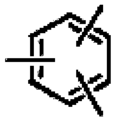
(2-24)

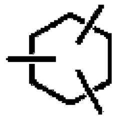
(2-25)

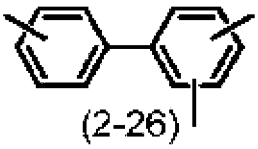
(2-26)

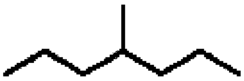
(2-27)

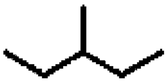
(2-28)

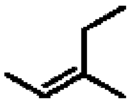
(2-29)

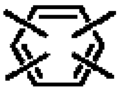
(2-30)

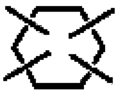
(2-31)

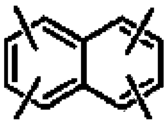
(2-32)

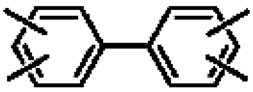
(2-33)

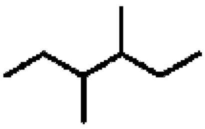
(2-34)

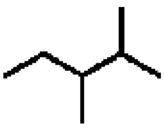
(2-35)

Note that "-" in formulae (2-1) to (2-4), (2-20) to (2-26), and (2-30) to (2-33) represents a bond with $Y^1$, $Y^2$, $Y^3$, or $Y^4$ that extends from any position in a ring.

Moreover, "-" located at the end of a chain structure in formulae (2-5) to (2-19), (2-27) to (2-29), (2-34), and (2-35) represents a bond with $Y^1$, $Y^2$, $Y^3$, or $Y^4$.

Note that in a case in which x is 0, the structure represented by formula (2) (i.e., a hydrogen atom) can be any

hydrogen atom included in the group represented by any one of formulae (2-1) to (2-35), and in a case in which y is 0, the structure represented by formula (3) (i.e., a hydrogen atom) can be any hydrogen atom included in the group represented by any one of formulae (2-1) to (2-35).

Examples of possible substituents of the group represented by any one of formulae (2-1) to (2-35) include halogen atoms, alkyl groups having a carbon number of 1 to 4, and the like, for example.

Moreover, from a viewpoint of further improving low-temperature tensile elongation and heat shrinkage resistance of a resin molded product, Ax is preferably a group represented by any one of formulae (2-5), (2-6), (2-8), (2-20), and (2-27), more preferably a group represented by any one of formulae (2-5), (2-6), (2-8), and (2-20), and even more preferably a group represented by formula (2-6).

<$Y^1$ and $Y^4$>

In formula (1), $Y^1$ and $Y^4$ each independently represent a single bond, -C(=O)-, -C(=O)-$NR^1$-, or -C(=O)-O-. Note that $R^1$ represents a hydrogen atom, a methyl group, or an ethyl group.

Moreover, from a viewpoint of further improving low-temperature tensile elongation and heat shrinkage resistance of a resin molded product and improving adhesiveness of the resin molded product to a foamed polyurethane molded product, $Y^1$ and $Y^4$ are each, independently of each other, preferably a single bond, -C(=O)-, or -C(=O)-$NR^1$-, more preferably a single bond or -C(=O)-, and even more preferably -C(=O)-.

<$Y^2$ and $Y^3$>

In formula (1), $Y^2$ and $Y^3$ each independently represent a single bond, -C(=O)-, -$NR^2$-C(=O)-, or -O-C(=O)-. Note that $R^2$ represents a hydrogen atom, a methyl group, or an ethyl group.

Moreover, from a viewpoint of further improving low-temperature tensile elongation and heat shrinkage resistance of a resin molded product and improving adhesiveness of the resin molded product to a foamed polyurethane molded product, $Y^2$ and $Y^3$ are each, independently of each other, preferably a single bond, -C(=O)-, or -C(=O)-$NR^2$-, more preferably a single bond or -C(=O)-, and even more preferably -C(=O)-.

<$SP^1$ and $SP^4$>

In formula (1), $SP^1$ and $SP^4$ each independently represent $-CH_2CH_2O-$, $-CH_2CH(CH_3)O-$, $-CH(CH_3)CH_2O-$, $-CH_2CH(CH_2CH_3)O-$, or $-CH(CH_2CH_3)CH_2O-$.

Moreover, $SP^1$ and $SP^4$ are each, independently of each other, preferably $-CH_2CH_2O-$, $-CH_2CH(CH_3)O-$, or $-CH(CH_3)CH_2O-$, and more preferably $-CH_2CH_2O-$.

<$SP^2$ and $SP^3$>

In formula (1), $SP^2$ and $SP^3$ each independently represent $-OCH_2CH_2-$, $-OCH(CH_3)CH_2-$, $-OCH_2CH(CH_3)-$, $-O(CH_3CH_2)CHCH_2-$, or $-OCH_2CH(CH_2CH_3)-$.

Moreover, $SP^2$ and $SP^3$ are each, independently of each other, preferably $-OCH_2CH_2-$, $-OCH(CH_3)CH_2-$, or $-OCH_2CH(CH_3)-$, and more preferably $-OCH_2CH_2-$.

<a, b, c, and d>

In formula (1), a, b, c, and d each independently represent an integer of 1 to 30.

From a viewpoint of further improving heat shrinkage resistance of a resin molded product and also improving adhesiveness of the resin molded product to a foamed polyurethane molded product, a, b, c, and d each, independently of one another, preferably represent an integer of 1 to 25, more preferably represent an integer of 2 to 20, even more preferably represent an integer of 3 to 15, further preferably represent an integer of 4 to 12, and even further preferably represent an integer of 5 to 10.

<$R^a$, $R^b$, $R^c$, and $R^d$>

In formula (1), $R^a$, $R^b$, $R^c$, and $R^d$ each independently represent an optionally substituted chain aliphatic hydrocarbon group having a carbon number of 12 to 18.

The carbon numbers of the optionally substituted chain aliphatic hydrocarbon groups having a carbon number of 12 to 18 that constitute $R^a$, $R^b$, $R^c$, and $R^d$ are each, independently of one another, preferably 13 to 17, and more preferably 14 to 16.

The optionally substituted chain aliphatic hydrocarbon groups having a carbon number of 12 to 18 that constitute $R^a$, $R^b$, $R^c$, and $R^d$ may each, independently of one another, be an unsaturated aliphatic hydrocarbon group that includes an unsaturated bond (carbon-carbon double bond or carbon-carbon triple bond) or be a saturated aliphatic hydrocarbon group that does not include an unsaturated bond. Moreover, in a case in which the chain aliphatic hydrocarbon group having a carbon number of 12 to 18 includes an unsaturated bond, the number of unsaturated bonds may be not less than 1 and not more than 5, for example. Note that an isomer formed as a result of a carbon-carbon double bond being present in the chain aliphatic hydrocarbon group having a carbon number of 12 to 18 may be a cis isomer or may be a trans isomer.

Examples of possible substituents of the chain aliphatic hydrocarbon groups having a carbon number of 12 to 18 that constitute $R^a$, $R^b$, $R^c$, and $R^d$ include alkyl groups having a carbon number of 1 to 3, halogen atoms, and the like, for example.

Furthermore, $R^a$, $R^b$, $R^c$, and $R^d$ are each, independently of one another, preferably any chain aliphatic hydrocarbon group having a carbon number of 15 among:

$-(CH_2)_{14}CH_3$;

$-(CH_2)_7CH=CH(CH_2)_5CH_3$;

$-(CH_2)_7CH=CHCH_2CH=CH(CH_2)_2CH_3$;

$-(CH_2)_7CH=CHCH_2CH=CHCH=CHCH_3$; and

$-(CH_2)_7CH=CHCH_2CH=CHCH_2CH=CH_2$, for example.

Note that one or more hydrogen atoms of the aforementioned chain aliphatic hydrocarbon group having a carbon number of 15 may be substituted.

<Production method of compound>

The presently disclosed compound can be synthesized through a combination of known synthesis reactions without any specific limitations. Examples of literature describing such known synthesis reactions include Organic Functional Group Preparations Vol. I and Vol. II (Sandler & Karo; Hirokawa Shoten) and March's Advanced Organic Chemistry Sixth Edition (Michael B. Smith and Jerry March; WILEY).

The presently disclosed compound represented by formula (1) can typically be produced through appropriate bonding and/or modification of a plurality of compounds serving as raw materials through a formation reaction of an ether bond (-O-), an ester bond (-C(=O)-O- or -O-C(=O)-), a urethane bond ($-C(=O)-NR^1-$ or $-NR^2-C(=O)-$), or the like. Note that $R^1$ and $R^2$ represent the same as previously described.

Formation of an ether bond can, more specifically, be performed as follows, for example.

(i) A compound represented by a formula D1-hal (hal represents a halogen atom; same applies below) and a compound represented by a formula D2-OMet (Met represents an alkali metal (mainly sodium); same applies below) are mixed and caused to undergo condensation (Williamson synthesis). Note that D1 and D2 in the formula represent any organic groups (same applies below).

(ii) A compound represented by a formula D1-hal and a compound represented by a formula D2-OH are mixed and caused to undergo condensation in the presence of a base such as sodium hydroxide or potassium hydroxide.

(iii) A compound represented by a formula D1-J (J represents an epoxy group) and a compound represented by a formula D2-OH are mixed and caused to undergo condensation in the presence of a base such as sodium hydroxide or potassium hydroxide.

(iv) A compound represented by a formula D1-OFN (OFN represents a group including an unsaturated bond) and a compound represented by a formula D2-OMet are mixed and caused to undergo an addition reaction in the presence of a base such as sodium hydroxide or potassium hydroxide

(v) A compound represented by a formula D1-hal and a compound represented by a formula D2-OMet are mixed and caused to undergo condensation in the presence of copper or cuprous chloride (Ullmann condensation).

Formation of an ester bond can, more specifically, be performed as follows, for example.

(vi) A compound represented by a formula D1-COOH and a compound represented by a formula D2-OH are caused to undergo dehydration condensation in the presence of a dehydration condensation agent (for example, WSC (1-ethyl-3-(3-dimethylaminopropyl)carbodiimide hydrochloride)).

(vii) A halogenating agent is caused to act on a compound represented by a formula D1-COOH to obtain a compound represented by a formula D1-C(=O)-hal, and then this compound and a compound represented by a formula D2-OH are caused to react in the presence of a base.

(viii) An acid anhydride is caused to act on a compound represented by a formula D1-COOH to obtain a mixed acid anhydride, and then a compound represented by a formula D2-OH is caused to react with this compound.

(ix) A compound represented by a formula D1-COOH and a compound represented by a formula D2-OH are caused to undergo dehydration condensation in the presence of an acid catalyst or a base catalyst.

Formation of a urethane bond can, more specifically, be performed as follows, for example.

(x) A compound represented by a formula D1-N=C=O and a compound represented by a formula D2-OH are caused to react in the presence of a base catalyst.

The presently disclosed compound can, for example, be produced through a reaction illustrated below.

[Chem. 9]

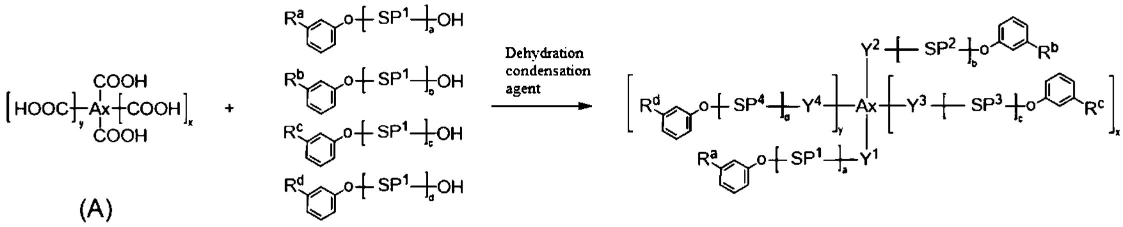


(A)

(B)

(In the formula, Ax, $Y^1$ to $Y^4$, $R^1$, $R^2$, $SP^1$ to $SP^4$, a, b, c, d, $R^a$, $R^b$, $R^c$, $R^d$, x, and y represent the same as previously described.)

In other words, the presently disclosed compound represented by formula (1) that is a target can be produced with high selectivity and high yield by reacting a carboxyl group-containing compound represented by formula (A) with hydroxyl group-containing compounds represented by formula (B) in proportions such that a ratio of "number of moles of carboxyl groups included in compound represented by formula (A):number of moles of hydroxyl groups included in compounds represented by formula (B)" is normally 1:2 to 2:1, preferably 1:1.5 to 1.5:1, and more preferably 1:1.0 to 1:1.5.

Examples of dehydration condensation agents that may be used in this case include carbodiimides such as 1-cyclohexyl-3-(2-morpholinoethyl)carbodiimide metho-p-toluenesulfonate, dicyclohexylcarbodiimide, 1-ethyl-3-(3-dimethylaminopropyl)carbodiimide, 1-ethyl-3-(3-dimethylaminopropyl)carbodiimide hydrochloride, bis(2,6-diisopropylphenyl)carbodiimide, bis(trimethylsilyl)carbodiimide, and bisisopropylcarbodiimide, 2-methyl-6-nitrobenzoic anhydride, 2,2'-carbonylbis-1H-imidazole, 1,1'-oxalyldiimidazole, diphenylphosphoryl azide, 1-(4-nitrobenzenesulfonyl)-1H-1,2,4-triazole, 1H-benzotriazol-1-yloxytripyrrolidinophosphonium hexafluorophosphate, 1H-benzotriazol-1-yloxytris(dimethylamino)phosphonium hexafluorophosphate, N,N,N',N'-tetramethyl-O-(N-succinimidyl)uronium tetrafluoroborate, N-(1,2,2,2-tetrachloroethoxycarbonyloxy)succinimide, N-carbobenzoxysuccinimide, O-(6-chlorobenzotriazol-1-yl)-N,N,N',N'-tetramethyluronium tetrafluoroborate, O-(6-chlorobenzotriazol-1-yl)-N,N,N',N'-tetramethyluronium hexafluorophosphate, 2-bromo-1-ethylpyridinium tetrafluoroborate, 2-chloro-1,3-dimethylimidazolinium chloride, 2-chloro-1,3-dimethylimidazolinium hexafluorophosphate, 2-chloro-1-methylpyridinium iodide, 2-chloro-1-methylpyridinium p-toluenesulfonate, 2-fluoro-1-methylpyridinium p-toluenesulfonate, and trichloroacetic acid pentachlorophenyl ester.

In terms of reactivity, cost, and useable solvents, dicyclohexylcarbodiimide, 1-ethyl-3-(3-dimethylaminopropyl) carbodiimide, 1-ethyl-3-(3-dimethylaminopropyl)carbodiimide hydrochloride, bis(2,6-diisopropylphenyl)carbodiimide, bis(trimethylsilyl)carbodiimide, bisisopropylcarbodiimide, and 2,2'-carbonylbis-1H-imidazole are more preferable.

The additive amount of the dehydration condensation agent is normally 1 mole to 1.5 moles, preferably 1 mole to 1.2 moles, and more preferably 1 mole to 1.1 moles relative to the number of moles of carboxyl groups included in the compound represented by formula (A). Moreover, the dehydration condensation agent may be added in that form or may be added as a solution after being dissolved or dispersed in a suitable solvent.

An activator can be used in this case. The activator may be 4-(dimethylamino)pyridine (N,N-dimethyl-4-aminopyridine) or the like.

The additive amount of the activator is normally 0.01 moles to 1.0 moles, and preferably 0.01 moles to 0.5 moles relative to the number of moles of the dehydration condensation agent. Moreover, the activator may be added in that form or may be added as a solution after being dissolved or dispersed in a suitable solvent.

A solvent that is used in this reaction may be any solvent without any specific limitations so long as it is inert in the

reaction. Examples of solvents that may be used include ether solvents such as diethyl ether, tetrahydrofuran, 1,2-dimethoxyethane, 1,4-dioxane, cyclopentyl methyl ether, and methyl tert-butyl ether; ester solvents such as ethyl acetate, propyl acetate, methyl propionate, and γ-butyrolactone; aromatic hydrocarbon solvents such as benzene, toluene, and xylene; aliphatic hydrocarbon solvents such as n-pentane, n-hexane, and n-heptane; amide solvents such as N,N-dimethylformamide, N,N-dimethylacetamide, N-methylpyrrolidone, N,N-dimethylimidazolidinone, and hexamethylphosphoric triamide; sulfur-containing solvents such as dimethyl sulfoxide and sulfolane; halogenated solvents such as methylene chloride, chloroform, 1,2-dichloroethane, and chlorobenzene; and mixed solvents of two or more of these solvents. Of these solvents, ether solvents, ester solvents, amide solvents, and halogenated solvents are preferable, and, in particular, amide solvents are preferable, and N-methylpyrrolidone, N,N-dimethylacetamide, and N,N-dimethylformamide are particularly preferable.

The used amount of the solvent is not specifically limited and can be set as appropriate in consideration of the types of compounds that are used, the scale of the reaction, and so forth, but is normally 1 g to 100 g relative to 1 g of the carboxyl group-containing compound represented by formula (A).

Although the reaction can proceed smoothly in a temperature range from -10°C to the boiling point of the solvent that is used, the temperature is preferably 10°C to 80°C, and more preferably 15°C to 50°C. The reaction time of each reaction depends on the scale of the reaction, but is normally a few minutes to tens of hours, preferably a few minutes to 70 hours, and more preferably a few hours to 30 hours.

The presently disclosed compound can also be produced through a reaction illustrated below, for example.

[Chem. 10]

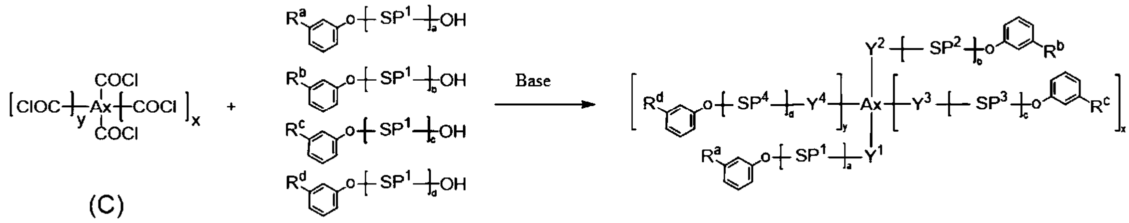


(C)

(B)

(In the formula, Ax, $Y^1$ to $Y^4$, $R^1$, $R^2$, $SP^1$ to $SP^4$, a, b, c, d, $R^a$, $R^b$, $R^c$, $R^d$, x, and y represent the same as previously described.)

In other words, the presently disclosed compound represented by formula (1) that is a target can be produced with high selectivity and high yield by reacting an acid chloride group-containing compound represented by formula (C) with hydroxyl group-containing compounds represented by formula (B) in proportions such that a ratio of "number of moles of hydroxyl groups included in compounds represented by formula (B):number of moles of acid chloride groups included in compound represented by formula (C)" is normally 1:2 to 2:1, preferably 1:1.5 to 1.5:1, and more preferably 1:1.0 to 1:1.2.

In this case, the used base may be an organic base such as triethylamine, diisopropylethylamine, pyridine, 4-(dimethylamino)pyridine (N,N-dimethyl-4-aminopyridine), or 2,6-lutidine; or an inorganic base such as sodium hydroxide, sodium carbonate, or sodium hydrogen carbonate.

An activator can be used in this case. The activator may be 4-(dimethylamino)pyridine (N,N-dimethyl-4-aminopyridine) or the like.

The additive amount of the activator is normally 0.01 moles to 1.0 moles, preferably 0.01 moles to 0.5 moles, and more preferably 0.01 moles to 0.2 moles relative to the acid chloride group-containing compound represented by formula (C). Moreover, the activator may be added in that form or may be added as a solution after being dissolved or dispersed in a suitable solvent.

A solvent that is used in this reaction may be any solvent without any specific limitations so long as it is inert in the reaction. Examples of solvents that may be used include ether solvents such as diethyl ether, tetrahydrofuran, 1,2-dimethoxyethane, 1,4-dioxane, cyclopentyl methyl ether, and methyl tert-butyl ether; ester solvents such as ethyl acetate, propyl acetate, methyl propionate, and γ-butyrolactone; aromatic hydrocarbon solvents such as benzene, toluene, and xylene; aliphatic hydrocarbon solvents such as n-pentane, n-hexane, and n-heptane; amide solvents such as N,N-dimethylformamide, N,N-dimethylacetamide, N-methylpyrrolidone, N,N-dimethylimidazolidinone, and hexamethylphosphoric triamide; sulfur-containing solvents such as dimethyl sulfoxide and sulfolane; halogenated solvents such as methylene chloride, chloroform, 1,2-dichloroethane, and chlorobenzene; and mixed solvents of two or more of these solvents. Of these solvents, ether solvents, ester solvents, and halogenated solvents are preferable, ether solvents and halogenated solvents are more preferable, and tetrahydrofuran, 1,2-dimethoxyethane, cyclopentyl methyl ether, methyl

tert-butyl ether, chloroform, and 1,2-dichloroethane are particularly preferable.

The used amount of the solvent is not specifically limited and can be set as appropriate in consideration of the types of compounds that are used, the scale of the reaction, and so forth, but is normally 1 g to 100 g relative to 1 g of the carboxyl group-containing compound represented by formula (A).

Although the reaction can proceed smoothly in a temperature range from -20°C to the boiling point of the solvent that is used, the temperature is preferably -5°C to 50°C, and more preferably 0°C to 40°C. The reaction time of each reaction depends on the scale of the reaction, but is normally a few minutes to ten-odd hours, preferably a few minutes to 10 hours, and more preferably tens of minutes to 5 hours.

In this case, a purchased compound may be used as the acid chloride group-containing compound represented by formula (C), or a compound synthesized from the carboxyl group-containing compound represented by formula (A) may be used as the acid chloride group-containing compound represented by formula (C).

The acid chloride group-containing compound represented by formula (C) can be produced as follows.

[Chem. 11]

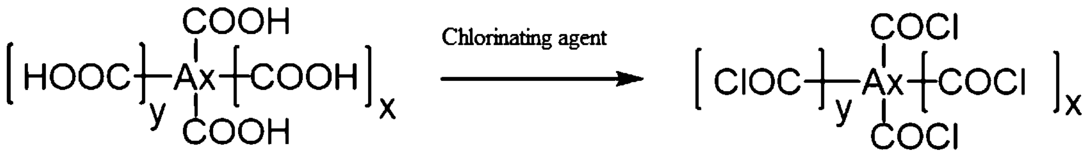


(A) (C)

(In the formula, x and y represent the same as previously described.)

In this case, the used chlorinating agent may be thionyl chloride ($SOCl_2$), oxalyl chloride ($(C(=O)Cl)_2$), sulfuryl chloride ($SO_2Cl_2$), phosphoryl chloride ($POCl_3$), phosphorus trichloride ($PCl_3$), phosphorus pentachloride ($PCl_5$), or the like, and is preferably thionyl chloride, oxalyl chloride, or sulfuryl chloride. One of these chlorinating agents can be used individually, or two or more of these chlorinating agents can be used in combination.

The acid chloride compound represented by formula (C) that is a target can be produced with high selectivity and high yield by reacting the used chlorinating agent with the carboxyl group-containing compound represented by formula (A) in proportions such that a ratio of "number of moles of carboxyl groups included in compound represented by formula (A):chlorinating agent" is normally 1:2 to 2:1, preferably 1:5 to 1:3, and more preferably 1:1.0 to 1:1.5.

An activator can be used in this case. The activator may be N,N-dimethylformamide, triethylamine, tetraalkylammonium, or the like, and is preferably N,N-dimethylformamide or tetraalkylammonium.

The additive amount of the activator is normally 0.01 moles to 3.0 moles, preferably 0.01 moles to 2.0 moles, and more preferably 0.01 moles to 0.5 moles relative to the carboxyl group-containing compound represented by formula (A).

A solvent that is used in this reaction may be any solvent without any specific limitations so long as it is inert in the reaction. Examples of solvents that may be used include ether solvents such as diethyl ether, tetrahydrofuran, 1,2-dimethoxyethane, 1,4-dioxane, cyclopentyl methyl ether, and methyl tert-butyl ether; ester solvents such as ethyl acetate, propyl acetate, methyl propionate, and γ-butyrolactone; aromatic hydrocarbon solvents such as benzene, toluene, and xylene; aliphatic hydrocarbon solvents such as n-pentane, n-hexane, and n-heptane; amide solvents such as N,N-dimethylformamide, N,N-dimethylacetamide, N-methylpyrrolidone, N,N-dimethylimidazolidinone, and hexamethylphosphoric triamide; sulfur-containing solvents such as dimethyl sulfoxide and sulfolane; halogenated solvents such as methylene chloride, chloroform, 1,2-dichloroethane, and chlorobenzene; and mixed solvents of two or more of these solvents. Of these solvents, ether solvents, ester solvents, and halogenated solvents are preferable, ether solvents and halogenated solvents are more preferable, and tetrahydrofuran, 1,2-dimethoxyethane, cyclopentyl methyl ether, methyl tert-butyl ether, chloroform, and 1,2-dichloroethane are particularly preferable.

The used amount of the solvent is not specifically limited and can be set as appropriate in consideration of the types of compounds that are used, the scale of the reaction, and so forth, but is normally 1 g to 100 g relative to 1 g of the carboxyl group-containing compound represented by formula (A).

Although the reaction can proceed smoothly in a temperature range from -20°C to the boiling point of the solvent that is used, the temperature is preferably -5°C to 150°C, and more preferably 0°C to 110°C. The reaction time of each reaction depends on the scale of the reaction, but is normally a few minutes to ten-odd hours, preferably a few minutes to 10 hours, and more preferably tens of minutes to 5 hours.

Cardanol and derivatives thereof that are produced from components extracted from cashew nut shell oil can suitably be used as the compounds represented by formula (B).

Any cardanol and hydroxyl group-containing cardanol derivatives can be used without any specific limitations,

and although the following gives specific examples thereof from Cardolite Corporation, any equivalent products from other companies can be used in the same manner.

The cardanol and cardanol derivatives may be produced by Cardolite Corporation, for example, and specific examples thereof, in terms of names of products from Cardolite Corporation, include NX-2021, NX-2022, NX-2023, NX-2023D, NX-2024, NX-2025, NX-2026, NX-5205, Ultra LITE 2023, UL-2023, NC-510 (hydrogenated product), LITE 2020 (ethylene oxide monoadduct), NX-7507 (ethylene oxide heptaadduct), GX-5166 (ethylene oxide heptaadduct), GX-5167 (ethylene oxide nonaadduct), GX-5170 (ethylene oxide dodecaadduct), LITE 2100, and LITE 2100R.

Of these examples, NX-2021, NX-2022, NX-2023, NX-2023D, NX-2024, NX-2025, NX-2026, NX-5205, Ultra LITE 2023, UL-2023, NC-510 (hydrogenated product), LITE 2020 (ethylene oxide monoadduct), NX-7507 (ethylene oxide heptaadduct), GX-5166 (ethylene oxide heptaadduct), GX-5167 (ethylene oxide nonaadduct), and GX-5170 (ethylene oxide dodecaadduct) are preferable, and NX-2021, NX-2022, NX-2023, NX-2024, NX-2025, NX-2026, Ultra LITE 2023, NC-510 (hydrogenated product), LITE 2020 (ethylene oxide monoadduct), NX-7507 (ethylene oxide heptaadduct), GX-5166 (ethylene oxide heptaadduct), GX-5167 (ethylene oxide nonaadduct), and GX-5170 (ethylene oxide dodecaadduct) are more preferable.

(Method of using compound)

The presently disclosed compound can be used as a plasticizer in production of a resin composition that is to be used to form a resin molded product and can impart flexibility and elasticity to the resin molded product, for example, but is not specifically limited to being used in this manner. By using the presently disclosed compound as a plasticizer, it is possible to sufficiently improve low-temperature tensile elongation and heat shrinkage resistance of a resin molded product obtained through molding of a resin composition.

Note that in a situation in which the presently disclosed compound is used as a plasticizer, two or more types of the presently disclosed compound that have different structures to each other can be used in combination. In other words, a mixture containing two or more types of the presently disclosed compound having different structures to each other can be used as a plasticizer.

(Plasticizer composition)

A feature of the presently disclosed plasticizer composition (hereinafter, also referred to simply as a "plasticizer composition") is that it contains at least the presently disclosed compound set forth above. By using a plasticizer composition that contains the presently disclosed compound in production of a resin composition, it is possible to sufficiently improve low-temperature tensile elongation and heat shrinkage resistance of a resin molded product that is formed from the produced resin composition.

The presently disclosed plasticizer composition may further contain a plasticizer other than the presently disclosed compound set forth above. In particular, it is preferable that the presently disclosed plasticizer composition further contains a polyester plasticizer in addition to the presently disclosed compound set forth above. Moreover, the presently disclosed plasticizer composition may further contain plasticizers other than the presently disclosed compound set forth above and the polyester plasticizer (hereinafter, also referred to as "other plasticizers").

<Presently disclosed compound>

A single compound having a single structure may be used individually as the presently disclosed compound that is used in production of the plasticizer composition, or two or more types of compounds having different structures to each other may be used in combination in a freely selected ratio as the presently disclosed compound that is used in production of the plasticizer composition.

In a case in which the plasticizer composition contains a polyester plasticizer, the content of the presently disclosed compound in the plasticizer composition is preferably 1 part by mass or more, more preferably 1.5 parts by mass or more, even more preferably 2 parts by mass or more, further preferably 2.5 parts by mass or more, even further preferably 3 parts by mass or more, and yet further preferably 8 parts by mass or more relative to 100 parts by mass of the polyester plasticizer, and is preferably 20 parts by mass or less, more preferably 18 parts by mass or less, even more preferably 16 parts by mass or less, and further preferably 12 parts by mass or less relative to 100 parts by mass of the polyester plasticizer. When the content of the presently disclosed compound in the plasticizer composition is not less than any of the lower limits set forth above, low-temperature tensile elongation of a formed resin molded product can be further improved, and adhesiveness of the resin molded product to a foamed polyurethane molded product can also be improved. On the other hand, when the content of the presently disclosed compound in the plasticizer composition is not more than any of the upper limits set forth above, sufficiently high heat shrinkage resistance of a formed resin molded product can be ensured.

<Polyester plasticizer>

The polyester plasticizer is a component that can impart adequate tensile characteristics (for example, tensile elongation and tensile strength) to a resin molded product that is formed using a resin composition containing the plasticizer composition. Moreover, in a case in which a polyester plasticizer is used, when a resin molded product that has been formed from a resin composition containing the plasticizer composition is lined with a foamed polyurethane molded product to form a laminate, the polyester plasticizer does not easily migrate from the resin molded product to the foamed polyurethane molded product even at high temperature, thus enabling further improvement of heat shrinkage resistance of the resin molded product.

The polyester plasticizer can be a polyester including a structural unit derived from adipic acid (adipic acid polyester), a polyester including a structural unit derived from sebacic acid (sebacic acid polyester), a polyester including a structural unit derived from phthalic acid (phthalic acid polyester), or any other such polyester, for example, without any specific limitations. Note that one of these polyesters may be used individually, or two or more of these polyesters may be used in a freely selected ratio as a mixture.

In particular, it is preferable to use an adipic acid polyester (polyester including a structural unit derived from adipic acid) as the polyester plasticizer from a viewpoint of even further improving heat shrinkage resistance of a resin molded product.

The viscosity of the polyester plasticizer is preferably 500 mPa·s or more, and more preferably 1,000 mPa·s or more, and is preferably 8,000 mPa·s or less, and more preferably 5,000 mPa·s or less.

Note that the "viscosity" can be measured in accordance with JIS Z8803 at a temperature of 25°C.

<Other plasticizers>

The plasticizer composition may optionally further contain other plasticizers besides the presently disclosed compound and the polyester plasticizer described above.

Specific examples of other plasticizers include plasticizers described in WO2016/098344A1 with the exception of (b1) polyester plasticizers and (b2) phenols and modified products thereof that have been described above. Of these other plasticizers, it is preferable to use epoxidized vegetable oil from a viewpoint of further improving low-temperature tensile elongation of a formed resin molded product, and more preferable to use epoxidized soybean oil.

The content of other plasticizers in the plasticizer composition is not specifically limited, but can be set as not less than 0 parts by mass and not more than 15 parts by mass relative to 100 parts by mass of the polyester plasticizer.

In a case in which epoxidized vegetable oil such as epoxidized soybean oil is used as another plasticizer, from a viewpoint of even further improving low-temperature tensile elongation of a formed resin molded product while also ensuring sufficiently high heat shrinkage resistance of the resin molded product, the content of the epoxidized vegetable oil as another plasticizer is preferably 2 parts by mass or more, more preferably 3 parts by mass or more, and even more preferably 4 parts by mass or more relative to 100 parts by mass of the polyester plasticizer, and is preferably 10 parts by mass or less, more preferably 7 parts by mass or less, and even more preferably 5 parts by mass or less relative to 100 parts by mass of the polyester plasticizer.

(Resin composition)

A feature of the presently disclosed resin composition is that it contains: (a) a resin; and (b) the presently disclosed plasticizer composition (hereinafter, also referred to simply as the "(b) plasticizer composition"). In other words, the presently disclosed resin composition contains at least the (a) resin and the presently disclosed compound set forth above, and optionally further contains a polyester plasticizer and other plasticizers.

Note that the presently disclosed resin composition may optionally further contain additives other than the (a) resin and the (b) plasticizer composition described above.

A resin molded product that has been formed using the presently disclosed resin composition has excellent low-temperature tensile elongation.

Moreover, in a situation in which a resin molded product that has been formed using the presently disclosed resin composition is lined with a foamed polyurethane molded product to produce a laminate, heat shrinkage of the resin molded product in the laminate can be sufficiently inhibited. In other words, a resin molded product that has been formed using the presently disclosed resin composition also has excellent heat shrinkage resistance.

Consequently, by using the presently disclosed resin composition, it is possible to obtain a resin molded product that is suitable as an automobile interior material, such as a surface skin for an automobile instrument panel or a surface skin for a door trim, having excellent low-temperature tensile elongation and heat shrinkage resistance.

Note that from a viewpoint of easily obtaining a resin molded product that be used well as an automobile interior material using the presently disclosed resin composition, for example, the presently disclosed resin composition is

preferably used in powder molding, and is more preferably used in powder slush molding.

<(a) Resin>

A known resin can be used as the (a) resin without any specific limitations so long as it is a resin with which the desired effects according to the present disclosure are obtained.

Note that the (a) resin contained in the presently disclosed resin composition is considered to be a different component to plasticizers such as the presently disclosed compound and the polyester plasticizer that are contained in the (b) plasticizer composition and also to additives that are optional components.

The glass-transition temperature of the (a) resin is preferably not lower than 50°C and not higher than 100°C.

Moreover, the (a) resin is preferably a resin that contains a halogen, more preferably a resin that contains fluorine or a resin that contains chlorine, even more preferably a resin that contains chlorine, and particularly preferably a vinyl chloride resin.

The proportional content of the vinyl chloride resin among the (a) resin is not specifically limited but is preferably 50 mass% or more, more preferably 70 mass% or more, even more preferably 90 mass% or more, and particularly preferably 100 mass%.

<<Vinyl chloride resin>>

A particulate vinyl chloride resin is normally used as the vinyl chloride resin. For example, one type or two or more types of vinyl chloride resin particles can be included as the vinyl chloride resin, and one type or two or more types of vinyl chloride resin fine particles can optionally be further included as the vinyl chloride resin. In particular, the vinyl chloride resin preferably includes at least vinyl chloride resin particles, and more preferably includes vinyl chloride resin particles and vinyl chloride resin fine particles.

The vinyl chloride resin can be produced by a conventionally known production method such as suspension polymerization, emulsion polymerization, solution polymerization, or bulk polymerization.

In the present specification, the term "resin particles" is used to refer to particles having a particle diameter of 30 μm or more, whereas the term "resin fine particles" is used to refer to particles having a particle diameter of less than 30 μm.

Examples of the vinyl chloride resin include homopolymers composed of vinyl chloride monomer units and vinyl chloride copolymers preferably comprising 50 mass% or more of vinyl chloride monomer units, and more preferably comprising 70 mass% or more of vinyl chloride monomer units. Specific examples of monomers (comonomers) that are copolymerizable with vinyl chloride monomer and can be used to form a vinyl chloride copolymer include monomers described in WO2016/098344A1, for example. One of these components may be used individually, or two or more of these components may be used in combination in a freely selected ratio.

[Vinyl chloride resin particles]

In the resin composition, the vinyl chloride resin particles normally function as a matrix resin (base material). The vinyl chloride resin particles are preferably produced by suspension polymerization.

-Average degree of polymerization-

The average degree of polymerization of a vinyl chloride resin forming the vinyl chloride resin particles is preferably 800 or more, and more preferably 1,000 or more, and is preferably 5,000 or less, more preferably 3,000 or less, and even more preferably 2,800 or less. When the average degree of polymerization of the vinyl chloride resin forming the vinyl chloride resin particles is not less than any of the lower limits set forth above, physical strength of a resin molded product formed using the resin composition can be sufficiently ensured while also improving tensile characteristics (particularly tensile elongation) of the resin molded product, for example. A resin molded product having good tensile elongation can suitably be used as an automobile interior material, such as a surface skin of an automobile instrument panel, that has excellent ductility and that ruptures as designed without scattering of fragments when an airbag expands and is deployed, for example. On the other hand, when the average degree of polymerization of the vinyl chloride resin forming the vinyl chloride resin particles is not more than any of the upper limits set forth above, meltability of the resin composition can be improved.

The "average degree of polymerization" referred to in the present disclosure can be measured in accordance with JIS K6720-2.

-Average particle diameter-

The average particle diameter of the vinyl chloride resin particles is normally 30 μm or more, preferably 50 μm or more, and more preferably 100 μm or more, and is preferably 500 μm or less, and more preferably 200 μm or less. When the average particle diameter of the vinyl chloride resin particles is not less than any of the lower limits set forth above, powder fluidity of the resin composition can be improved. On the other hand, when the average particle diameter of the vinyl chloride resin particles is not more than any of the upper limits set forth above, meltability of the resin composition can be improved, and surface smoothness of a formed resin molded product can be improved.

The "average particle diameter" referred to in the present disclosure can be measured as the volume-average particle diameter by laser diffraction in accordance with JIS Z8825.

-Proportional content-

The proportional content of the vinyl chloride resin particles in the vinyl chloride resin is preferably 70 mass% or more, and more preferably 80 mass% or more, can be set as 100 mass%, and is preferably 95 mass% or less, and more preferably 90 mass% or less. When the proportional content of the vinyl chloride resin particles in the vinyl chloride resin is not less than any of the lower limits set forth above, physical strength of a resin molded product formed using the resin composition can be sufficiently ensured while also improving tensile elongation of the resin molded product. On the other hand, when the proportional content of the vinyl chloride resin particles in the vinyl chloride resin is not more than any of the upper limits set forth above, powder fluidity of the resin composition can be improved.

[Vinyl chloride resin fine particles]

In the resin composition, the vinyl chloride resin fine particles typically function as a dusting agent (powder fluidity modifier). The vinyl chloride resin fine particles are preferably produced by emulsion polymerization.

-Average degree of polymerization-

The average degree of polymerization of a vinyl chloride resin forming the vinyl chloride resin fine particles is preferably 500 or more, and more preferably 700 or more, and is preferably 2,600 or less, and more preferably 2,400 or less. When the average degree of polymerization of the vinyl chloride resin forming the vinyl chloride resin fine particles that serve as a dusting agent is not less than any of the lower limits set forth above, powder fluidity of the resin composition can be improved, and tensile elongation of a formed resin molded product can also be improved. On the other hand, when the average degree of polymerization of the vinyl chloride resin forming the vinyl chloride resin fine particles is not more than any of the upper limits set forth above, meltability of the resin composition can be improved, and surface smoothness of a formed resin molded product can be improved.

-Average particle diameter-

The average particle diameter of the vinyl chloride resin fine particles is normally less than 30 μm, preferably 10 μm or less, and more preferably 5 μm or less, and is preferably 0.1 μm or more, and more preferably 1 μm or more. When the average particle diameter of the vinyl chloride resin fine particles is not less than any of the lower limits set forth above, the vinyl chloride resin fine particles are not too small to function as a dusting agent, for example, and powder fluidity of the resin composition can be improved. On the other hand, when the average particle diameter of the vinyl chloride resin fine particles is not more than any of the upper limits set forth above, meltability of the resin composition can be improved, and surface smoothness of a formed resin molded product can be improved.

-Proportional content-

The proportional content of the vinyl chloride resin fine particles in the vinyl chloride resin may be 0 mass%, but is preferably 5 mass% or more, and more preferably 10 mass% or more, and is preferably 30 mass% or less, and more preferably 20 mass% or less. When the proportional content of the vinyl chloride resin fine particles in the vinyl chloride resin is not less than any of the lower limits set forth above, powder fluidity of the resin composition can be improved. On the other hand, when the proportional content of the vinyl chloride resin fine particles in the vinyl chloride resin is not more than any of the upper limits set forth above, physical strength of a formed resin molded product can be increased.

<(b) Plasticizer composition>

A plasticizer composition that contains at least the presently disclosed compound set forth above and that optionally further contains a polyester plasticizer and other plasticizers is used as the (b) plasticizer composition.

The content of the presently disclosed compound in the resin composition is preferably 1 part by mass or more, more preferably 1.5 parts by mass or more, even more preferably 2 parts by mass or more, further preferably 2.5 parts by mass or more, even further preferably 3 parts by mass or more, and yet further preferably 8 parts by mass or more relative to 100 parts by mass of the (a) resin, and is preferably 20 parts by mass or less, more preferably 18 parts by mass or less, even more preferably 16 parts by mass or less, and further preferably 12 parts by mass or less relative to 100 parts by mass of the (a) resin. When the content of the presently disclosed compound in the resin composition is not less than any of the lower limits set forth above, low-temperature tensile elongation of a formed resin molded product can be further improved, and adhesiveness of the resin molded product to a foamed polyurethane molded product can also be improved. On the other hand, when the content of the presently disclosed compound in the resin composition is not more than any of the upper limits set forth above, sufficiently high heat shrinkage resistance of a formed resin molded product can be ensured.

In a case in which the (b) plasticizer composition contains a polyester plasticizer, the content of the polyester plasticizer in the resin composition is preferably 30 parts by mass or more, more preferably 50 parts by mass or more, even more preferably 70 parts by mass or more, further preferably 80 parts by mass or more, and even further preferably 85 parts by mass or more relative to 100 parts by mass of the (a) resin, and is preferably 200 parts by mass or less, more preferably 180 parts by mass or less, even more preferably 150 parts by mass or less, further preferably 130 parts by mass or less, even further preferably 110 parts by mass or less, and particularly preferably 100 parts by mass or less relative to 100 parts by mass of the (a) resin. When the content of the polyester plasticizer in the resin composition is not less than any of the lower limits set forth above, heat shrinkage resistance of a formed resin molded product can be further improved. On the other hand, when the content of the polyester plasticizer in the resin composition is not more than any of the upper limits set forth above, sufficiently high adhesiveness of a formed resin molded product to a foamed polyurethane molded product can be ensured.

The content of other plasticizers in the resin composition is not specifically limited, but can be set as not less than 0 parts by mass and not more than 15 parts by mass relative to 100 parts by mass of the (a) resin.

In a case in which epoxidized vegetable oil such as epoxidized soybean oil is used as another plasticizer, from a viewpoint of even further improving low-temperature tensile elongation of a formed resin molded product while also ensuring sufficiently high heat shrinkage resistance of the resin molded product, the content of the epoxidized vegetable oil as another plasticizer is preferably 2 parts by mass or more, more preferably 3 parts by mass or more, and even more preferably 4 parts by mass or more relative to 100 parts by mass of the (a) resin, and is preferably 10 parts by mass or less, and more preferably 7 parts by mass or less relative to 100 parts by mass of the (a) resin.

Note that the total content of the presently disclosed compound, the polyester plasticizer, and other plasticizers (i.e., the content of the (b) plasticizer composition) in the resin composition is preferably 31 parts by mass or more, more preferably 53.5 parts by mass or more, even more preferably 75 parts by mass or more, further preferably 86.5 parts by mass or more, and even further preferably 92 parts by mass or more relative to 100 parts by mass of the resin, and is preferably 235 parts by mass or less, more preferably 208 parts by mass or less, even more preferably 176 parts by mass or less, further preferably 154 parts by mass or less, even further preferably 129 parts by mass or less, and particularly preferably 119 parts by mass or less relative to 100 parts by mass of the resin. When the content of the plasticizer composition in the resin composition is within any of the specific ranges set forth above, it is easy to perform molding (for example, powder molding) of the resin composition, and a balance of high levels of low-temperature tensile elongation, heat shrinkage resistance, and adhesiveness to a foamed polyurethane molded product of a formed resin molded product can be achieved.

<Additives>

The presently disclosed resin composition may further contain various additives besides the components described above. Examples of additives that may be used include, but are not specifically limited to, lubricants; stabilizers such as perchloric acid-treated hydrotalcite, zeolites, β-diketones, and fatty acid metal salts; mold release agents; dusting agents other than the previously described vinyl chloride resin fine particles; impact modifiers; perchloric acid compounds other than perchloric acid-treated hydrotalcite (sodium perchlorate, potassium perchlorate, etc.); antioxidants; fungicides; flame retardants; antistatic agents; fillers; light stabilizers; foaming agents; and pigments.

Additives that are described in WO2016/098344A1, for example, can be used as the aforementioned additives that can be contained in the presently disclosed resin composition, and suitable amounts of these additives can also be the same as described in WO2016/098344A1.

<Production method of resin composition>

The presently disclosed resin composition can be produced by mixing the components described above.

The mixing method of the above-described (a) resin, (b) plasticizer composition, and various additives that are further compounded as necessary may be a method in which components other than a dusting agent (inclusive of the vinyl chloride resin fine particles) are mixed by dry blending and then the dusting agent is subsequently added and mixed, for example, but is not specifically limited thereto. The dry blending is preferably carried out using a Henschel mixer. Although the temperature during dry blending is not specifically limited, the temperature is preferably 50°C or higher, and more preferably 70°C or higher, and is preferably 200°C or lower.

<Use of resin composition>

The obtained resin composition can be suitably used in powder molding, and more suitably used in powder slush molding.

(Resin molded product)

A feature of the presently disclosed resin molded product is that it is obtained through molding of the resin composition set forth above by any method. As a result of the presently disclosed resin molded product being formed using the resin composition set forth above, the presently disclosed resin molded product normally contains at least the (a) resin and the (b) plasticizer composition. In other words, the presently disclosed resin molded product normally contains at least the (a) resin and the presently disclosed compound, and optionally further contains a polyester plasticizer, other plasticizers, and additives. Moreover, the presently disclosed resin molded product has excellent low-temperature tensile elongation and heat shrinkage resistance.

Consequently, the presently disclosed resin molded product can suitably be used as an automobile interior material such as a surface skin of an automobile instrument panel.

<Formation method of resin molded product>

Although no specific limitations are placed on the mold temperature in powder slush molding in a situation in which the resin molded product is formed by powder slush molding, the mold temperature is preferably 200°C or higher, and more preferably 220°C or higher, and is preferably 300°C or lower, and more preferably 280°C or lower.

The following method, for example, may be used in production of the resin molded product without any specific limitations. In this method, the presently disclosed resin composition is sprinkled onto a mold having a temperature within any of the ranges set forth above. The resin composition is initially left for not less than 5 seconds and not more than 30 seconds and, after shaking off any excess resin composition, is then further left for not less than 30 seconds and not more than 3 minutes at an arbitrary temperature. The mold is subsequently cooled to a temperature of not lower than 10°C and not higher than 60°C, and the presently disclosed resin molded product that is obtained is removed from the mold. A sheet-shaped molded product that imitates the shape of the mold is obtained.

(Laminate)

The presently disclosed laminate includes a foamed polyurethane molded product and the resin molded product set forth above. The resin molded product typically constitutes one surface of the laminate.

Moreover, the presently disclosed laminate includes a resin molded product that has been formed using the presently disclosed resin composition and that has excellent low-temperature tensile elongation and heat shrinkage resistance, for example. Consequently, the presently disclosed laminate may suitably be used as an automobile interior material forming an automobile interior component (particularly an automobile instrument panel).

The method by which the foamed polyurethane molded product and the resin molded product are stacked is not specifically limited and may, for example, be a method such as described below. Specifically, (1) a method in which the foamed polyurethane molded product and the resin molded product are separately prepared and are subsequently adhered to each other by thermal fusion bonding, thermal adhesion, or using a commonly known adhesive, or (2) a method in which raw materials of the foamed polyurethane molded product such as an isocyanate and a polyol are caused to react and polymerize on the resin molded product while carrying out polyurethane foaming by a commonly known method to directly form the foamed polyurethane molded product on the resin molded product may be adopted. The latter method (2) is more suitable because it involves a simple process and enables laminates of various different shapes to be obtained while easily achieving strong adhesion of the resin molded product and the foamed polyurethane molded product.

## EXAMPLES

The following provides a more specific description of the present disclosure based on examples. However, the present disclosure is not limited to the following examples. In the following description, "%" and "parts" used in expressing quantities are by mass, unless otherwise specified.

Moreover, the following methods were used to measure and evaluate the tensile characteristics at low temperature, heat shrinkage resistance, and adhesiveness to a foamed polyurethane molded product of a vinyl chloride resin molded sheet (vinyl chloride resin molded product).

<Tensile characteristics at low temperature (tensile strength and tensile elongation)>

An obtained vinyl chloride resin molded sheet was punched out with a No. 1 dumbbell described in JIS K6251, and then tensile breaking elongation (%) and tensile breaking stress (MPa) at a low temperature of -25°C were measured in accordance with JIS K7113 at a tensing rate of 200 mm/min. A larger value for tensile breaking elongation indicates that the vinyl chloride resin molded sheet has better tensile elongation at low temperature (low-temperature tensile elongation) in an initial (unheated) state. Moreover, a larger value for tensile breaking stress indicates that the vinyl chloride resin molded sheet has better tensile strength at low temperature in an initial (unheated) state.

<Heat shrinkage resistance>

A 3D coordinate measuring instrument (Crysta-Plus M443 produced by Mitutoyo Corporation) was used to measure the transverse direction length of a vinyl chloride resin molded sheet in an obtained laminate so as to determine an actual measurement value for before heating.

Thereafter, the laminate was stored and heated in a 120°C Geer-type oven (Geer Oven produced by Toyo Seiki Seisaku-Sho, Ltd.). Once 600 hours had passed, the laminate was removed from the oven, and the transverse direction length of the vinyl chloride resin molded sheet in the laminate was measured by the 3D coordinate measuring instrument to determine an actual measurement value for after heating. The actual measurement values for before and after heating were used to calculate a heat shrinkage rate (%) indicated by the following formula.

Heat shrinkage rate [%] = 100 $\times$ (Actual measurement value for before heating - Actual measurement value for after heating)/Actual measurement value for before heating

Note that a smaller value for the heat shrinkage rate indicates that there is less heat shrinkage of the vinyl chloride resin molded sheet and that the vinyl chloride resin molded sheet has better heat shrinkage resistance.

<Adhesiveness to foamed polyurethane molded product>

Using an obtained laminate in which a vinyl chloride resin molded sheet was lined with a foamed polyurethane molded product, the vinyl chloride resin molded sheet was peeled from the foamed polyurethane molded product. After peeling, a surface of the vinyl chloride resin molded sheet that had been lined with the foamed polyurethane molded product among surfaces of the vinyl chloride resin molded sheet was visually checked, and adhesiveness of the vinyl chloride resin molded sheet (vinyl chloride resin molded product) to the polyurethane molded product was evaluated.

An evaluation of "foam destruction" (evaluation "A" in Table 1) was given in a case in which a surface portion of the foamed polyurethane molded product remained over the entirety of the visually checked surface of the vinyl chloride resin molded sheet, an evaluation of "partial interfacial peeling" (evaluation "B" in Table 1) was given in a case in which a surface portion of the foamed polyurethane molded product remained at only some locations at the surface of the vinyl chloride resin molded sheet but did not remain at other locations, and an evaluation of "complete interfacial peeling" (evaluation "C" in Table 1) was given in a case in which a surface portion of the foamed polyurethane molded product did not remain at any location at the surface of the vinyl chloride resin molded sheet.

Note that when a surface portion of the foamed polyurethane molded product remains at more locations at the surface of the vinyl chloride resin molded sheet, this indicates that the vinyl chloride resin molded sheet (vinyl chloride resin molded product) has higher adhesiveness to the foamed polyurethane molded product.

(Synthesis examples)

Compounds used in the examples were produced as follows.

Note that spectra obtained through measurement of Cardolite LITE 2020 (produced by Cardolite Corporation)

used in the following synthesis examples by nuclear magnetic resonance (NMR) ($^1$H-NMR, $^{13}$C-NMR, and C-H COSY) and IR (ATR) are illustrated in FIGS. 1 to 4. From the $^1$H-NMR spectrum in FIG. 1, the signal position for a hydroxyl group was determined to be 2.15 ppm.

Moreover, spectra obtained through measurement of Cardolite NX-7507 (produced by Cardolite Corporation) used in the following synthesis examples by NMR ($^1$H-NMR, $^{13}$C-NMR, and C-H COSY) and IR (ATR) are illustrated in FIGS. 5 to 8. From the $^1$H-NMR spectrum in FIG. 5, the signal position for a hydroxyl group was determined to be 3.33 ppm.

<Synthesis Example 1> Synthesis of diester mixture 1

A diester mixture 1 was synthesized from Cardolite LITE 2020 (produced by Cardolite Corporation) according to the following synthesis scheme.

Note that in the synthesis scheme, R represents any one of the four types of hydrocarbon groups having a carbon number of 15 shown below, and each * indicates a position of bonding to a benzene ring via a single bond (hereinafter, the same applies in Synthesis Examples 2 to 7). Moreover, Cardolite LITE 2020 and the diester mixture 1 are each a mixture of compounds in which R is any one of the four types of hydrocarbon groups shown below, and the two R groups included in the diester mixture 1 may be the same as each other or different from each other.

[Chem. 12]

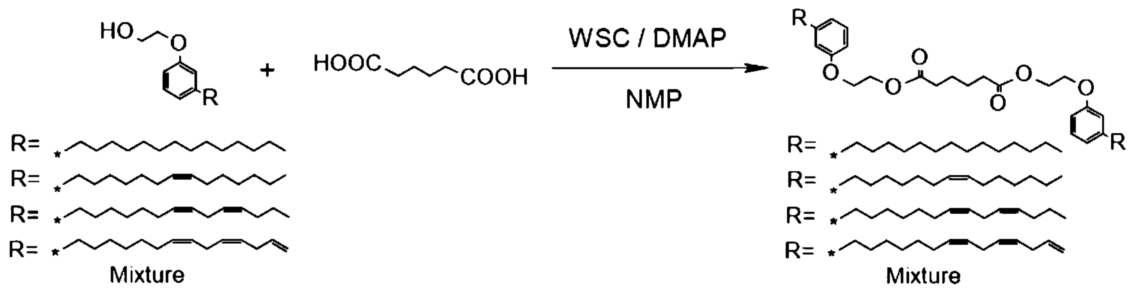


Cardolite LITE 2020

Diester mixture 1

In a three-necked reactor including a thermometer, 80 g (0.23 mol) of Cardolite LITE 2020 (produced by Cardolite Corporation) was dissolved in 250 mL of N-methylpyrrolidone (NMP) in a stream of nitrogen. Next, 16.15 g (0.11 mol) of adipic acid and 2.8 g (0.023 mol) of N,N-dimethyl-4-aminopyridine (DMAP) were added to and dissolved in this solution. The solution was adjusted to 25°C or lower in a water bath while slowly adding 44.5 g (0.23 mol) of 1-ethyl-3-(3-dimethylaminopropyl)carbodiimide hydrochloride (WSC) thereto, and then all contents of the reactor were stirred in that state at 25°C for 18 hours.

Once the reaction had ended, the reaction liquid was added into 2,500 mL of distilled water, and two extractions were performed with 300 mL of ethyl acetate. After drying the ethyl acetate layer with anhydrous sodium sulfate, the sodium sulfate was filtered off.

Solvent was removed by evaporation in a rotary evaporator, and the resultant residue was subsequently purified by silica gel column chromatography (hexane:ethyl acetate = 85:15 (volume ratio)) to yield 58 g of the diester mixture 1 as a pale yellow oil. The structure of the diester mixture 1 was identified by NMR ($^1$H-NMR and $^{13}$C-NMR) and IR (ATR).

$^1$H-NMR (500 MHz, $CDCl_3$, TMS, $\delta$ ppm): Disappearance of signal at 2.15 ppm originating from hydroxyl group of Cardolite LITE 2020 (produced by Cardolite Corporation) confirmed

$^{13}$C-NMR (500 MHz, $CDCl_3$, TMS, $\delta$ ppm): Generation of signal at 173.27 ppm originating from carbonyl carbon confirmed

IR (ATR): Generation of peak at 1736 $cm^{-1}$ originating from ester bond confirmed

The spectra for $^1$H-NMR, $^{13}$C-NMR, and IR (ATR) are respectively illustrated in FIGS. 9 to 11.

<Synthesis Example 2> Synthesis of dicarbamate mixture 1

A dicarbamate mixture 1 was synthesized from Cardolite LITE 2020 (produced by Cardolite Corporation) according to the following synthesis scheme.

Note that Cardolite LITE 2020 and the dicarbamate mixture 1 are each a mixture of compounds in which R is any

one of the four types of hydrocarbon groups shown below, and the two R groups included in the dicarbamate mixture 1 may be the same as each other or different from each other.

[Chem. 13]

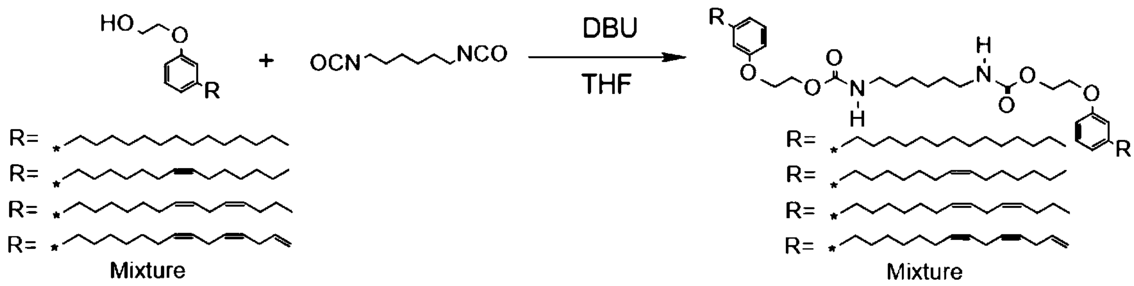


Cardolite LITE 2020

Dicarbamate mixture 1

In a three-necked reactor including a thermometer, 92 g (0.27 mol) of Cardolite LITE 2020 (produced by Cardolite Corporation) was dissolved in 200 mL of tetrahydrofuran (THF) in a stream of nitrogen, and was cooled to 5°C in an ice bath. Next, 2.0 g (0.013 mol) of 1,8-diazabicyclo[5.4.0]undec-7-ene (DBU) was slowly added to this solution. A solution obtained by dissolving 22.5 g (0.13 mol) of hexamethylene diisocyanate in 130 mL of THF was slowly added dropwise to this solution while adjusting the solution to 10°C or lower in an ice bath. Thereafter, the solution was slowly restored to room temperature, was then heated to 50°C, and was stirred in that state for 2 hours. Once the reaction had ended, the reaction liquid was added into 2,500 mL of dilute hydrochloric acid, and two extractions were performed with 300 mL of ethyl acetate. After drying the ethyl acetate layer with anhydrous sodium sulfate, the sodium sulfate was filtered off.

Solvent was removed by evaporation in a rotary evaporator, and the resultant residue was subsequently purified by silica gel column chromatography (hexane:ethyl acetate = 80:20 (volume ratio)) to yield 54 g of the dicarbamate mixture 1 as a pale yellow solid. The melting point was 94°C to 96°C. The structure of the dicarbamate mixture 1 was identified by NMR ($^1$H-NMR and $^{13}$C-NMR) and IR (ATR).

$^1$H-NMR (500 MHz, $CDCl_3$, TMS, $\delta$ ppm): Disappearance of signal at 2.15 ppm originating from hydroxyl group of Cardolite LITE 2020 (produced by Cardolite Corporation) confirmed and generation of signal at 4.79 ppm originating from N-H of carbamate confirmed

$^{13}$C-NMR (500 MHz, $CDCl_3$, TMS, $\delta$ ppm): Generation of signal at 155.99 ppm originating from carbonyl carbon confirmed

IR (ATR): Generation of peaks at 1685 $cm^{-1}$ and 1718 $cm^{-1}$ originating from carbamate bond and generation of peak at 3327 $cm^{-1}$ originating from N-H confirmed

The spectra for $^1$H-NMR, $^{13}$C-NMR, and IR (ATR) are respectively illustrated in FIGS. 12 to 14.

<Synthesis Example 3> Synthesis of diether mixture 1

A diether mixture 1 was synthesized from Cardolite LITE 2020 (produced by Cardolite Corporation) according to the following synthesis scheme.

Note that Cardolite LITE 2020 and the diether mixture 1 are each a mixture of compounds in which R is any one of the four types of hydrocarbon groups shown below, and the two R groups included in the diether mixture 1 may be the same as each other or different from each other.

[Chem. 14]

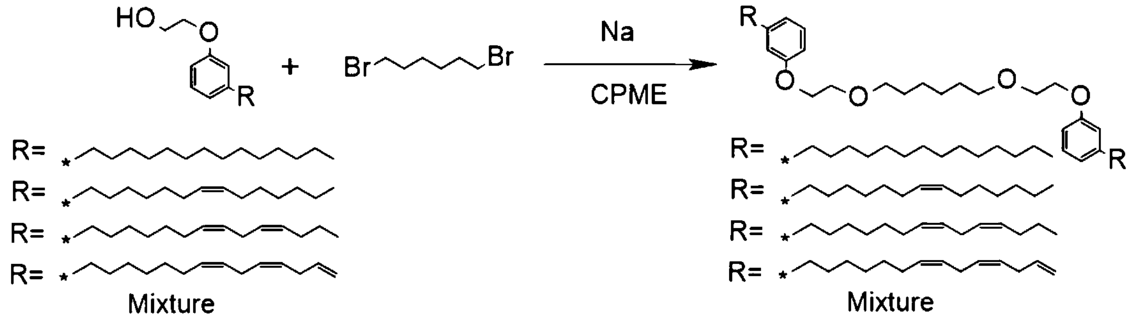


Cardolite LITE 2020

Diether mixture 1

In a three-necked reactor including a thermometer, 35.5 g (net 19.5 g (0.81 mol)) of 55% content sodium hydride and 250 mL of cyclopentyl methyl ether (CPME) were added under ice bath cooling in a stream of nitrogen. While still in the ice bath, this slurry was adjusted to an internal temperature of around 10°C while a solution of 80 g (0.23 mol) of Cardolite LITE 2020 (produced by Cardolite Corporation) dissolved in 50 mL of CPME was slowly added dropwise. This reaction liquid was restored to room temperature and was stirred at 23°C for 30 minutes. In addition, this reaction liquid was subsequently heated to 80°C, and a solution of 19.9 g (0.082 mol) of dibromohexane dissolved in 10 mL of CPME was slowly added dropwise thereto. Thereafter, 11 hours of heating under reflux was performed at 110 °C. Once the reaction had ended, cooling was performed in an ice bath, and then a mixed solution of 100 mL of tetrahydrofuran and 50 mL of water was slowly added dropwise while still in the ice bath. Next, 300 mL of 1N hydrochloric acid was added to this solution to adjust the solution to acidic, and two extractions were performed with 300 mL of ethyl acetate. After drying the ethyl acetate layer with anhydrous sodium sulfate, the sodium sulfate was filtered off.

Solvent was removed by evaporation in a rotary evaporator, and the resultant residue was subsequently purified by silica gel column chromatography (hexane:ethyl acetate = 90:10 (volume ratio)) to yield 57 g of the diether mixture 1 as a pale yellow oil. The structure of the diether mixture 1 was identified by NMR ($^1$H-NMR) and IR (ATR).

$^1$H-NMR (500 MHz, $CDCl_3$, TMS, $\delta$ ppm): Disappearance of signal at 2.15 ppm originating from hydroxyl group of Cardolite LITE 2020 (produced by Cardolite Corporation) confirmed

IR (ATR): Generation of peak at 1124 $cm^{-1}$ originating from ether bond confirmed

The spectra for $^1$H-NMR and IR (ATR) are respectively illustrated in FIGS. 15 and 16.

<Synthesis Example 4> Synthesis of diester mixture 2

A diester mixture 2 was synthesized from Cardolite NX-7505 (produced by Cardolite Corporation) according to the following synthesis scheme.

Note that Cardolite NX-7505 and the diester mixture 2 are each a mixture of compounds in which R is any one of the four types of hydrocarbon groups having a carbon number of 15 shown below, and the two R groups included in the diester mixture 2 may be the same as each other or different from each other.

[Chem. 15]

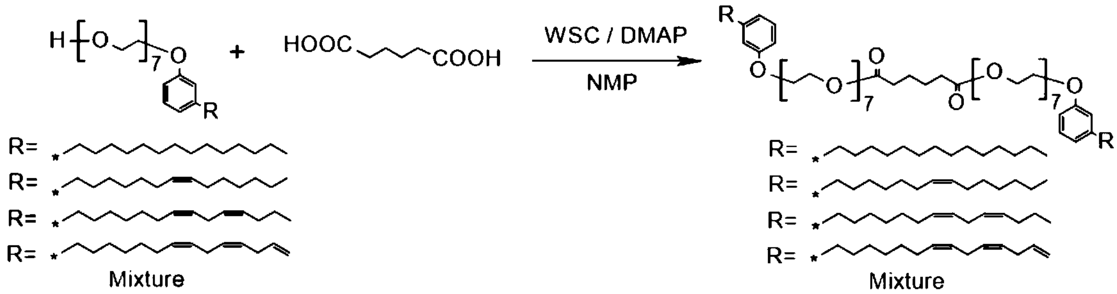


Cardolite NX-7507

Diester mixture 2

In a three-necked reactor including a thermometer, 80 g (0.13 mol) of Cardolite NX-7505 (produced by Cardolite Corporation) was dissolved in 150 mL of N-methylpyrrolidone (NMP) in a stream of nitrogen. Next, 9.13 g (0.062 mol) of adipic acid and 3.22 g (0.026 mol) of N,N-dimethyl-4-aminopyridine (DMAP) were added to and dissolved in this solution. The solution was adjusted to 25°C or lower in a water bath while slowly adding 25.24 g (0.13 mol) of 1-ethyl-3-(3-dimethylaminopropyl)carbodiimide hydrochloride (WSC) thereto, and then all contents of the reactor were stirred in that state at 25°C for 24 hours.

Once the reaction had ended, the reaction liquid was added into 2,500 mL of distilled water, and two extractions were performed with 300 mL of ethyl acetate. After drying the ethyl acetate layer with anhydrous sodium sulfate, the sodium sulfate was filtered off.

Solvent was removed by evaporation in a rotary evaporator, and the resultant residue was subsequently purified by silica gel column chromatography (chloroform:methanol = 90:10 (volume ratio)) to yield 84 g of the diester mixture 2 as a pale yellow oil. The structure of the diester mixture 2 was identified by NMR and IR (ATR).

> $^1$H-NMR (500 MHz, $CDCl_3$, TMS, δ ppm): Disappearance of signal at 3.33 ppm originating from hydroxyl group of Cardolite NX-7507 (produced by Cardolite Corporation) confirmed
> $^{13}$C-NMR (500 MHz, $CDCl_3$, TMS, δ ppm): Generation of signals at 175.03 ppm and 173.23 ppm originating from carbonyl carbon confirmed
> IR (ATR): Generation of peaks at 1736 $cm^{-1}$ and 1685 $cm^{-1}$ originating from ester bond confirmed
> The spectra for $^1$H-NMR, $^{13}$C-NMR, and IR (ATR) are respectively illustrated in FIGS. 17 to 19.

<Synthesis Example 5> Synthesis of triester mixture 1

A triester mixture 1 was synthesized from Cardolite NX-7505 (produced by Cardolite Corporation) according to the following synthesis scheme.

Note that Cardolite NX-7505 and the triester mixture 1 are each a mixture of compounds in which R is any one of the four types of hydrocarbon groups shown below, and the three R groups included in the triester mixture 1 may be the same as one another or different from one another.

[Chem. 16]

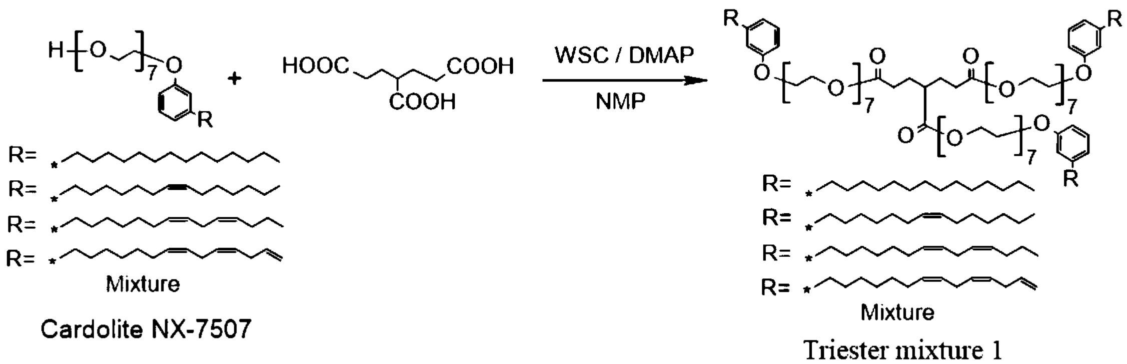


Cardolite NX-7507

Triester mixture 1

In a three-necked reactor including a thermometer, 81.8 g (0.134 mol) of Cardolite NX-7505 (produced by Cardolite Corporation) was dissolved in 200 mL of N-methylpyrrolidone (NMP) in a stream of nitrogen. Next, 9.0 g (0.044 mol) of 1,3,5-pentanetricarboxylic acid and 3.6 g (0.029 mol) of N,N-dimethyl-4-aminopyridine (DMAP) were added to and dissolved in this solution. The solution was adjusted to 25°C or lower in a water bath while slowly adding 28.3 g (0.148 mol) of 1-ethyl-3-(3-dimethylaminopropyl)carbodiimide hydrochloride (WSC) thereto, and then all contents of the reactor were stirred in that state at 25°C for 18 hours.

Once the reaction had ended, the reaction liquid was added into 2,500 mL of distilled water, and two extractions were performed with 300 mL of ethyl acetate. After drying the ethyl acetate layer with anhydrous sodium sulfate, the sodium sulfate was filtered off.

Solvent was removed by evaporation in a rotary evaporator, and the resultant residue was subsequently purified by silica gel column chromatography (chloroform:methanol = 90:10 (volume ratio)) to yield 81 g of the triester mixture 1 as a pale yellow oil. The structure of the triester mixture 1 was identified by NMR and IR (ATR).

> $^1$H-NMR (500 MHz, $CDCl_3$, TMS, δ ppm): Disappearance of signal at 3.33 ppm originating from hydroxyl group of Cardolite NX-7507 (produced by Cardolite Corporation) confirmed

$^{13}C$-NMR (500 MHz, $CDCl_3$, TMS, δ ppm): Generation of signal at 175.05 ppm originating from carbonyl carbon confirmed
IR (ATR): Generation of peaks at 1734 $cm^{-1}$ and 1685 $cm^{-1}$ originating from ester bond confirmed
The spectra for $^1H$-NMR, $^{13}C$-NMR, and IR (ATR) are respectively illustrated in FIGS. 20 to 22.

<Synthesis Example 6> Synthesis of diester mixture 3

A diester mixture 3 was synthesized from Cardolite NX-7505 (produced by Cardolite Corporation) according to the following synthesis scheme.

Note that Cardolite NX-7505 and the diester mixture 3 are each a mixture of compounds in which R is any one of the four types of hydrocarbon groups shown below, and the two R groups included in the diester mixture 3 may be the same as each other or different from each other.

[Chem. 17]

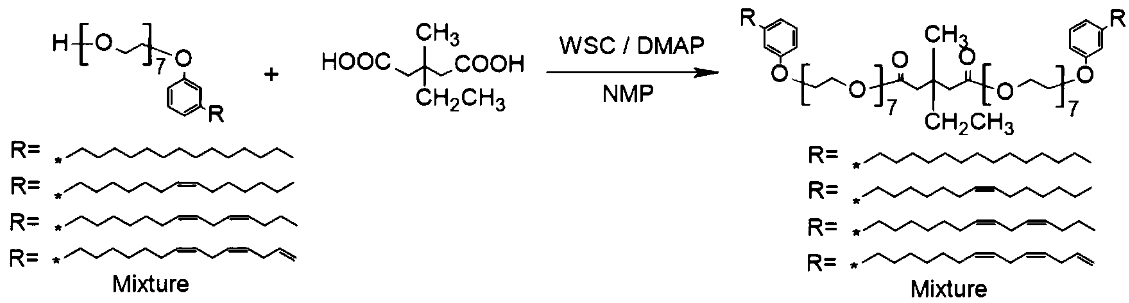


Cardolite NX-7507

Diester mixture 3

In a three-necked reactor including a thermometer, 73.3 g (0.121 mol) of Cardolite NX-7507 (produced by Cardolite Corporation) was dissolved in 200 mL of N-methylpyrrolidone (NMP) in a stream of nitrogen. Next, 10 g (0.057 mol) of 3-ethyl-3-methylglutaric acid and 3.24 g (0.027 mol) of N,N-dimethyl-4-aminopyridine (DMAP) were added to and dissolved in this solution. The solution was adjusted to 25°C or lower in a water bath while slowly adding 25.4 g (0.132 mol) of 1-ethyl-3-(3-dimethylaminopropyl)carbodiimide hydrochloride (WSC) thereto, and then all contents of the reactor were stirred in that state at 25°C for 18 hours. Once the reaction had ended, the reaction liquid was added into 2,500 mL of distilled water, and two extractions were performed with 300 mL of ethyl acetate. After drying the ethyl acetate layer with anhydrous sodium sulfate, the sodium sulfate was filtered off. Solvent was removed by evaporation in a rotary evaporator, and the resultant residue was subsequently purified by silica gel column chromatography (chloroform:methanol = gradient from 90:10 to 85:15 (volume ratio)) to yield 81 g of the diester mixture 3 as a pale yellow oil. The structure of the diester mixture 3 was identified by NMR and IR (ATR).

$^1H$-NMR (500 MHz, $CDCl_3$, TMS, δ ppm): Disappearance of signal at 3.33 ppm originating from hydroxyl group of Cardolite NX-7507 (produced by Cardolite Corporation) confirmed
$^{13}C$-NMR (500 MHz, $CDCl_3$, TMS, δ ppm): Generation of signals at 175.11 ppm and 166.39 ppm originating from carbonyl carbon confirmed
IR (ATR): Generation of peaks at 1730 $cm^{-1}$, 1685 $cm^{-1}$, and 1672 $cm^{-1}$ originating from ester bond confirmed
The spectra for $^1H$-NMR, $^{13}C$-NMR, and IR (ATR) are respectively illustrated in FIGS. 23 to 25.

<Synthesis Example 7> Synthesis of diester mixture 4

A diester mixture 4 was synthesized from Cardolite NX-7505 (produced by Cardolite Corporation) according to the following synthesis scheme.

Note that Cardolite NX-7505 and the diester mixture 4 are each a mixture of compounds in which R is any one of the four types of hydrocarbon groups shown below, and the two R groups included in the diester mixture 4 may be the same as each other or different from each other.

[Chem. 18]

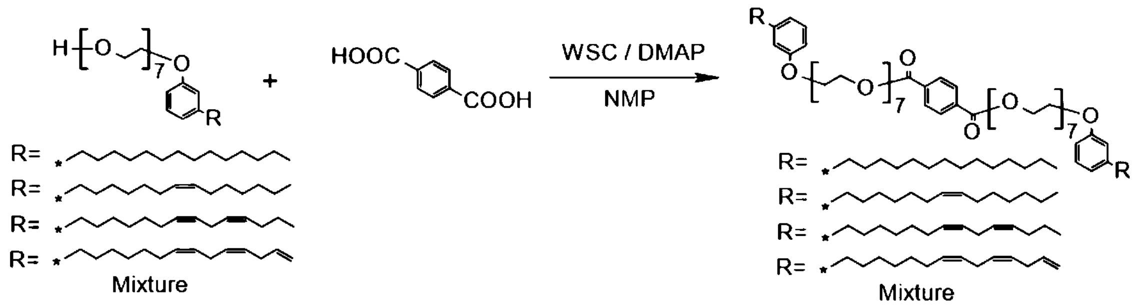


Cardolite NX-7507

Diester mixture 4

In a three-necked reactor including a thermometer, 84.58 g (0.139 mol) of Cardolite NX-7507 (produced by Cardolite Corporation) was dissolved in 250 mL of N-methylpyrrolidone (NMP) in a stream of nitrogen. Next, 11.0 g (0.066 mol) of terephthalic acid and 3.74 g (0.031 mol) of N,N-dimethyl-4-aminopyridine (DMAP) were added to and dissolved in this solution. The solution was adjusted to 25°C or lower in a water bath while slowly adding 29.32 g (0.153 mol) of 1-ethyl-3-(3-dimethylaminopropyl)carbodiimide hydrochloride (WSC) thereto, and then all contents of the reactor were stirred in that state at 25°C for 22 hours. Once the reaction had ended, the reaction liquid was added into 2,500 mL of distilled water, and two extractions were performed with 300 mL of ethyl acetate. After drying the ethyl acetate layer with anhydrous sodium sulfate, the sodium sulfate was filtered off. Solvent was removed by evaporation in a rotary evaporator, and the resultant residue was subsequently purified by silica gel column chromatography (chloroform:methanol = 85:15 (volume ratio)) to yield 82 g of the diester mixture 4 as a pale yellow oil. The structure of the diester mixture 4 was identified by NMR and IR (ATR).

$^1$H-NMR (500 MHz, $CDCl_3$, TMS, $\delta$ ppm): Disappearance of signal at 3.33 ppm originating from hydroxyl group of Cardolite NX-7507 (produced by Cardolite Corporation) confirmed and generation of aromatic hydrogen of terephthalic acid at 8.11 ppm confirmed
$^{13}$C-NMR (500 MHz, $CDCl_3$, TMS, $\delta$ ppm): Generation of signals at 174.96 ppm and 165.62 ppm originating from carbonyl carbon confirmed
IR (ATR): Generation of peaks at 1720 $cm^{-1}$ and 1689 $cm^{-1}$ originating from ester bond confirmed
The spectra for $^1$H-NMR, $^{13}$C-NMR, and IR (ATR) are respectively illustrated in FIGS. 26 to 28.

(Example 1)

<Production of vinyl chloride resin composition>

With the exception of a plasticizer composition (polyester plasticizer, compound, and other plasticizers) and vinyl chloride resin fine particles used as a dusting agent, components indicated in Table 1 were loaded into and mixed in a Henschel mixer. At the point at which the temperature of the mixture rose to 80°C, all of the plasticizer composition was added, drying up of the mixture was caused to occur (i.e., the mixture changed to a dry state through absorption of the plasticizers by vinyl chloride resin particles used as a vinyl chloride resin), and mixing was continued until the maximum temperature reached not lower than 100°C and not higher than 200°C. Thereafter, once the dried-up mixture had cooled to a temperature of lower than 100°C, the vinyl chloride resin fine particles used as the dusting agent were added to the mixture to produce a vinyl chloride resin composition.

<Formation of vinyl chloride resin molded sheet>

The obtained vinyl chloride resin composition was sprinkled onto a textured mold that was heated to a temperature of 250°C, and, after being left to melt for an arbitrary time, excess vinyl chloride resin composition was shaken off. Thereafter, the textured mold onto which the vinyl chloride resin composition had been sprinkled was placed at rest in an oven set to a temperature of 200°C, and, once 60 seconds had passed after being placed at rest, the textured mold was cooled with cooling water. Once the mold temperature had dropped to 40°C, a 200 mm × 150 mm × 1 mm vinyl chloride resin molded sheet was removed from the mold as a vinyl chloride resin molded product.

The obtained vinyl chloride resin molded sheet was used to evaluate tensile characteristics (tensile strength and tensile elongation) at low temperature in an initial (unheated) state. The results are shown in Table 1.

<Formation of laminate>

Two obtained vinyl chloride resin molded sheets (dimensions: 200 mm × 150 mm × 1 mm) were placed inside of a 200 mm × 300 mm × 10 mm mold with the textured surface facing downward.

Separately thereto, a polyol mixture containing 50 parts by mass of a propylene oxide/ethylene oxide (PO/EO) block adduct of propylene glycol (hydroxyl value: 28; terminal EO unit content: 10%; internal EO unit content: 4%), 50 parts by mass of a PO/EO block adduct of glycerin (hydroxyl value: 21; terminal EO unit content: 14%), 2.5 parts by mass of water, 0.2 parts by mass of an ethylene glycol solution of triethylenediamine (produced by Tosoh Corporation; product name: TEDA-L33), 1.2 parts by mass of triethanolamine, 0.5 parts by mass of triethylamine, and 0.5 parts by mass of a foam stabilizer (produced by Shin-Etsu Chemical Co., Ltd.; product name: F-122) was mixed with polymethylene polyphenylene polyisocyanate (polymeric MDI) in a ratio determined to give an index of 98 so as to prepare a mixed liquid. The prepared mixed liquid was poured onto the two vinyl chloride resin molded sheets that had been placed in the mold as described above. Thereafter, the mold was covered by a 348 mm × 255 mm × 10 mm aluminum plate to seal the mold. The mold was left for 5 minutes after sealing to form a laminate in which a vinyl chloride resin molded sheet (thickness: 1 mm) serving as a surface skin was lined with a foamed polyurethane molded product.

The formed laminate was removed from the mold, and adhesiveness of the vinyl chloride resin molded sheet to the foamed polyurethane molded product in the laminate was evaluated. The result is shown in Table 1.

(Examples 2 to 10 and Comparative Examples 1 to 4)

A vinyl chloride resin composition, a vinyl chloride resin molded sheet, and a laminate were produced in the same way as in Example 1 with the exception that the formulation in production of the vinyl chloride resin composition was changed as indicated in Table 1. Moreover, the obtained vinyl chloride resin molded sheet and laminate were used to evaluate tensile characteristics at low temperature, heat shrinkage resistance, and adhesiveness to a foamed polyurethane molded product of the vinyl chloride resin molded sheet. The results are shown in Table 1.

Note that in Table 1:

"EO modified" indicates "ethylene oxide modified"; and
"n" indicates "number of repetitions n of ethylene oxide unit".

[Table 1]

| | | | | Comparative example | | | Example | | | Comparative example | Example | | | | | | |
|---|---|---|---|---|---|---|---|---|---|---|---|---|---|---|---|---|---|
| | | | | 1 | 2 | 3 | 1 | 2 | 3 | 4 | 4 | 5 | 6 | 7 | 8 | 9 | 10 |
| Formulation | Vinyl chloride resin | | Vinyl chloride resin particles(1) [parts by mass] | 100 | 100 | 100 | 100 | 100 | 100 | 100 | 100 | 100 | 100 | 100 | 100 | 100 | 100 |
| | | | Vinyl chloride resin fine partieles(2) [parts by mass] | 12 | 12 | 12 | 12 | 12 | 12 | 12 | 12 | 12 | 12 | 12 | 12 | 12 | 12 |
| | Plasticizer composition | Polyester plasticizer | Adipic acid polyester(3) [parts by mass] | 110 | 100 | 100 | 100 | 100 | 100 | 100 | 100 | 100 | 100 | 100 | 100 | 100 | 100 |
| | | Compound | LITE 2020 (EO modified cardanol (n=1))(4) [parts by mass] | - | - | 10 | - | - | - | - | - | - | - | - | - | - | - |
| | | | NX-7507 (EO modified cardanol (n=7))(5) [parts by mass] | - | - | - | - | - | - | 10 | - | - | - | - | - | - | - |
| | | | Diester mixture 1 [parts by mass] (ester compound of EO modified cardanol (n=1) and adipic acid) | - | - | - | 10 | - | - | - | - | - | - | - | 5 | - | - |
| | | | Diether mixture 1 [parts by mass] (ether compound of EO modified cardanol (n=1) and 1,6-hexanediol) | - | - | - | - | 10 | - | - | - | - | - | - | - | - | - |
| | | | Dicarbamate mixture 1 [parts by mass] (carbamate compound of EO modified | - | - | - | - | - | 10 | - | - | - | - | - | - | - | - |

(continued)

| | | | | Comparative example | | | Example | | | Comparative example | Example | | | | | | |
|---|---|---|---|---|---|---|---|---|---|---|---|---|---|---|---|---|---|
| | | | | 1 | 2 | 3 | 1 | 2 | 3 | 4 | 4 | 5 | 6 | 7 | 8 | 9 | 10 |
| | | | cardanol (n=1) and hexamethylene diiso-cyanate) | | | | | | | | | | | | | | |
| | | | Diester mixture 2 [parts by mass] (ester compound of EO modified cardanol (n=7) and adipic acid) | - | - | - | - | - | - | - | 10 | - | - | - | - | 5 | 15 |
| | | | Diester mixture 4 [parts by mass] (ester compound of EO modified cardanol (n=7) and terephthalic acid) | - | - | - | - | - | - | - | - | 10 | - | - | - | - | - |
| | | | Triester mixture 1 [parts by mass] (ester compound of EO modified cardanol (n=7) and 1,3,5-pentanetricarboxylic acid) | - | - | - | - | - | - | - | - | - | 10 | - | - | - | - |
| | | | Diester mixture 3 [parts by mass] (ester compound of EO modified cardanol (n=7) and 3-ethyl-3-methylglutaric acid) | - | - | - | - | - | - | - | - | - | - | 10 | - | - | - |
| | | Other plasticizers | Di(2-ethylhexvl) dodccancdioatc(6) [parts by mass] | - | 10 | - | - | - | - | - | - | - | - | - | - | - | - |
| | | | Epoxidized soybean oil(7) [parts by mass] | 5 | 5 | 5 | 5 | 5 | 5 | 5 | 5 | 5 | 5 | 5 | 5 | 5 | 5 |

(continued)

| | | | Comparative example | | | Example | | | Comparative example | Example | | | | | | |
|---|---|---|---|---|---|---|---|---|---|---|---|---|---|---|---|---|
| | | | 1 | 2 | 3 | 1 | 2 | 3 | 4 | 4 | 5 | 6 | 7 | 8 | 9 | 10 |
| | Stabilizers | Perchloric acid substituted hydrotalcite(8) [parts by mass] | 2.5 | 2.5 | 2.5 | 2.5 | 2.5 | 2.5 | 2.5 | 2.5 | 2.5 | 2.5 | 2.5 | 2.5 | 2.5 | 2.5 |
| | | Zeolite(9) [part by mass] | 1.5 | 1.5 | 1.5 | 1.5 | 1.5 | 1.5 | 1.5 | 1.5 | 1.5 | 1.5 | 1.5 | 1.5 | 1.5 | 1.5 |
| | | Zinc stearate(10) [parts by mass] | 0.3 | 0.3 | 0.3 | 0.3 | 0.3 | 0.3 | 0.3 | 0.3 | 0.3 | 0.3 | 0.3 | 0.3 | 0.3 | 0.3 |
| | | Hindered amine light stabilizer(11) [parts by mass] | 0.2 | 0.2 | 0.2 | 0.2 | 0.2 | 0.2 | 0.2 | 0.2 | 0.2 | 0.2 | 0.2 | 0.2 | 0.2 | 0.2 |
| | Mold release agent | 12-Hydroxystcaric acid(12) [parts by mass] | 0.4 | 0.4 | 0.4 | 0.4 | 0.4 | 0.4 | 0.4 | 0.4 | 0.4 | 0.4 | 0.4 | 0.4 | 0.4 | 0.4 |
| | Pigment | Black(13) [parts by mass] | 3.8 | 3.8 | 3.8 | 3.8 | 3.8 | 3.8 | 3.8 | 3.8 | 3.8 | 3.8 | 3.8 | 3.8 | 3.8 | 3.8 |
| Evaluation results | Tensile characteristics at low temperature (-25°C) | Tensile breaking elongation [%] | 140 | 170 | 160 | 170 | 170 | 150 | 160 | 170 | 160 | 150 | 160 | 160 | 160 | 180 |
| | | Tensile breaking stress [MPa] | 22.0 | 21.0 | 21.5 | 22.0 | 22.0 | 21.0 | 22.0 | 22.0 | 21.5 | 20.5 | 21.5 | 21.0 | 20.5 | 22.0 |
| | Heat shrinkage resistance | Heat shrinkage rate (after heating at 120°C × 500 hr) [%] | 1.3 | 1.9 | 2.3 | 1.7 | 1.7 | 1.5 | 2.1 | 1.5 | 1.5 | 1.7 | 1.5 | 1.4 | 1.4 | 1.8 |
| | Adhesiveness to foamed polyurethane molded product | | C | C | B | B | B | C | A | A | A | A | A | B | A | A |

(1) Product name: ZEST® (ZEST is a registered trademark in Japan, other countries, or both) 1300SI (produced by suspension polymerization; average degree of polymerization: 1,300; average particle diameter: 115 μm); produced by Shin Dai-ichi Vinyl Corporation
(2) Product name: ZEST PQLTX (produced by emulsion polymerization; average degree of polymerization: 800; average particle diameter: 1.8 μm); produced by Shin Dai-ichi Vinyl Corporation
(3) Product name: ADK CIZER HPN -3130 (adipic acid polyester; viscosity (25°C): 3,000 mPa-s); produced by Adeka Corporation
(4) Product name: Cardolite LITE 2020 (ethylene oxide modified cardanol; number of repetitions n of ethylene oxide unit: 1); produced by Cardolite Corporation
(5) Product name: Cardolite NX-7507 (ethylene oxide modified cardanol; number of repetitions n of ethylene oxide unit: 7); produced by Cardolite Corporation
(6) Product name: DODN; produced by Taoka Chemical Co., Ltd.
(7) Product name: ADK CIZER O-130S; produced by Adeka Corporation
(8) Product name: ALCAMIZER® 5 (ALCAMIZER is a registered trademark in Japan, other countries, or both); produced by Kyowa Chemical Industry Co., Ltd.
(9) Product name: MIZUKALIZER DS; produced by Mizusawa Industrial Chemicals, Ltd.
(10) Product name: SAKAI SZ2000; produced by Sakai Chemical Industry Co., Ltd.
(11) Product name: ADK STAB LA-72; produced by Adeka Corporation
(12) Product name: ADK STAB LS-12; produced by Adeka Corporation
(13) Product name: DA P 4720 Black; produced by Dainichiseika Color and Chemicals Mfg. Co., Ltd.

It can be seen from Table 1 that through the resin compositions of Examples 1 to 10, which were each produced using a compound having a specific structure as a plasticizer, it is possible to form a resin molded product having excellent low-temperature tensile elongation and heat shrinkage resistance.

In contrast, it can be seen that a resin molded product formed from the resin composition of Comparative Example 1, which was produced without using a compound having a specific structure, has poor low-temperature tensile elongation.

It can also be seen that a resin molded product formed from the resin composition of Comparative Example 2, which was produced using di(2-ethylhexyl) dodecanedioate instead of a compound having a specific structure, has poor heat shrinkage resistance.

It can also be seen that resin molded products formed from the resin compositions of Comparative Examples 3 and 4, which were each produced using ethylene oxide modified cardanol instead of a compound having a specific structure, each have poor heat shrinkage resistance.

INDUSTRIAL APPLICABILITY

According to the present disclosure, it is possible to provide a compound that can be used in production of a resin composition that is capable of forming a resin molded product having excellent low-temperature tensile elongation and heat shrinkage resistance and a method of using this compound.

Moreover, according to the present disclosure, it is possible to provide a plasticizer composition that can be used in production of a resin composition that is capable of forming a resin molded product having excellent low-temperature tensile elongation and heat shrinkage resistance.

Furthermore, according to the present disclosure, it is possible to provide a resin composition that is capable of forming a resin molded product having excellent low-temperature tensile elongation and heat shrinkage resistance.

Also, according to the present disclosure, it is possible to provide a resin molded product having excellent low-temperature tensile elongation and heat shrinkage resistance.

Moreover, according to the present disclosure, it is possible to provide a laminate including this resin molded product.

## Claims

**1.** A compound represented by formula (1), shown below:

[Chem. 1]

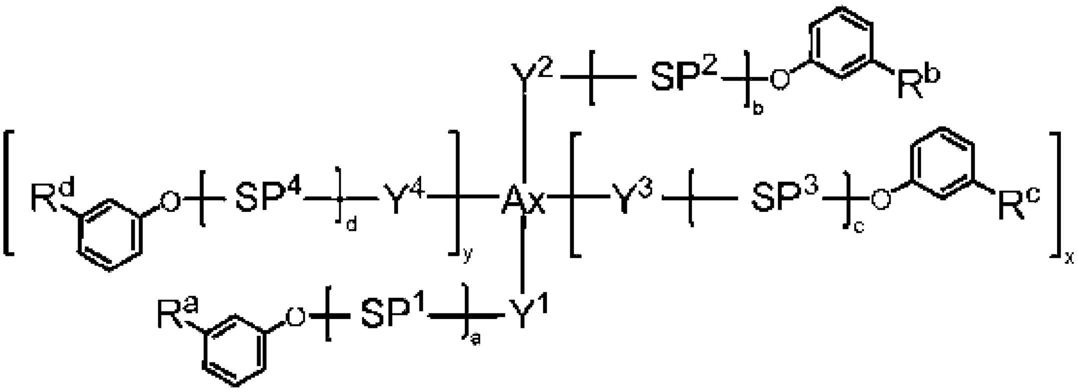

. . . (1)

where, in formula (1):

Ax represents an organic group having a carbon number of 3 to 20;
$Y^1$ and $Y^4$ each independently represent a single bond, -C(=O)-, -C(=O)-$NR^1$-, or -C(=O)-O-;
$R^1$ represents a hydrogen atom, a methyl group, or an ethyl group;
$Y^2$ and $Y^3$ each independently represent a single bond, -C(=O)-, -$NR^2$-C(=O)-, or -O-C(=O)-;
$R^2$ represents a hydrogen atom, a methyl group, or an ethyl group;
$SP^1$ and $SP^4$ each independently represent $-CH_2CH_2O-$, $-CH_2CH(CH_3)O-$, $-CH(CH_3)CH_2O-$, $-CH_2CH(CH_2CH_3)O-$, or $-CH(CH_2CH_3)CH_2O-$;
$SP^2$ and $SP^3$ each independently represent $-OCH_2CH_2-$, $-OCH(CH_3)CH_2-$, $-OCH_2CH(CH_3)-$, $-OCH(CH_2CH_3)CH_2-$, or $-OCH_2CH(CH_2CH_3)-$;
a, b, c, and d each independently represent an integer of 1 to 30;
$R^a$, $R^b$, $R^c$, and $R^d$ each independently represent an optionally substituted chain aliphatic hydrocarbon group having a carbon number of 12 to 18;
x and y each independently represent 0 or 1;
in a case in which x is 0, a structure represented by formula (2), shown below:

[Chem. 2]

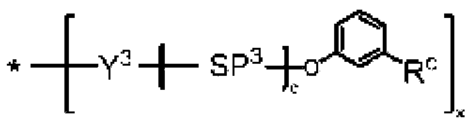

. . . (2)

represents a hydrogen atom;
in a case in which y is 0, a structure represented by formula (3), shown below:

[Chem. 3]

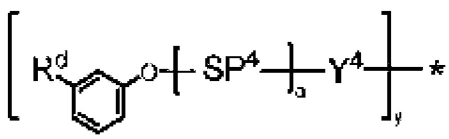

. . . (3)

represents a hydrogen atom; and
* in formula (2) and formula (3) represents a bonding position with Ax.

**2.** The compound according to claim 1, wherein Ax is any organic group among:

(i) an optionally substituted chain aliphatic hydrocarbon group having a carbon number of 3 to 20;
(ii) an optionally substituted cyclic aliphatic hydrocarbon group having a carbon number of 4 to 12;
(iii) an optionally substituted aromatic hydrocarbon group having a carbon number of 6 to 14; and
(iv) an organic group having a carbon number of 3 to 20 in which at least one single bond included in an optionally substituted chain aliphatic hydrocarbon group has been replaced by -O-, -C(=O)-, -O-C(=O)-, or -C(=O)-O-, with a proviso that a case in which -O- is consecutively present twice or more, a case in which -C(=O)- is consecutively present twice or more, and a case in which -O- or -C(=O)- is located at an end bonded to $Y^1$, $Y^2$, $Y^3$, or $Y^4$ are excluded.

**3.** The compound according to claim 1 or 2, wherein Ax is a group represented by any one of formulae (2-1) to (2-35), shown below:

[Chem. 4]

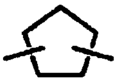 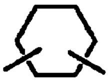 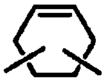 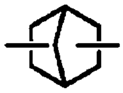 $-(CH_2)_3-$ $-(CH_2)_4-$

(2-1) (2-2) (2-3) (2-4) (2-5) (2-6)

$-(CH_2)_5-$ $-(CH_2)_6-$ $-(CH_2)_7-$ $-(CH_2)_8-$ $-(CH_2)_9-$

(2-7) (2-8) (2-9) (2-10) (2-11)

$-(CH_2)_{10}-$ $-(CH_2)_{11}-$ $-(CH_2)_{12}-$ $-(CH_2)_{13}-$ $-(CH_2)_{14}-$

(2-12) (2-13) (2-14) (2-15) (2-16)

$-(CH_2)_{15}-$ $-(CH_2)_{16}-$ 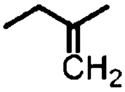 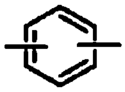 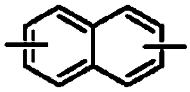

(2-17) (2-18) (2-19) (2-20) (2-21)

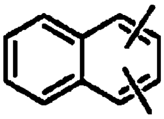 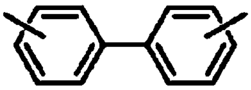 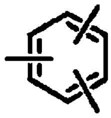 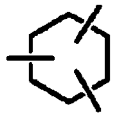

(2-22) (2-23) (2-24) (2-25)

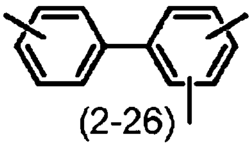 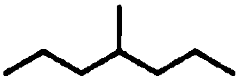 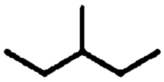 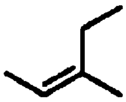

(2-26) (2-27) (2-28) (2-29)

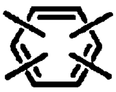 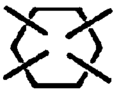 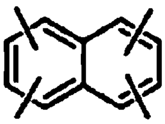 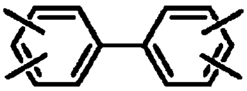

(2-30) (2-31) (2-32) (2-33)

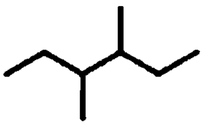 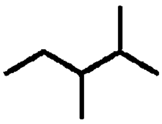

(2-34) (2-35)

that is optionally substituted.

**4.** A method comprising using the compound according to any one of claims 1 to 3 as a plasticizer.

**5.** A plasticizer composition comprising the compound according to any one of claims 1 to 3.

**6.** The plasticizer composition according to claim 5, comprising two or more types of the compound that have different structures to each other.

**7.** The plasticizer composition according to claim 5 or 6, further comprising a polyester plasticizer.

**8.** The plasticizer composition according to claim 7, wherein the polyester plasticizer includes an adipic acid polyester.

**9.** The plasticizer composition according to claim 7 or 8, wherein content of the compound is not less than 1 part by mass and not more than 20 parts by mass relative to 100 parts by mass of the polyester plasticizer.

**10.** A resin composition comprising: a resin; and the plasticizer composition according to any one of claims 5 to 9.

**11.** The resin composition according to claim 10, wherein the resin contains a halogen.

**12.** The resin composition according to claim 10 or 11, wherein the resin has a glass-transition temperature of not lower than 50°C and not higher than 100°C.

**13.** The resin composition according to any one of claims 10 to 12, wherein the resin includes a vinyl chloride resin.

**14.** The resin composition according to any one of claims 10 to 13, wherein content of the compound is not less than 1 part by mass and not more than 20 parts by mass relative to 100 parts by mass of the resin.

**15.** The resin composition according to any one of claims 10 to 14 used in powder molding.

**16.** The resin composition according to any one of claims 10 to 15 used in powder slush molding.

**17.** A resin molded product obtained through molding of the resin composition according to any one of claims 10 to 16.

**18.** The resin molded product according to claim 17 for an automobile instrument panel surface skin.

**19.** A laminate comprising: a foamed polyurethane molded product; and the resin molded product according to claim 17 or 18.

**20.** The laminate according to claim 19 for an automobile instrument panel.

FIG. 1

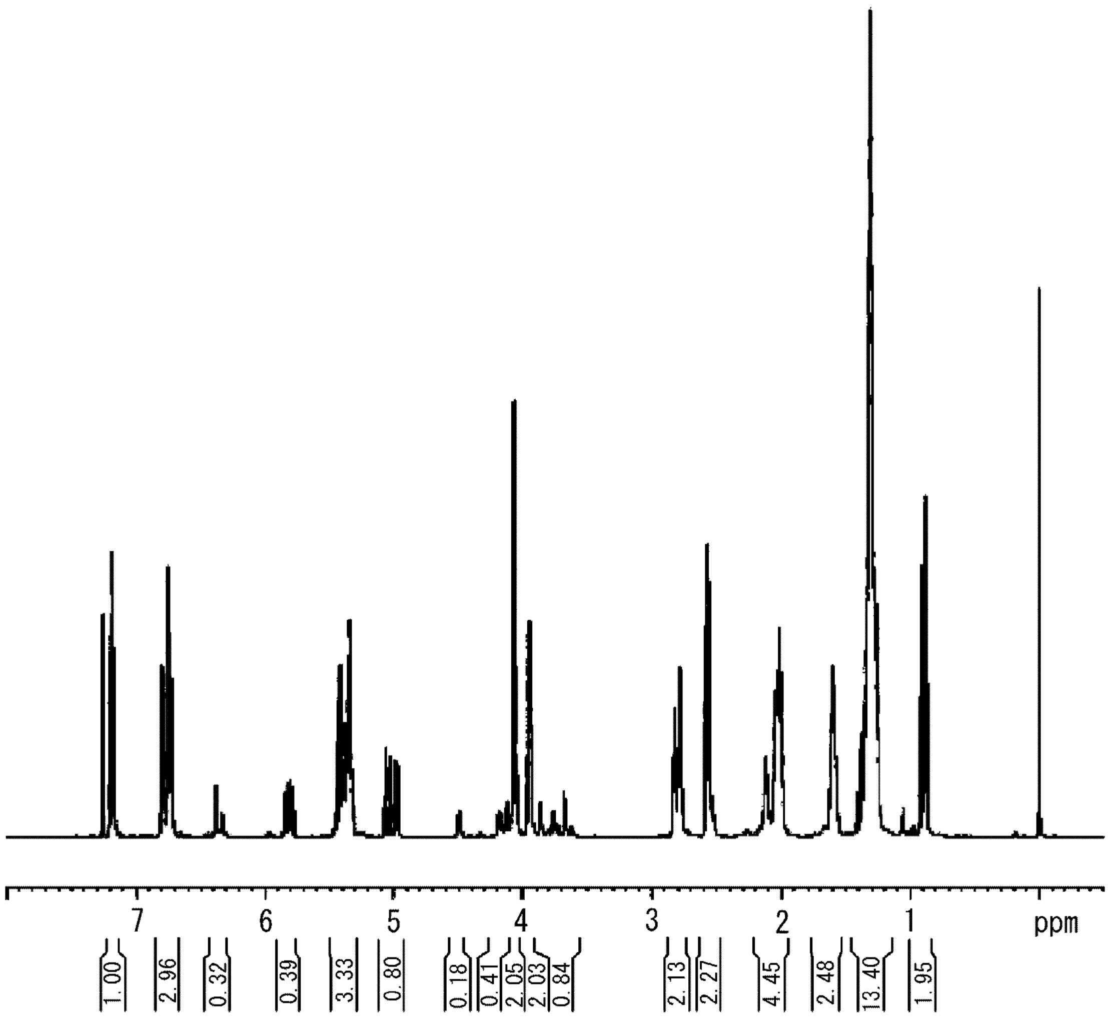
7
6
5
4
3
2
1
ppm
1.00
2.96
0.32
0.39
3.33
0.80
0.18
0.41
2.05
2.03
0.84
2.13
2.27
4.45
2.48
13.40
1.95

FIG. 2

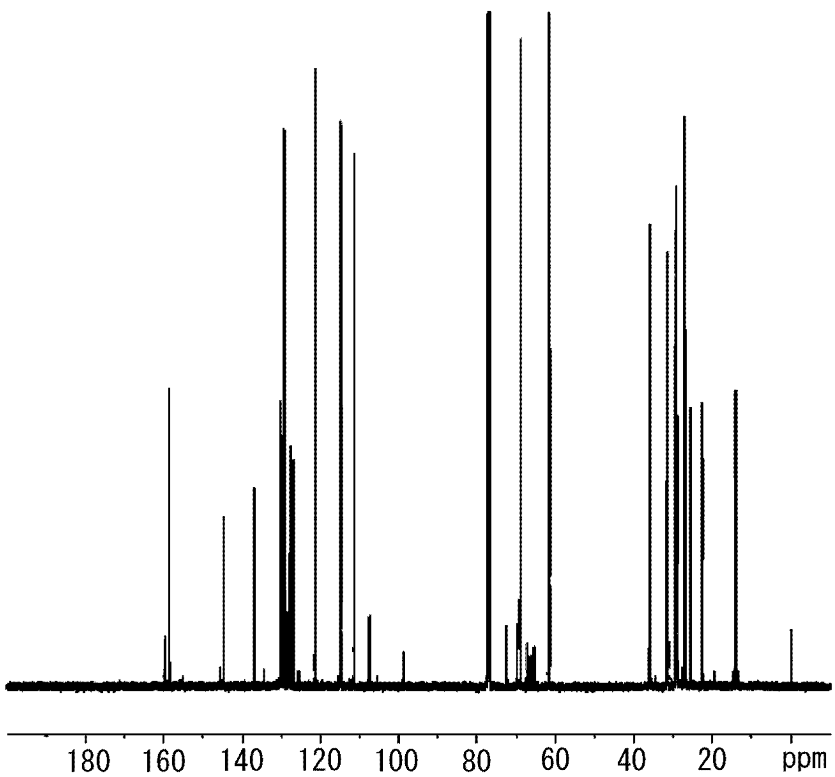

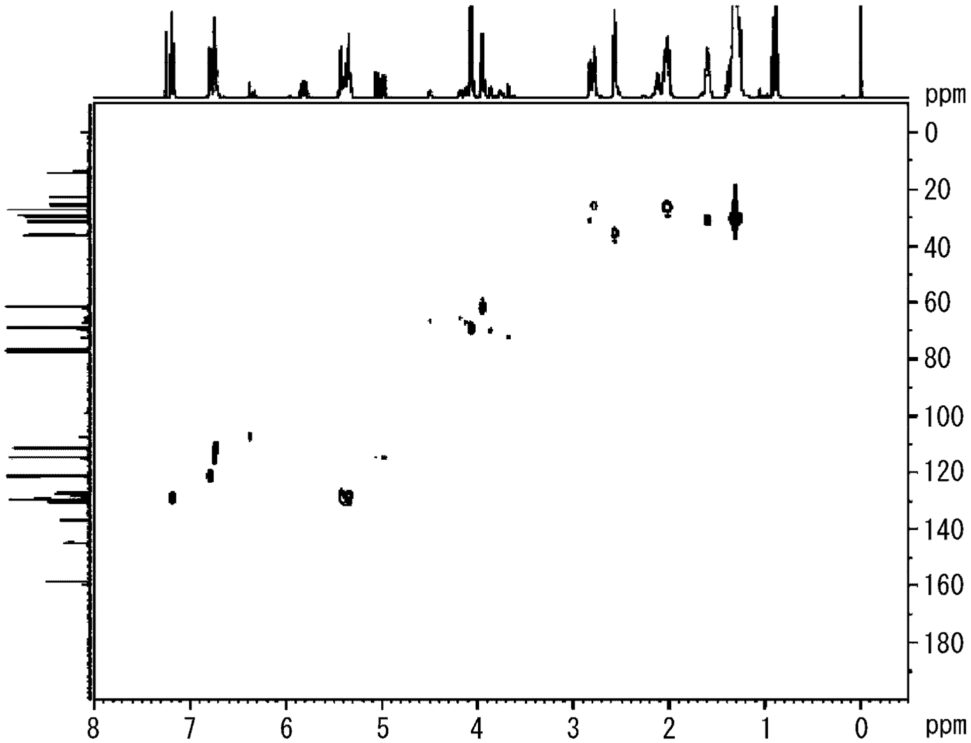
ppm
0
20
40
60
80
100
120
140
160
180
8
7
6
5
4
3
2
1
0
ppm

FIG. 3

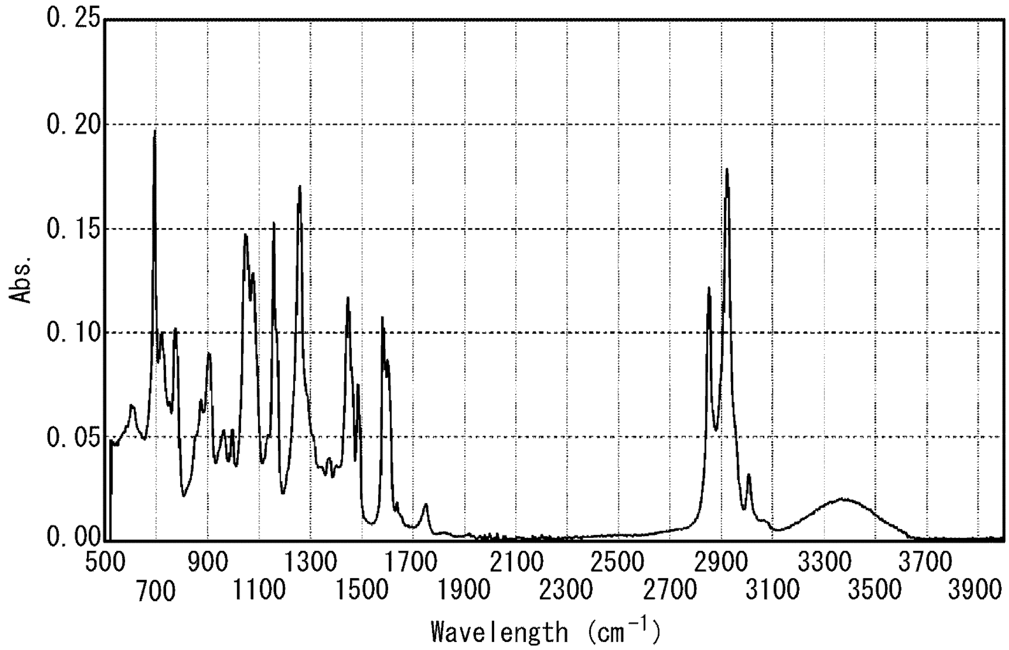
0.25
0.20
0.15
0.10
0.05
0.00
Abs.
500
700
900
1100
1300
1500
1700
1900
2100
2300
2500
2700
2900
3100
3300
3500
3700
3900
Wavelength (cm$^{-1}$)

FIG. 4

FIG. 5

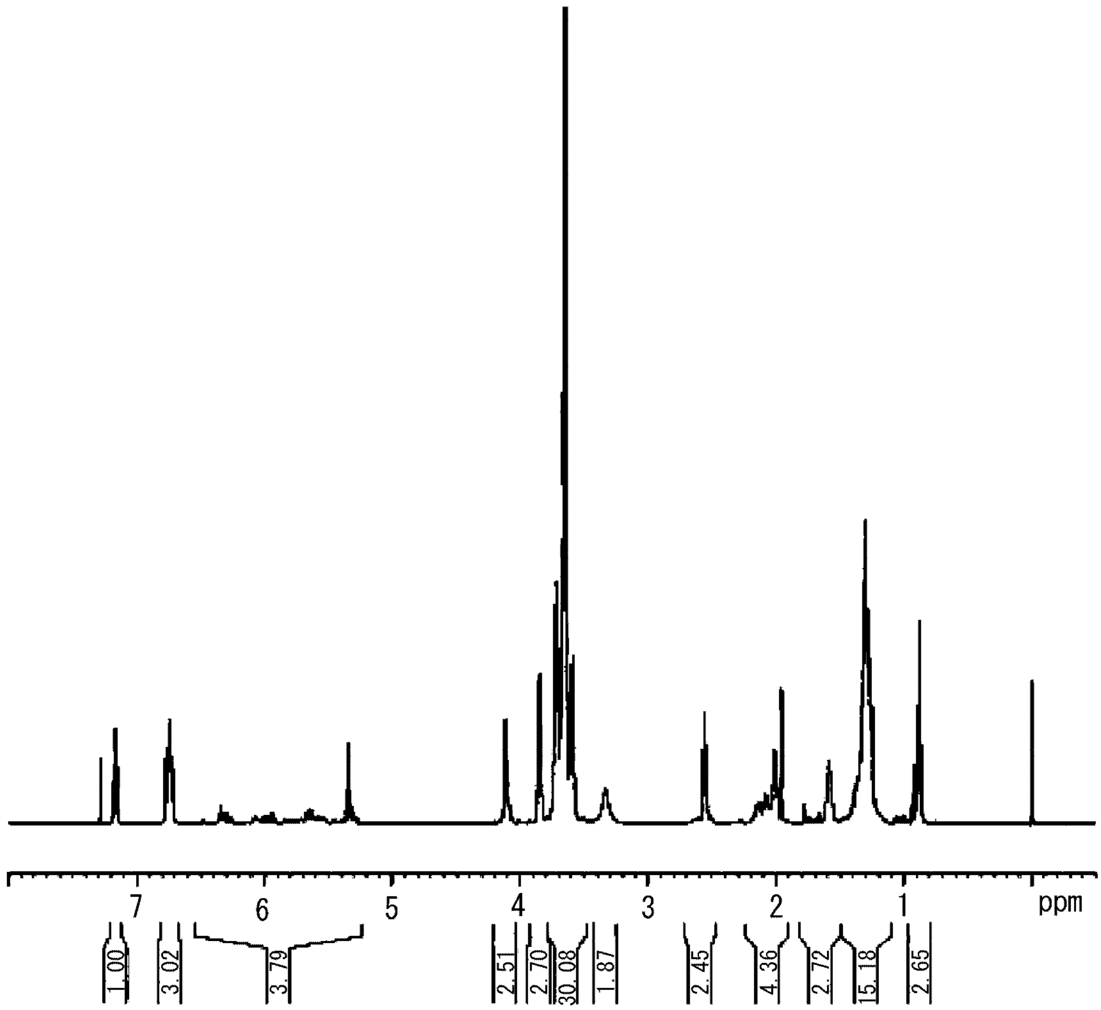

FIG. 6

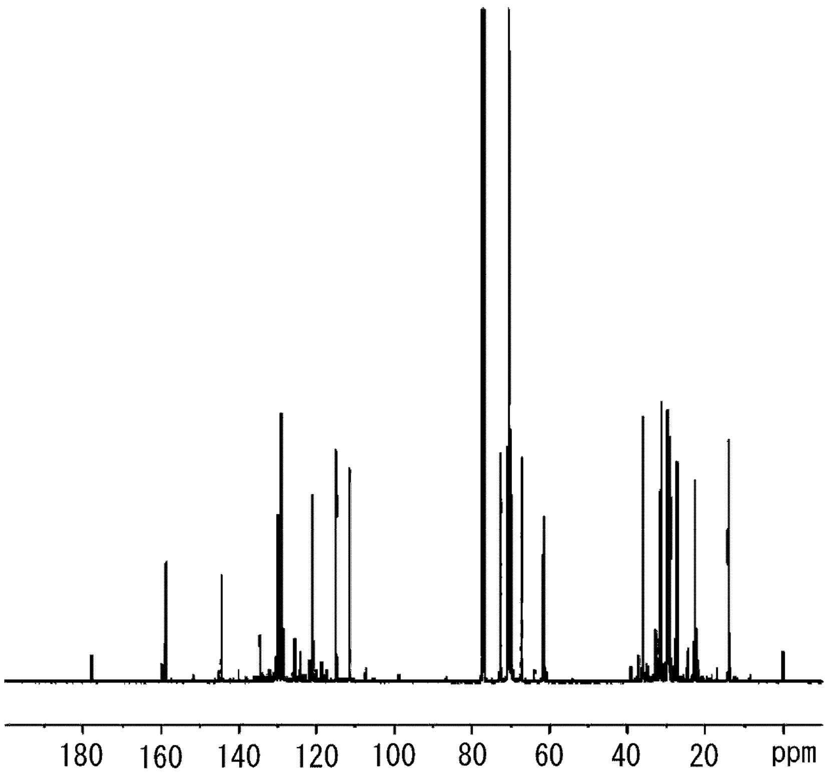

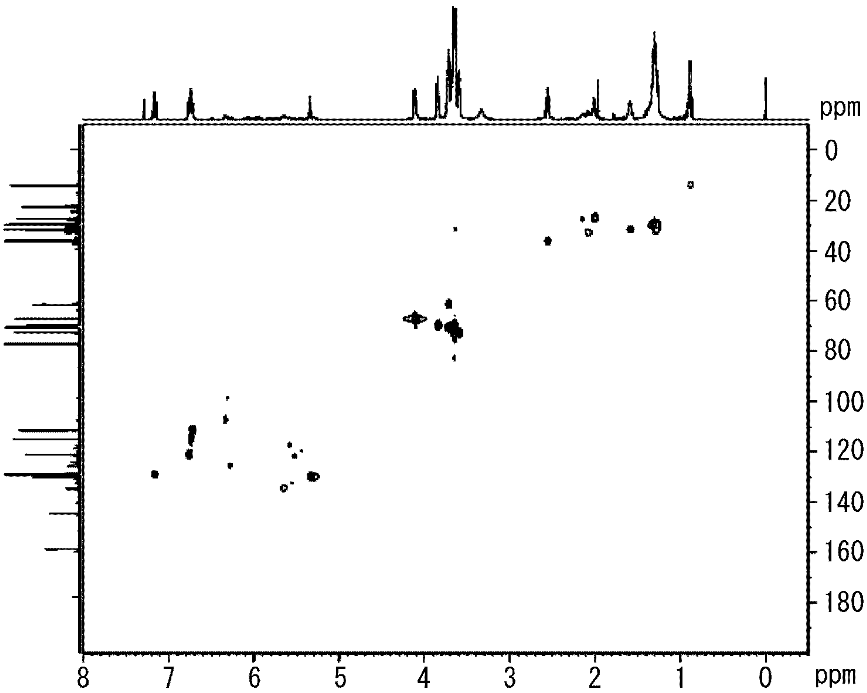
ppm
0
20
40
60
80
100
120
140
160
180
8
7
6
5
4
3
2
1
0
ppm

FIG. 7

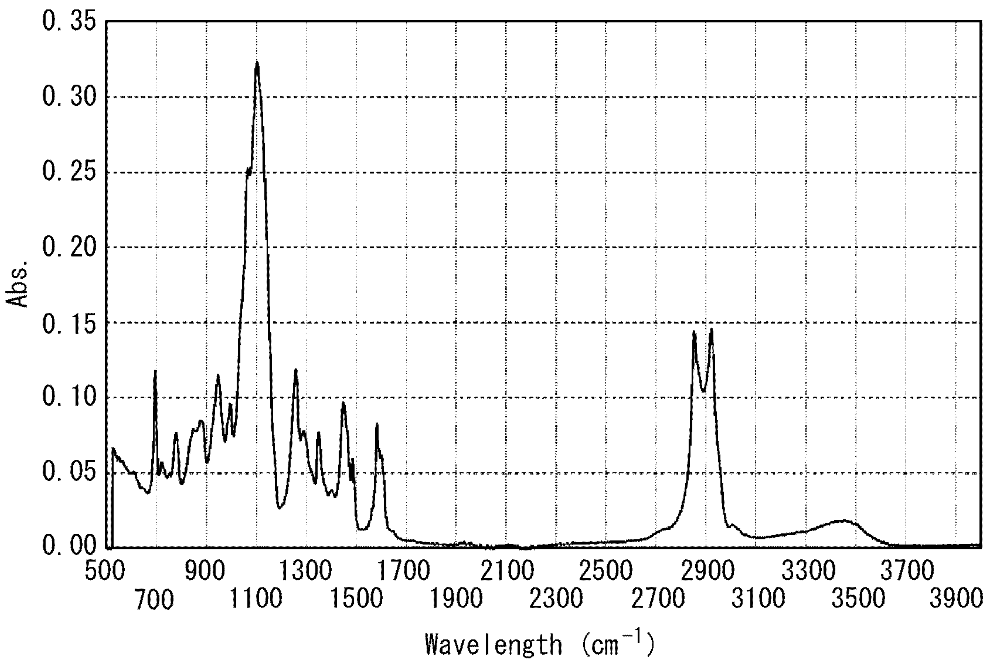
0.35
0.30
0.25
0.20
0.15
0.10
0.05
0.00
Abs.
500
700
900
1100
1300
1500
1700
1900
2100
2300
2500
2700
2900
3100
3300
3500
3700
3900
Wavelength (cm-1)

FIG. 8

FIG. 9

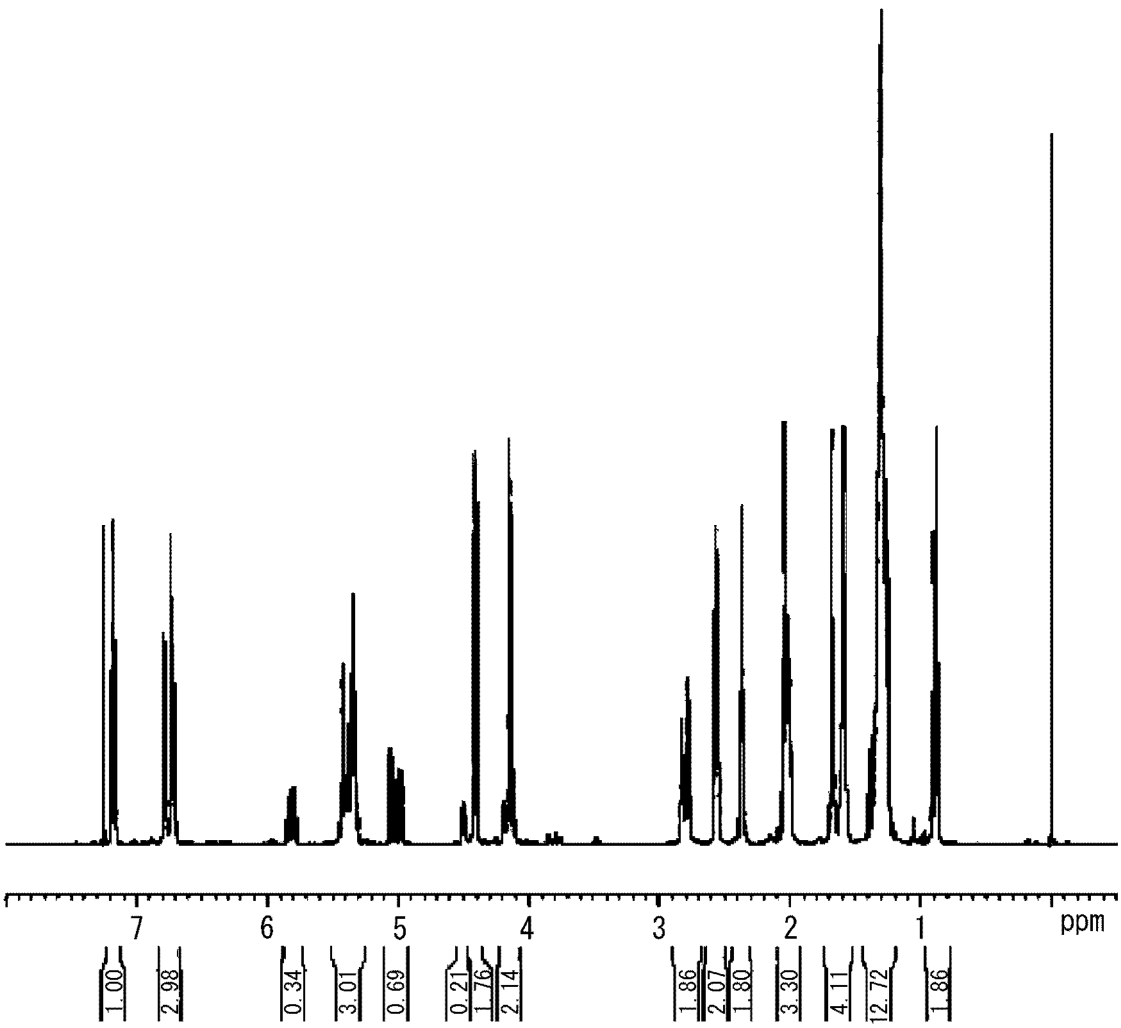
7
6
5
4
3
2
1
ppm
1.00
2.98
0.34
3.01
0.69
0.21
1.76
2.14
1.86
2.07
1.80
3.30
4.11
12.72
1.86

FIG. 10

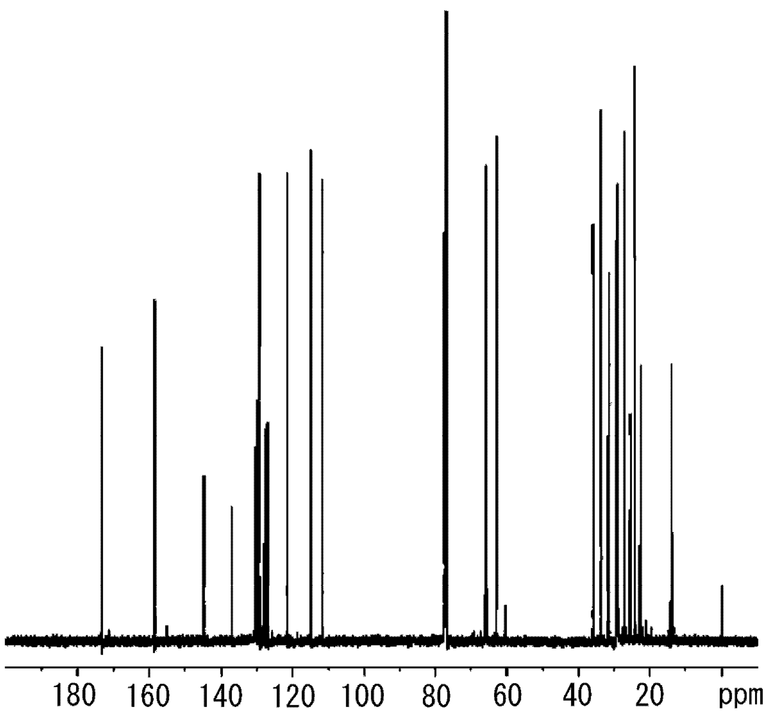

FIG. 11

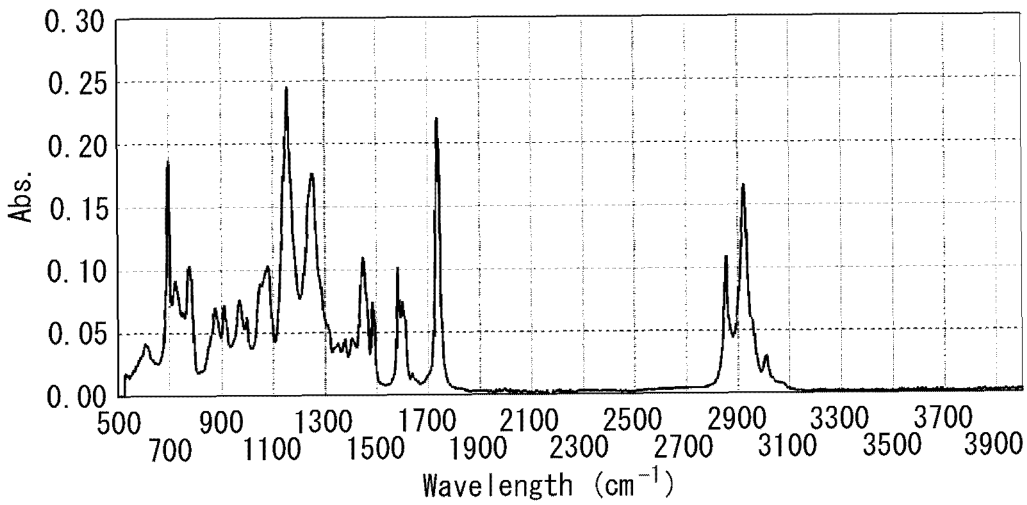
0. 30
0. 25
0. 20
0. 15
0. 10
0. 05
0. 00
Abs.
500
700
900
1100
1300
1500
1700
1900
2100
2300
2500
2700
2900
3100
3300
3500
3700
3900
Wavelength (cm$^{-1}$)

FIG. 12

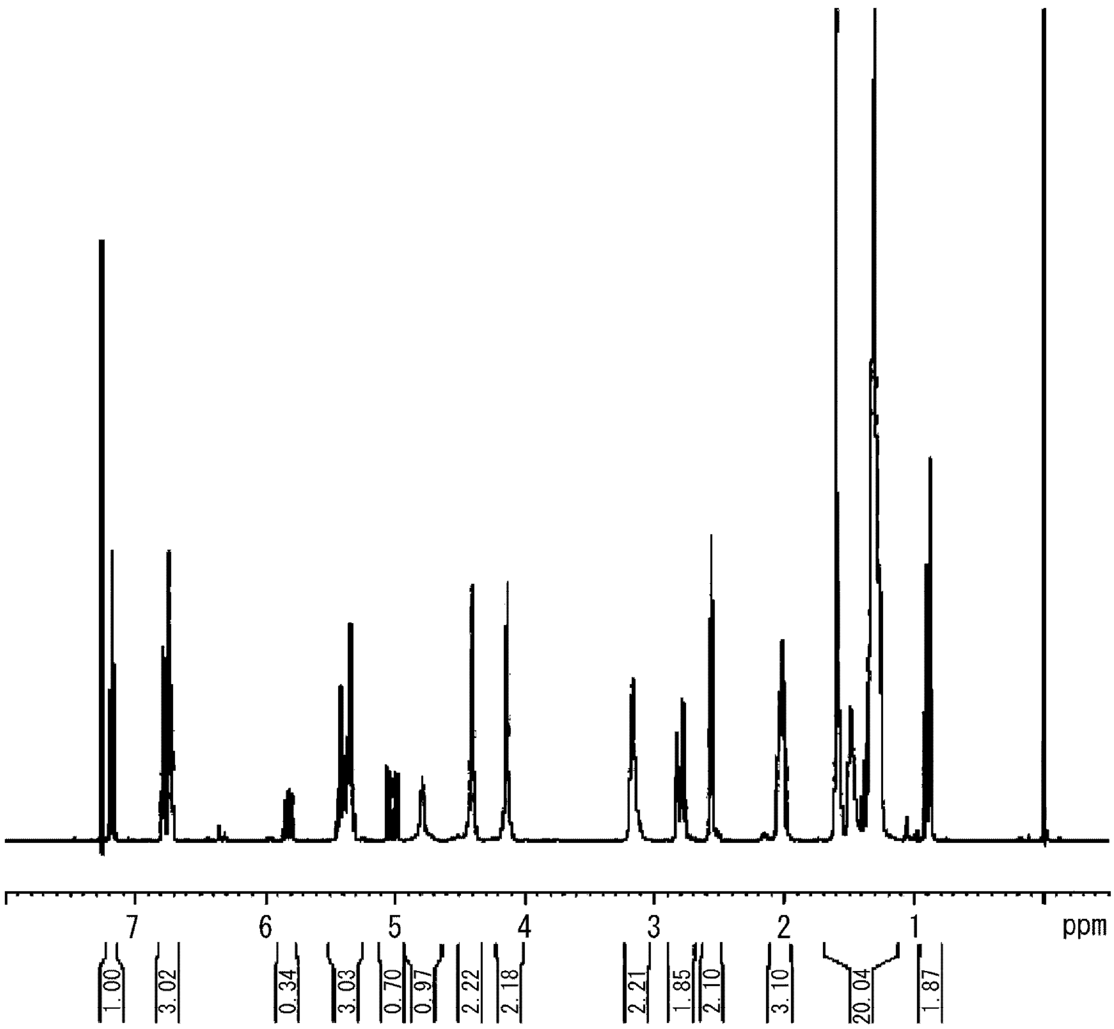
7
6
5
4
3
2
1
ppm
1.00
3.02
0.34
3.03
0.70
0.97
2.22
2.18
2.21
1.85
2.10
3.10
20.04
1.87

FIG. 13

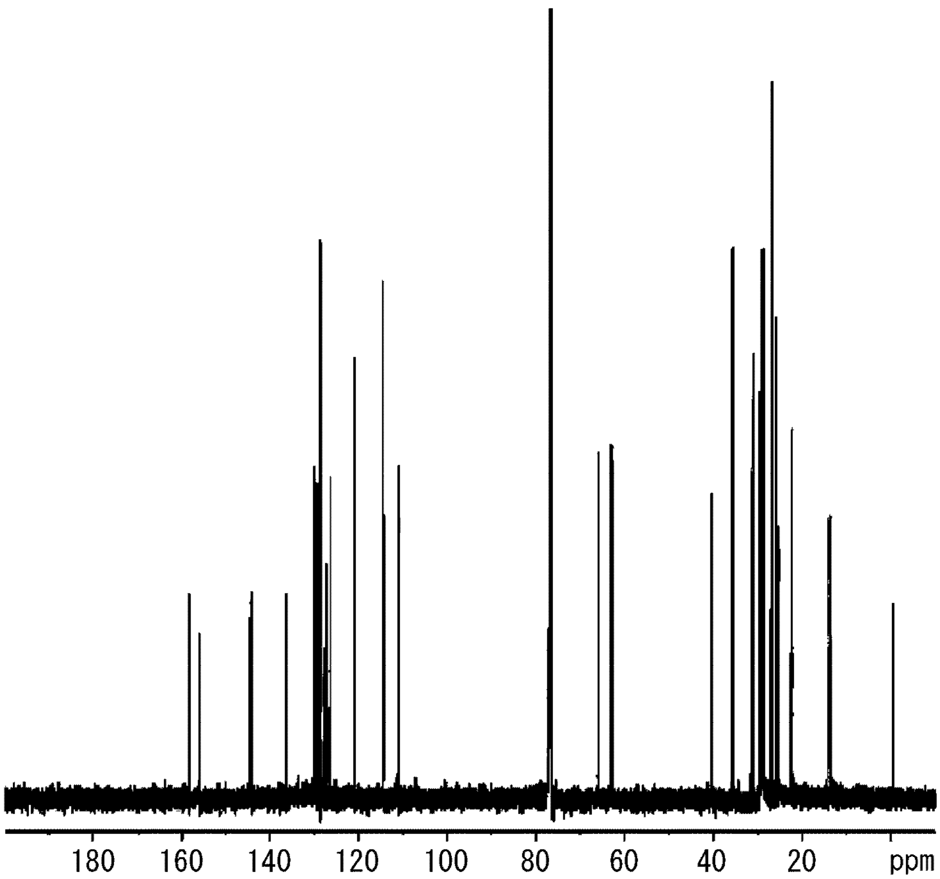
180
160
140
120
100
80
60
40
20
ppm

FIG. 14

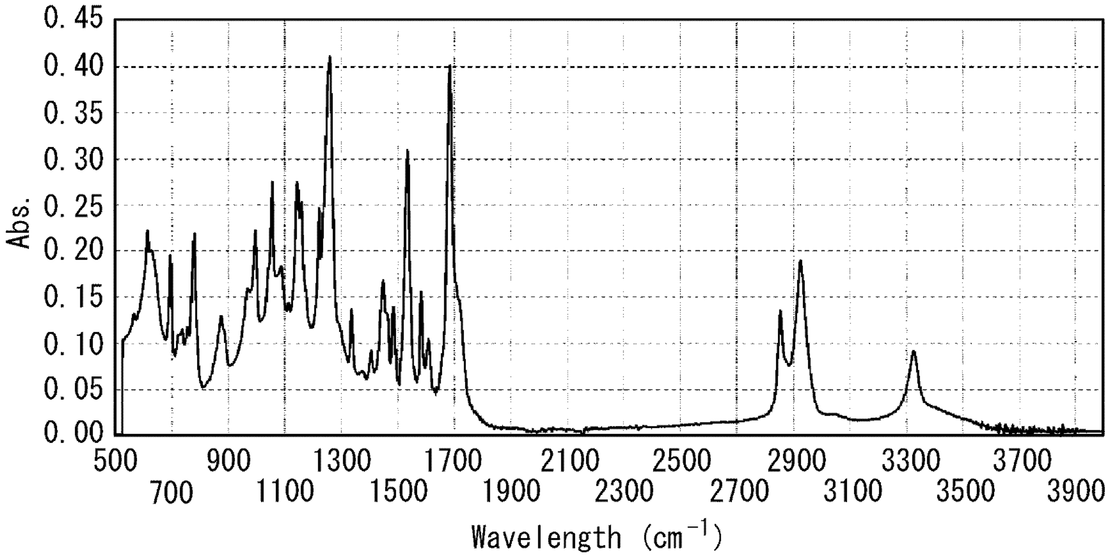
0.45
0.40
0.35
0.30
0.25
0.20
0.15
0.10
0.05
0.00
Abs.
500
700
900
1100
1300
1500
1700
1900
2100
2300
2500
2700
2900
3100
3300
3500
3700
3900
Wavelength (cm-1)

FIG. 15

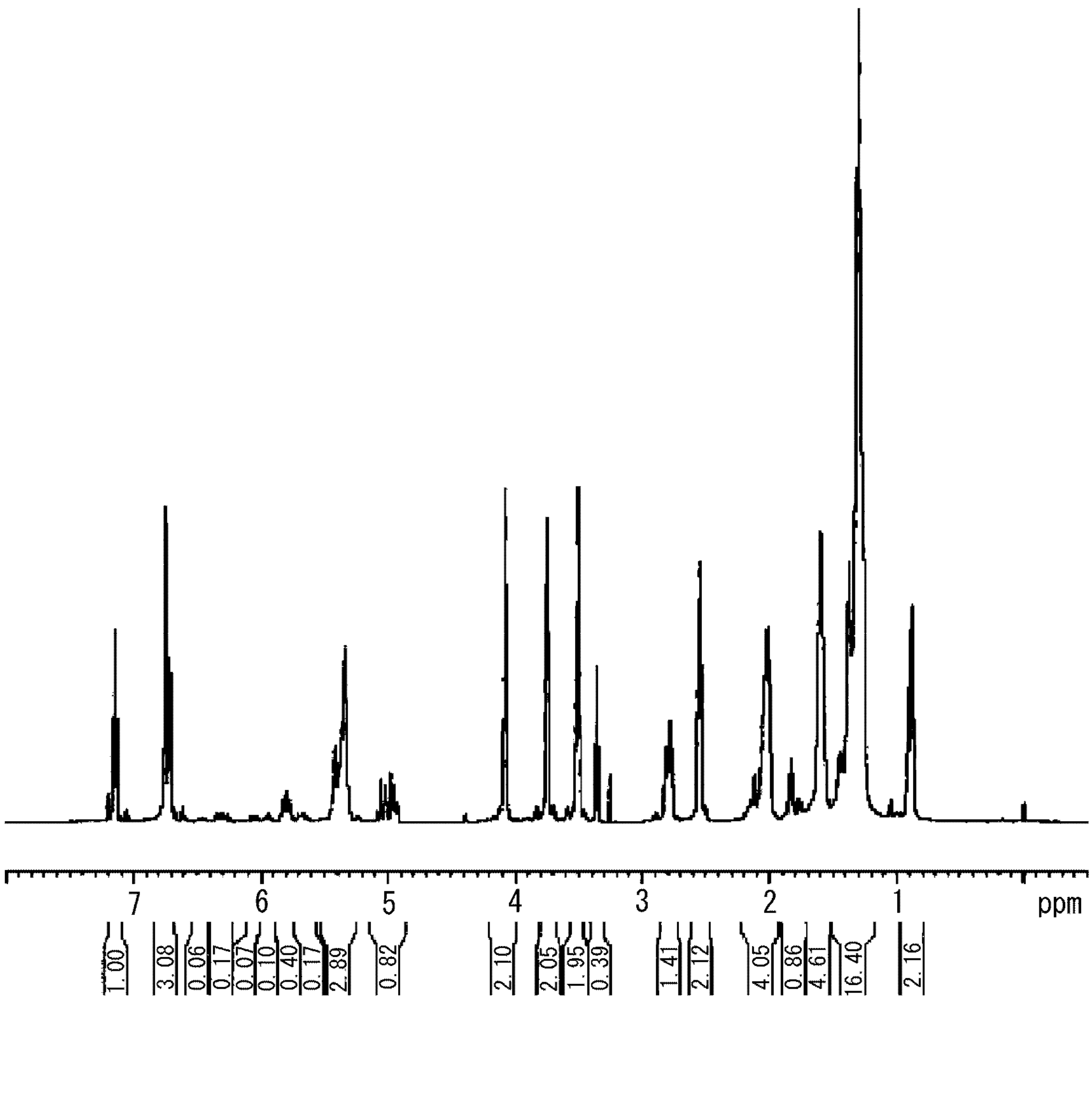
7
6
5
4
3
2
1
ppm
1.00
3.08
0.06
0.17
0.07
0.10
0.40
0.17
2.89
0.82
2.10
2.05
1.95
0.39
1.41
2.12
4.05
0.86
4.61
16.40
2.16

FIG. 16

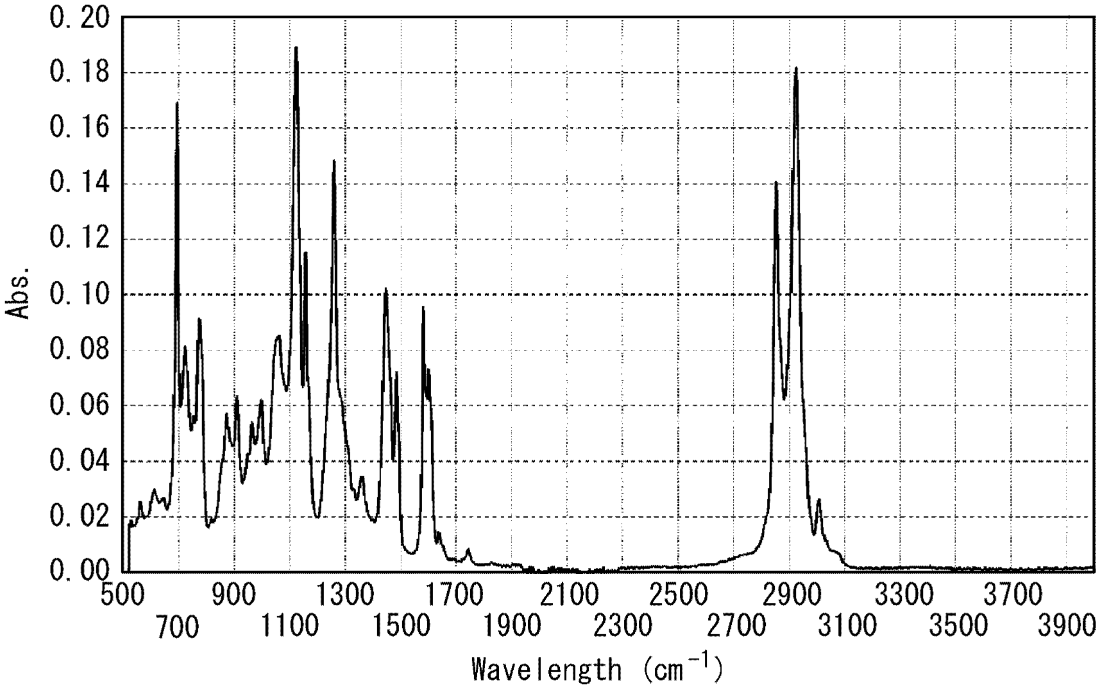
Abs.
0.20
0.18
0.16
0.14
0.12
0.10
0.08
0.06
0.04
0.02
0.00
500
700
900
1100
1300
1500
1700
1900
2100
2300
2500
2700
2900
3100
3300
3500
3700
3900
Wavelength (cm-1)

FIG. 17

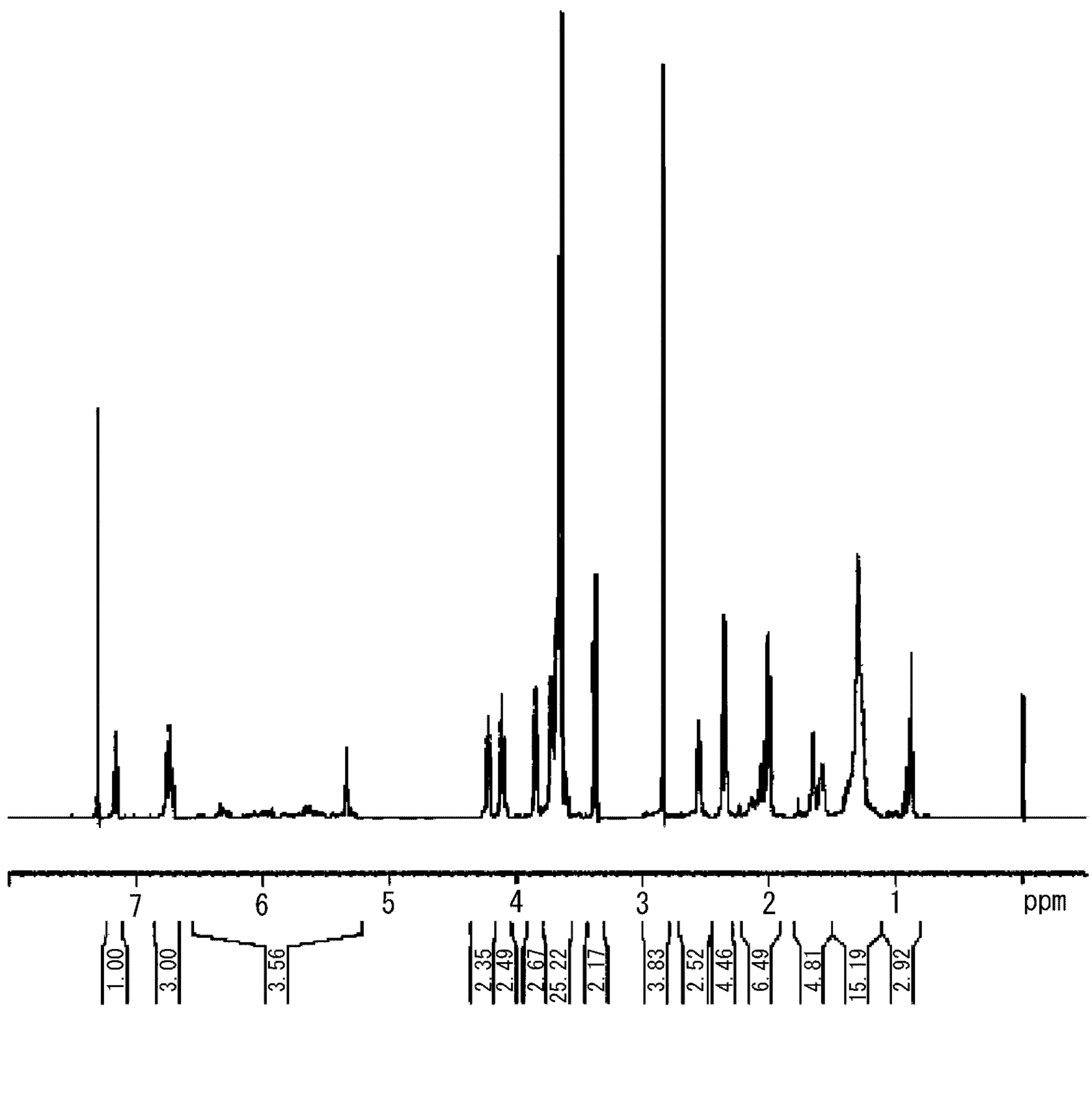
7
6
5
4
3
2
1
ppm
1.00
3.00
3.56
2.35
2.49
2.67
25.22
2.17
3.83
2.52
4.46
6.49
4.81
15.19
2.92

FIG. 18

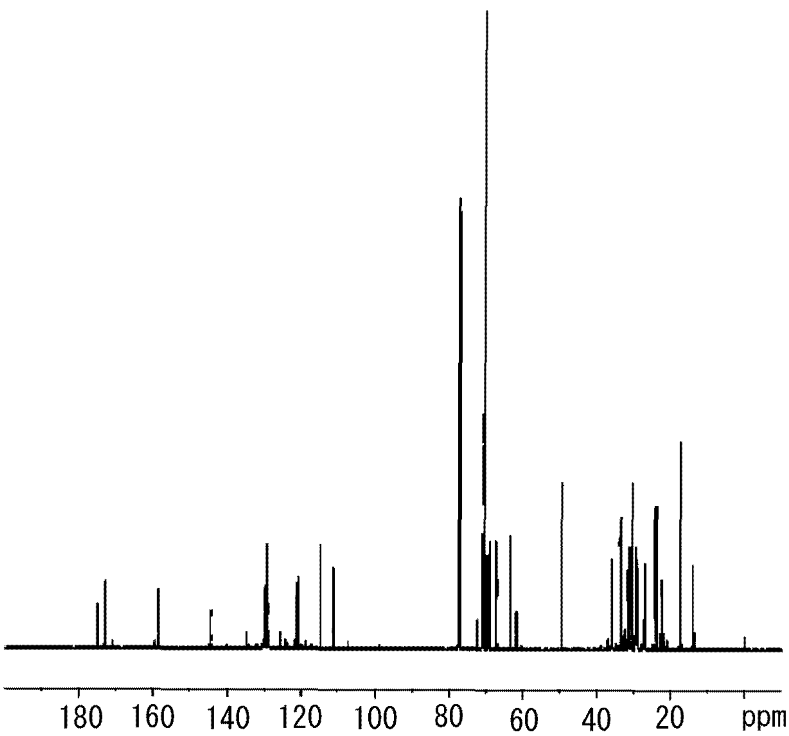

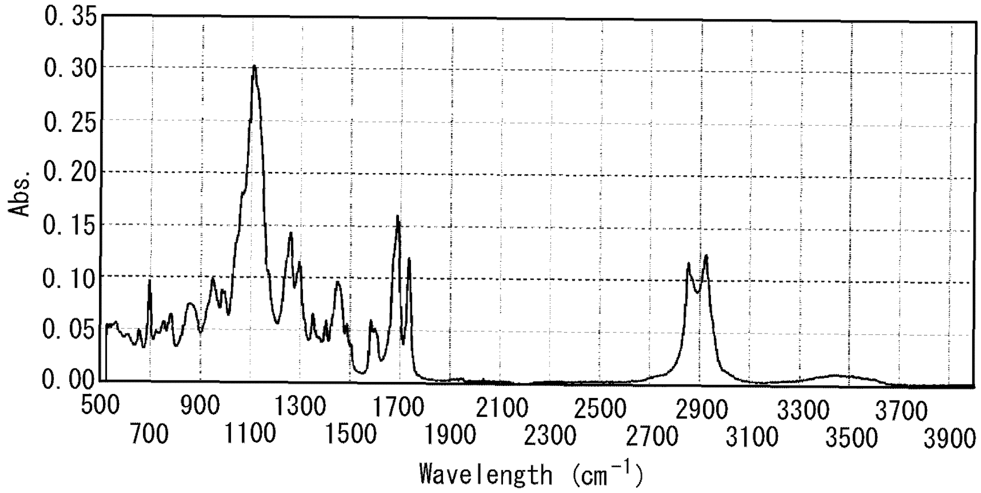
0. 35
0. 30
0. 25
0. 20
0. 15
0. 10
0. 05
0. 00
Abs.
500
700
900
1100
1300
1500
1700
1900
2100
2300
2500
2700
2900
3100
3300
3500
3700
3900
Wavelength (cm$^{-1}$)

FIG. 19

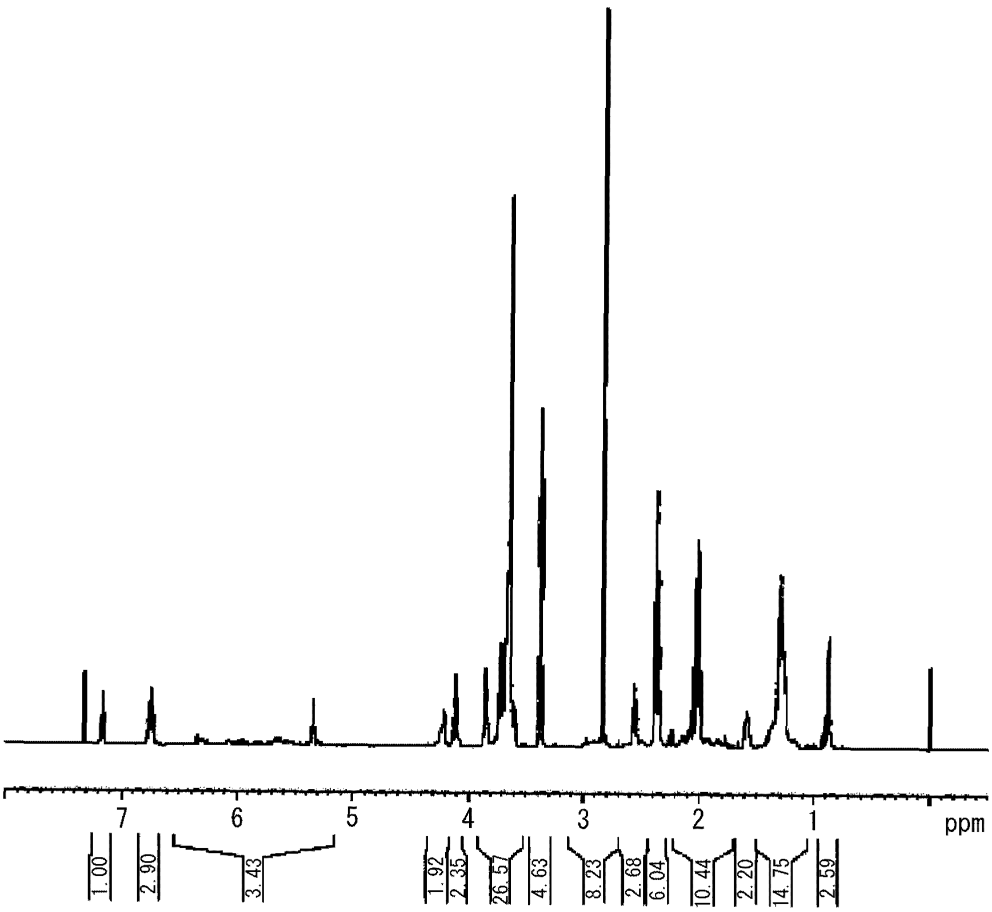
7
6
5
4
3
2
1
ppm
1.00
2.90
3.43
1.92
2.35
26.57
4.63
8.23
2.68
6.04
10.44
2.20
14.75
2.59

FIG. 20

FIG. 21

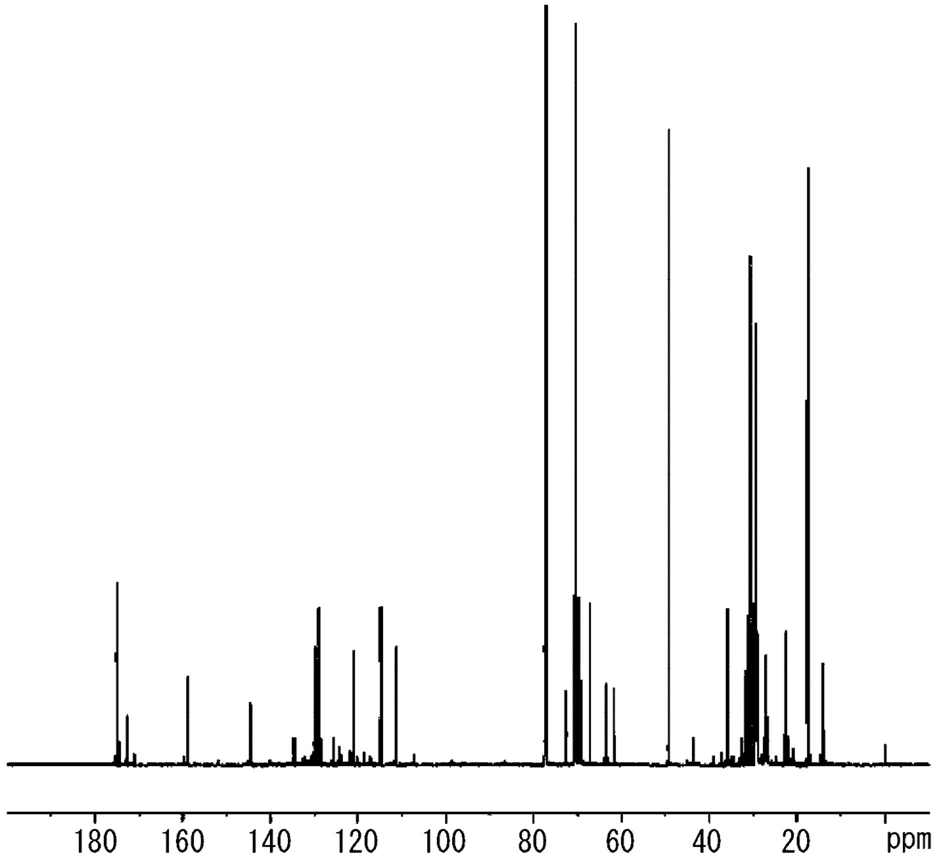
180
160
140
120
100
80
60
40
20
ppm

FIG. 22

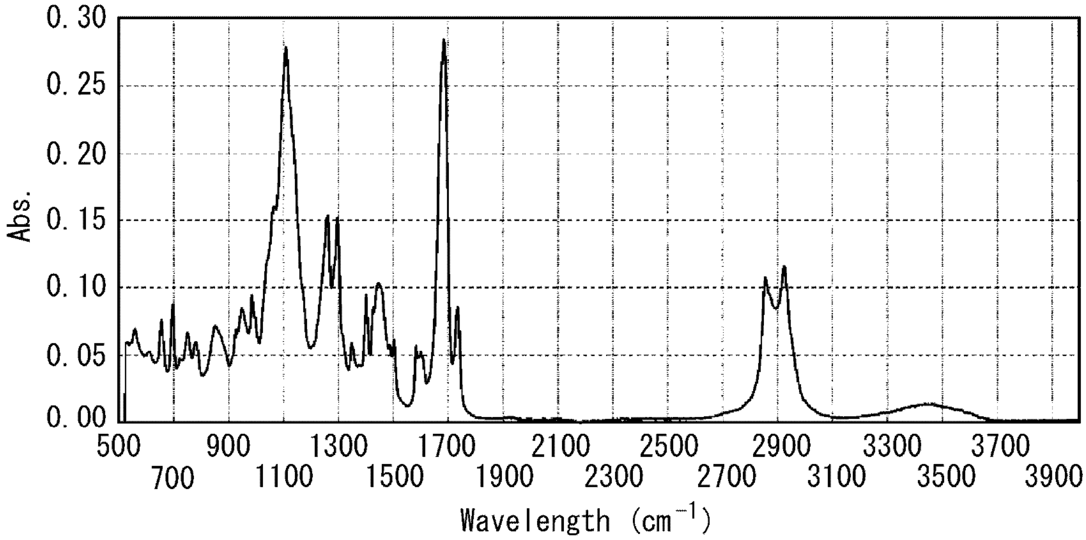
0.30
0.25
0.20
0.15
0.10
0.05
0.00
Abs.
500
700
900
1100
1300
1500
1700
1900
2100
2300
2500
2700
2900
3100
3300
3500
3700
3900
Wavelength (cm-1)

FIG. 23

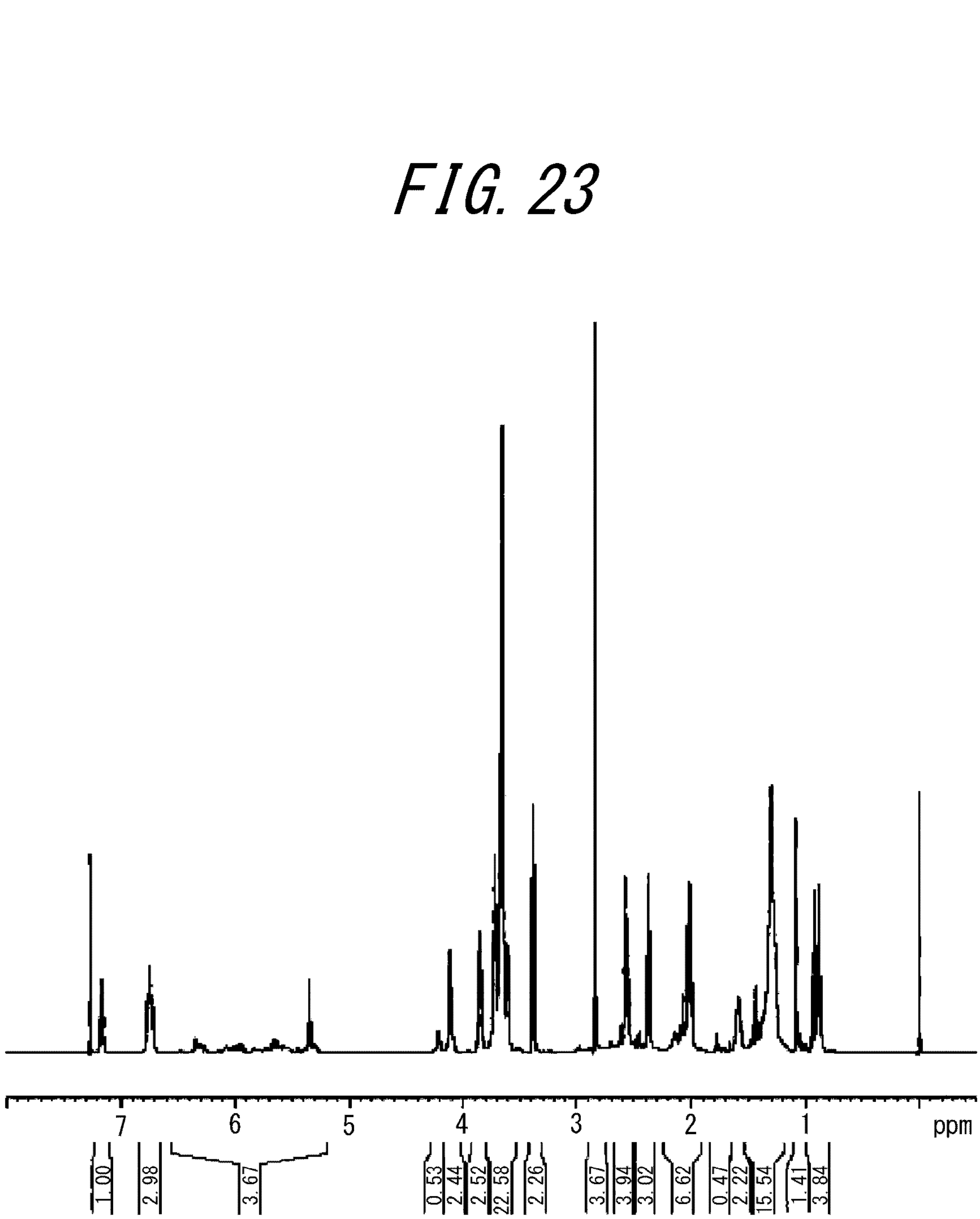
7
6
5
4
3
2
1
ppm
1.00
2.98
3.67
0.53
2.44
2.52
22.58
2.26
3.67
3.94
3.02
6.62
0.47
2.22
15.54
1.41
3.84

FIG. 24

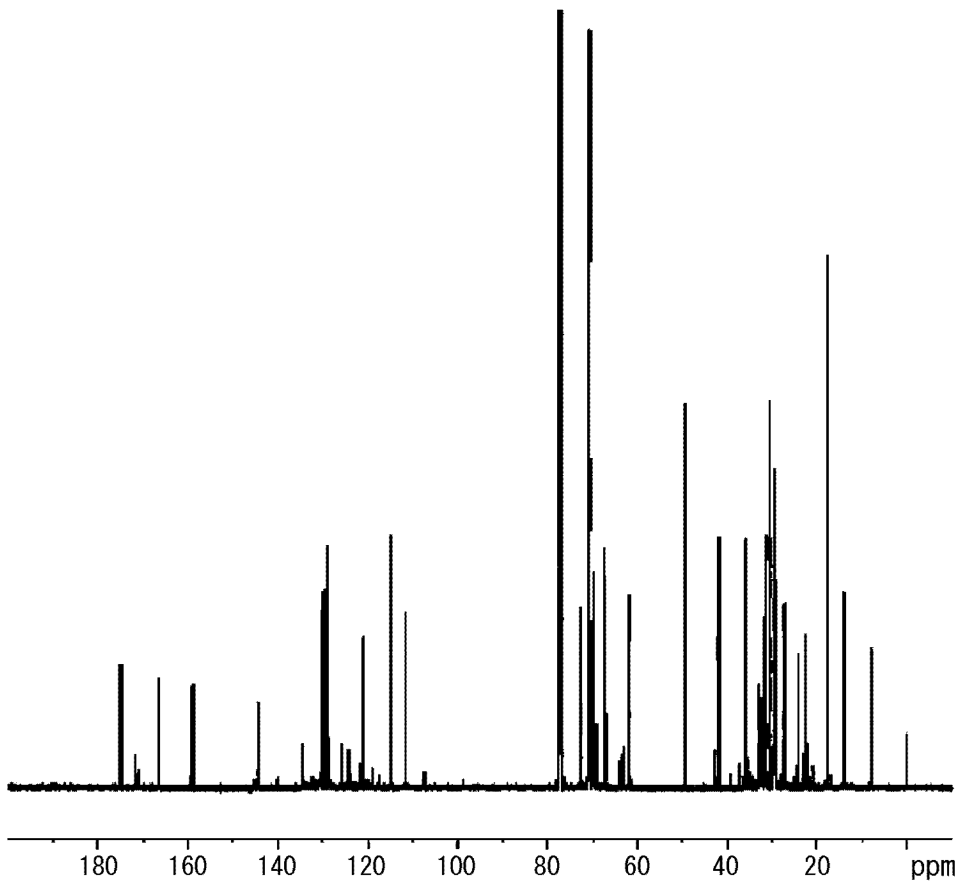

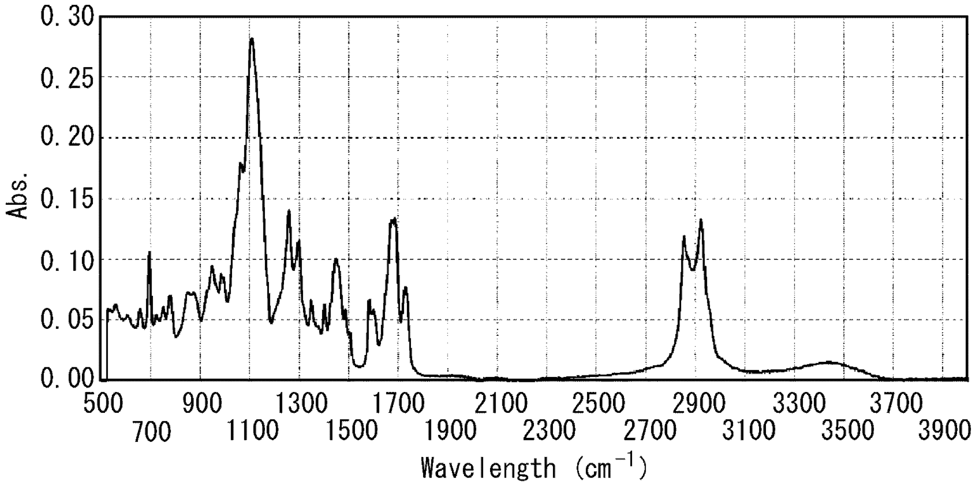
0.30
0.25
0.20
0.15
0.10
0.05
0.00
Abs.
500
700
900
1100
1300
1500
1700
1900
2100
2300
2500
2700
2900
3100
3300
3500
3700
3900
Wavelength (cm$^{-1}$)

FIG. 25

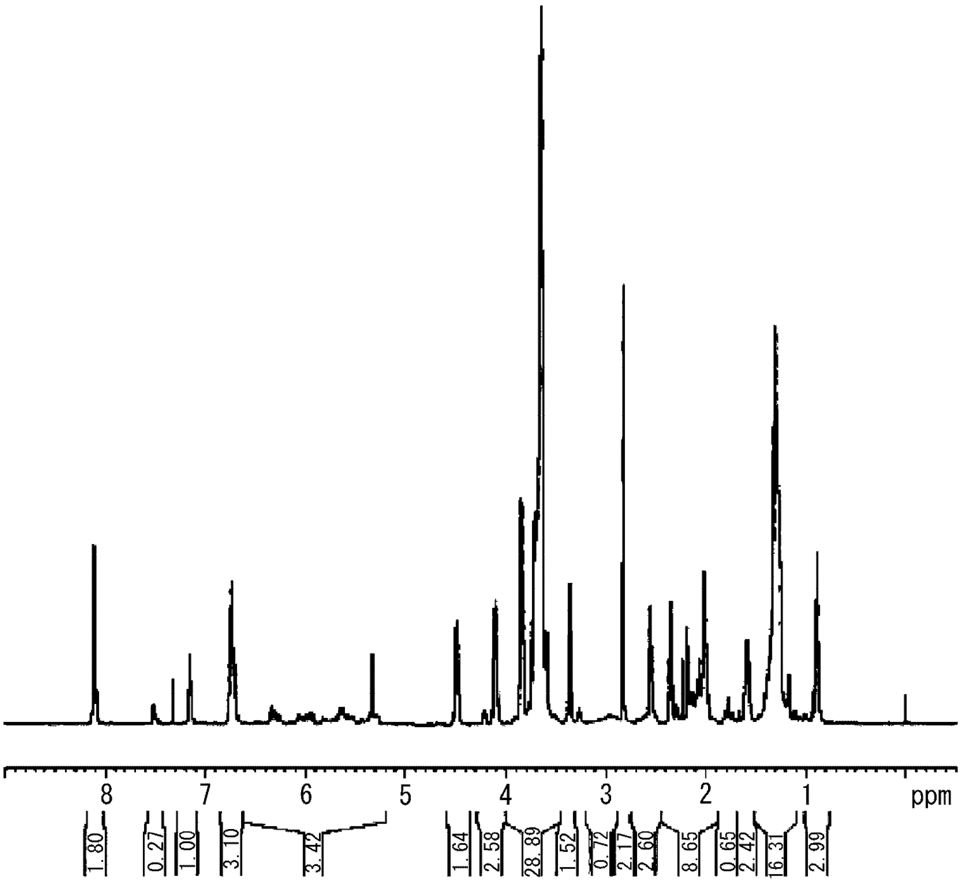
8
7
6
5
4
3
2
1
ppm
1.80
0.27
1.00
3.10
3.42
1.64
2.58
28.89
1.52
0.72
2.17
2.60
8.65
0.65
2.42
16.31
2.99

FIG. 26

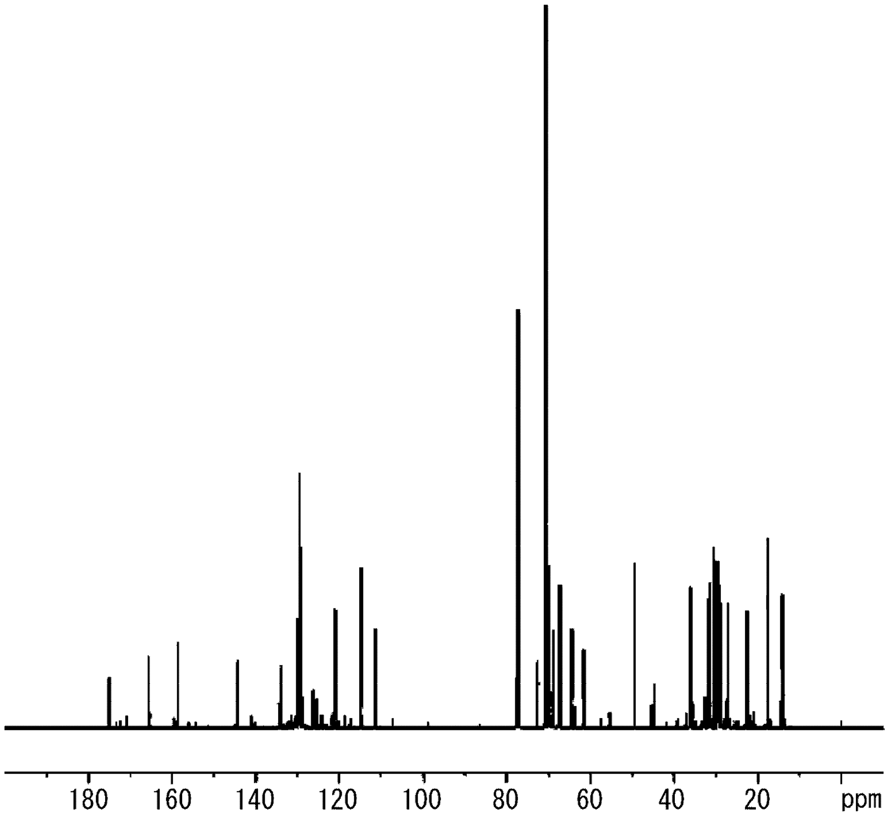
180
160
140
120
100
80
60
40
20
ppm

FIG. 27

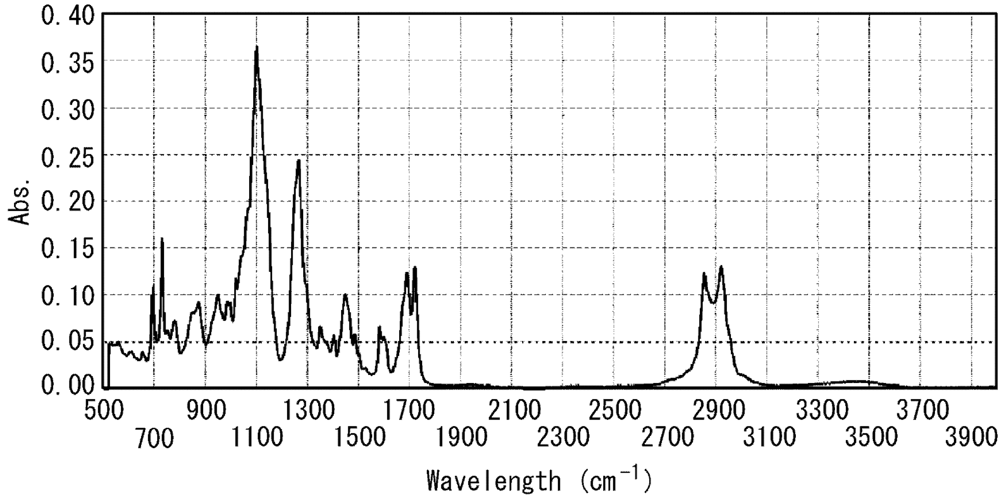
0. 40
0. 35
0. 30
0. 25
0. 20
0. 15
0. 10
0. 05
0. 00
Abs.
500
700
900
1100
1300
1500
1700
1900
2100
2300
2500
2700
2900
3100
3300
3500
3700
3900
Wavelength (cm$^{-1}$)

FIG. 28

# INTERNATIONAL SEARCH REPORT

International application No.
**PCT/JP2024/000325**

## A. CLASSIFICATION OF SUBJECT MATTER

***C07C 69/44***(2006.01)i; ***B32B 5/18***(2006.01)i; ***B32B 27/22***(2006.01)i; ***B32B 27/40***(2006.01)i; ***C07C 43/205***(2006.01)i; ***C07C 43/215***(2006.01)i; ***C07C 69/34***(2006.01)i; ***C07C 69/82***(2006.01)i; ***C07C 271/12***(2006.01)i; ***C08K 5/00***(2006.01)i; ***C08L 27/06***(2006.01)i; ***C08L 67/00***(2006.01)i; ***C08L 101/00***(2006.01)i
FI: C07C69/44 CSP; B32B5/18; B32B27/22; B32B27/40; C07C43/205 C; C07C43/215; C07C69/34; C07C69/82 B; C07C271/12; C08K5/00; C08L27/06; C08L67/00; C08L101/00

According to International Patent Classification (IPC) or to both national classification and IPC

## B. FIELDS SEARCHED

Minimum documentation searched (classification system followed by classification symbols)

C07C69/44; B32B5/18; B32B27/22; B32B27/40; C07C43/205; C07C43/215; C07C69/34; C07C69/82; C07C271/12; C08K5/00; C08L27/06; C08L67/00; C08L101/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

## C. DOCUMENTS CONSIDERED TO BE RELEVANT

| Category* | Citation of document, with indication, where appropriate, of the relevant passages | Relevant to claim No. |
|---|---|---|
| A | WO 2021/172076 A1 (NIPPON ZEON CO.) 02 September 2021 (2021-09-02) paragraphs [0035]-[0042], examples | 1-20 |
| A | CN 112794828 A (HONGLIDA (GUANGDONG) SYNTHESIS MATERIAL TECHNOLOGY CO., LTD.) 14 May 2021 (2021-05-14) claim 1, examples | 1-20 |
| A | CN 112480565 A (JIANGSU HUAXIN NEW MATERIAL CO., LTD.) 12 March 2021 (2021-03-12) claim 1, examples | 1-20 |
| P, A | WO 2023/127609 A1 (NIPPON ZEON CO.) 06 July 2023 (2023-07-06) claims 1-20, paragraphs [0049]-[0073], examples | 1-20 |

☐ Further documents are listed in the continuation of Box C.　　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 March 2024** | **26 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/000325**

| Patent document cited in search report | Publication date (day/month/year) | Patent family member(s) | Publication date (day/month/year) |
|---|---|---|---|
| WO 2021/172076 A1 | 02 September 2021 | US 2023/0095316 A1<br>paragraphs [0060]-[0079], examples<br>EP 4112688 A1<br>CN 115135716 A | |
| CN 112794828 A | 14 May 2021 | (Family: none) | |
| CN 112480565 A | 12 March 2021 | (Family: none) | |
| WO 2023/127609 A1 | 06 July 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012197394 A
- WO 2016098344 A1


### Non-patent literature cited in the description


- **SANDLER** ; **KARO** ; **HIROKAWA SHOTEN**. *Organic Functional Group Preparations*, vol. I,II
- **MICHAEL B. SMITH** ; **JERRY MARCH**. March's Advanced Organic Chemistry. WILEY